(19) Europäisches Patentamt / European Patent Office / Office européen des brevets

(11) **EP 1 427 409 B1**

(12) **EUROPEAN PATENT SPECIFICATION**

(45) Date of publication and mention of the grant of the patent: **15.10.2008 Bulletin 2008/42**

(21) Application number: **02770534.2**

(22) Date of filing: **19.09.2002**

(51) Int Cl.:
***A61K 31/397*** *(2006.01)* ***A61K 45/06*** *(2006.01)*
***C07D 205/08*** *(2006.01)* ***A61P 9/00*** *(2006.01)*

(86) International application number: **PCT/US2002/029756**

(87) International publication number: **WO 2003/026644 (03.04.2003 Gazette 2003/14)**

(54) **METHODS FOR TREATING OR PREVENTING VASCULAR INFLAMMATION USING STEROL ABSORPTION INHIBITOR(S)**

VERFAHREN ZUR BEHANDLUNG ODER VERHINDERUNG VON VASKULÄRER ENTZÜNDUNG MIT STEROL-ABSORBIERUNGS- INHIBITOR(EN)

METHODES DE TRAITEMENT OU DE PREVENTION D'UNE INFLAMMATION VASCULAIRE AU MOYEN D'UN OU D'INHIBITEURS D'ABSORPTION DE STEROL

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **21.09.2001 US 323937 P**

(43) Date of publication of application: **16.06.2004 Bulletin 2004/25**

(73) Proprietor: **Schering Corporation**
**Kenilworth, NJ 07033-0530 (US)**

(72) Inventor: **DAVIS, Harry, R.**
**Berkeley Heights, NJ 07922 (US)**

(74) Representative: **HOFFMANN EITLE**
**Patent- und Rechtsanwälte**
**Arabellastrasse 4**
**81925 München (DE)**

(56) References cited:
**WO-A-93/02048**
**WO-A-94/14433**
**WO-A-02/058685**
**WO-A-02/058696**
**WO-A-02/058731**

- **SAGER P.T. ET AL: 'Effect of coadministration of ezetimibe and simvastatin on High-Sensivity C-reactive protein' AM J CARDIOL vol. 92, 2003, pages 1414 - 1418**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 427 409 B1

## Description

### CROSS-REFERENCE TO RELATED APPLICATIONS

This application claims the benefit of priority from U.S. Provisional Patent Application Serial No. 60/323,937, filed September 21, 2001, and is a continuation-in-part of U.S. Patent Application Serial No. 10/166,942, filed June 11, 2002, each incorporated herein by reference.

### FIELD OF THE INVENTION

The present invention relates to the use as defined in the claims for treating or preventing vascular inflammation in a subject comprising administering to the subject a composition comprising a combination of (1) at least one sterol absorption inhibitor and/or 5α-stanol absorption inhibitor and (2) at least one cholesterol biosynthesis inhibitor.

### BACKGROUND OF THE INVENTION

Vascular inflammation is an etiological event that often precedes the development and the continual process of atherosclerotic coronary heart disease. Vascular inflammation, beginning with an injury or change in the endothelial wall of the artery, may cause an alteration in the intimal layer that increases platelet adhesion to the endothelium.

Vascular stimuli to mammals, such as cellular injury or inflammation, may lead to the production of various proteins, commonly called acute response proteins, in the body. One particular type of acute phase protein is c-reactive protein (CRP). Manufactured in the liver and deposited in damaged tissue, CRP is found in high levels in inflammatory fluids and in both the intimal layer of the atherosclerotic artery and within the lesions of atherosclerotic plaque.

Studies have shown a positive association between CRP and coronary artery disease. For example, in a survey of 388 British men aged 50-69, the prevalence of coronary artery disease increased 1.5 fold for each doubling of CRP level (Mendall MA, Patel P, Ballam L, et al., "C-reactive protein and its relation to cardiovascular risk factor: A population based cross sectional study", BMJ. 1996;312:1061-1065.). Multiple prospective studies have also demonstrated that baseline CRP is a good marker of future cardiovascular events (Riker P, Haughie P. Prospective studies of C-reactive protein as a risk factor for cardiovascular disease. J Investig Med. 1998;46:391-395.).

Thus, there is a need in the art for compositions and treatments for preventing or treating vascular inflammation. Furthermore, there is a need in the art for reducing or treating c-reactive protein in vascular systems.

### SUMMARY OF THE INVENTION

One embodiment of the present invention provides the use of a combination of (1) at least one sterol absorption inhibitor, at least one 5α-stanol absorption inhibitor or mixtures thereof and (2) at least one cholesterol biosynthesis inhibitor for the manufacture of a medicament for treating or preventing vascular inflammation or for reducing c-reactive protein levels comprising the step of administering the combination to a subject in need of such administration.

Another embodiment of the present invention provides the use of a combination of (1) at least one sterol absorption inhibitor, at least one 5α-stanol absorption inhibitor or mixtures thereof and (2) at least one cholesterol biosynthesis inhibitor for the manufacture of a medicament for treating or preventing vascular inflammation in a subject comprising the step of administering the combination to a subject having a level of c-reactive protein which indicates the presence vascular inflammation or the potential for vascular inflammation.

In another embodiment, the present invention provides the use of a combination of (1) at least one sterol absorption inhibitor, at least one 5α-stanol absorption inhibitor or mixtures thereof and (2) at least one cholesterol biosynthesis inhibitor for the manufacture of a medicament for treating or preventing vascular inflammation or for reducing c-reactive protein comprising the step of administering to a subject the combination, wherein the at least one sterol absorption inhibitor is selected from the group of compounds represented by Formula (I):

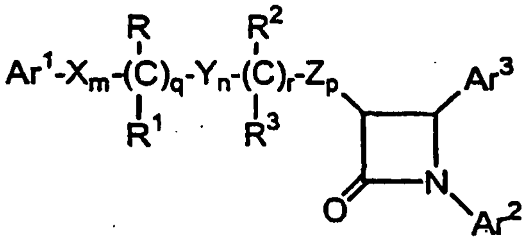

(I)

or a pharmaceutically acceptable salt thereof or a solvate thereof, wherein:

$Ar^1$ and $Ar^2$ are independently selected from the group consisting of aryl and $R^4$-substituted aryl;
$Ar^3$ is aryl or $R^5$-substituted aryl;
X, Y and Z are independently selected from the group consisting of- $CH_2$-, -CH(lower alkyl- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$and $R^3$ are independently selected from the group consisting of hydrogen, lower alkyl and aryl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene) $COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$,
$-COR^6$, $-SO_2NR^6R^7$, $S(O)_{0-2}R^9$, $-O(CH_2)_{1-10-}COOR^6$, $-O(CH_2)_{1-10}CONR^6R^7$, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl.

Other than in the operating examples, or where otherwise indicated, all numbers expressing quantities of ingredients, reaction conditions, and so forth used in the specification and claims are to be understood as being modified in all instances by the term "about".

## DETAILED DESCRIPTION

In one embodiment, the present invention is directed to the use of a combination of (1) at least one sterol absorption inhibitor, at least one 5α-stanol absorption inhibitor or mixtures thereof and (2) at least one cholesterol biosynthesis inhibitor for the manufacture of a medicament for treating or preventing vascular inflammation by administering to a subject a therapeutically effective amount of the combination, wherein the at least one (one or more) sterol absorption inhibitor, at least one 5α-stanol absorption inhibitor or mixtures thereof is, such as but not limited to, substituted azetidinone or substituted β-lactam sterol absorption inhibitors discussed in detail below.

Cholesteryl esters are a major component of atherosclerotic lesions which can result in vascular inflammation and an increase in plasma c-reactive protein levels. Cholesteryl esters are also a major component of and the major storage form of cholesterol in arterial wall cells. Formation of cholesteryl esters is also a step in the intestinal absorption of dietary cholesterol. Thus, inhibition of cholesteryl ester formation and reduction of serum cholesterol can inhibit the progression of atherosclerotic lesion formation, thereby treating or preventing vascular inflammation.

The present invention is directed towards the use as defined in the claims for preventing or treating vascular inflammation and/or lowering plasma c-reactive protein levels in a subject. The administration of the sterol absorption inhibitor compositions of the present invention prevents or treats vascular inflammation by inhibiting the progression of atherosclerotic lesion formation. With such administration the c-reactive protein levels are also reduced.

Patients with c-reactive protein levels greater than about 0.4 mgldL have been reported as having increased

vascular inflammation and increased risk for vascular disease as compared to patients with levels less than 0.4 mg/dL. (L. Gruberb, "Inflammatory Markers in Acute Coronary Syndromes: C-reactive Protein (CRP) and Chlamydia", American Heart Assoc. Scientific Sessions 2000). Patients with levels greater 3.4 mg/dL of c-reactive protein were reported to be in the highest quartile of risk. Patients in the second quartile (0.4 to 1.0 mg/dL of c-reactive protein) and third quartile (1.0 to 3.4 mg/dL of c-reactive protein) also have increased risk of vascular disease as compared to patients in the lowest quartile (<0.4 mgldL c-reactive protein).

C-reactive protein assays and methodologies for the same are available from Behring Diagnostics Inc., of Somerville, NJ. Moreover, methods for analyzing c-reactive proteins are described in U.S. Patents Nos. 5,358,852; 6,040,147; and 6,277,584, whose contents are incorporated herein by reference. One particularly useful method is described in an analytical procedure section of this application.

The term "therapeutically effective amount" means that amount of a therapeutic agent of the composition, such as the sterol absorption inhibitor(s) and other pharmacological or therapeutic agents described below, that will elicit a biological or medical response of a tissue, system, or subject that is being sought by the administrator (such as a researcher, doctor or veterinarian) which includes alleviation of the symptoms of the condition or disease being treated and the prevention, slowing or halting of progression of the vascular inflammation condition and/or lowering the level of plasma c-reactive protein in a subject.

Examples of suitable subjects that can be treated according to the methods of the present invention include mammals, such as humans or dogs, and other animals.

As used herein, "vascular" means relating to blood vessels, including but not limited to arteries and/or veins, and includes cardiovascular, cerebrovascular, peripheral vascular and combinations thereof. The term "inflammation" generally refers to injury or the bodily response to an injury. The term "vascular inflammation" more specifically refers to arterial damage and bodily responses thereto that can lead to atherosclerosis or coronary heart disease. Atherosclerosis is often indicated by a thickening and build-up of plaque in the arteries and typically occurs when the innermost layer of an artery, the endothelium, becomes damaged by cholesterol, toxins, oxidants, infectious agents and the like. The damaged endothelial cells in the artery walls produce adhesion molecules that allow white blood cells to accumulate in the vessel wall. Fats and cholesterol also build-up with the white blood cells causing inflammation of the artery. Such build-up can thicken to a point where the artery becomes vulnerable to blockage from a clot resulting in heart attack or stroke.

As used herein, "combination therapy" or "therapeutic combination" means the administration of two or more therapeutic agents, such as sterol or 5α-stanol absorption inhibitor(s) and other pharmacological or therapeutic agents discussed below, to prevent or treat vascular inflammation. Such administration includes coadministration of these therapeutic agents in a substantially simultaneous manner, such as in a single tablet or capsule having a fixed ratio of active ingredients or in multiple, separate capsules for each therapeutic agent. Also, such administration includes use of each type of therapeutic agent in a sequential manner. In either case, the treatment using the combination therapy will provide beneficial effects in treating vascular inflammation. A potential advantage of the combination therapy disclosed herein may be a reduction in the required amount of an individual therapeutic compound or the overall total amount of therapeutic compounds that are effective in treating vascular inflammation. By using a combination of therapeutic agents, the side effects of the individual compounds can be reduced as compared to a monotherapy, which can improve patient compliance. Also, therapeutic agents can be selected to provide a broader range of complimentary effects or complimentary modes of action.

As discussed above, the compositions, pharmaceutical compositions and therapeutic combinations of the present invention comprise one or more sterol absorption inhibitors such as are discussed in detail below. As used herein, "sterol absorption inhibitor" means a compound capable of inhibiting the absorption of one or more sterols, including but not limited to cholesterol, phytosterols (such as sitosterol, campesterol, stigmasterol and avenosterol), and "5α-stanol absorption inhibitor" means a compound capable of inhibiting the absorption of one or more 5α-stanols (such as cholestanol, 5α-campestanol, 5α-sitostanol), and mixtures thereof, when administered in a therapeutically effective (sterol or stanol absorption inhibiting) amount to a mammal or human.

These sterol and 5α-stanol absorption inhibitors can be useful in treating or preventing vascular inflammation. Moreover, these sterol and 5α-stanol absorption inhibitors can be useful for lowering or controlling c-reactive protein blood levels in a subject to less than about 3.4 mg/dL. Preferably, the c-reactive protein blood levels in a subject are reduced or controlled to less than 1.0 mg/dL by the methods of the present invention. More preferably, the c-reactive protein blood levels in a subject are reduced or controlled to less than 0.4 mg/dL by the methods of the present invention.

In one embodiment, sterol and 5α-stanol absorption inhibitors useful in the methods of the present invention are represented by Formula (I) below:

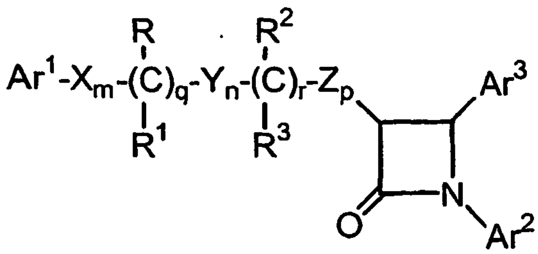

(I)

or a pharmaceutically acceptable salt thereof or a solvate thereof, wherein, in Formula (I) above:

$Ar^1$ and $Ar^2$ are independently selected from the group consisting of aryl and $R^4$-substituted aryl;
$Ar^3$ is aryl or $R^5$-substituted aryl;
X, Y and Z are independently selected from the group consisting of $-CH_2-$; -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1; r is 0 or 1; m, n and p are independently selected from 0,1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$,- $NR^6(CO)OR^9$, $-NR^6(CO)NR^7R^8$, $-NR^6SO_2R^9$, $-COOR^6$, $-CONR^6R^7$, $-COR^6$,$-SO_2NR^6R^7$, $S(O)_{0-2}R^9$, $-O(CH_2)_{1-10}-COOR^6$, $-O(CH_2)_{1-10}-CONR^6R^7$, -(lower alkylene)$COOR^6$ and
$-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl.

Preferably, $R^4$ is 1-3 independently selected substituents, and $R^5$ is preferably 1-3 independently selected substituents.

As used herein, the term "alkyl" or "lower alkyl" means straight or branched alkyl chains having from 1 to 6 carbon atoms and "alkoxy" means alkoxy groups having 1 to 6 carbon atoms. Non-limiting examples of lower alkyl groups include, for example methyl, ethyl, propyl, and butyl groups.

"Alkenyl" means straight or branched carbon chains having one or more double bonds in the chain, conjugated or unconjugated. Similarly, "alkynyl" means straight or branched carbon chains having one or more triple bonds in the chain. Where an alkyl, alkenyl or alkynyl chain joins two other variables and is therefore bivalent, the terms alkylene, alkenylene and alkynylene are used.

"Cycloalkyl" means a saturated carbon ring of 3 to 6 carbon atoms, while "cycloalkylene" refers to a corresponding bivalent ring, wherein the points of attachment to other groups include all positional isomers.

"Halogeno" refers to fluorine, chlorine, bromine or iodine radicals.

"Aryl" means phenyl, naphthyl, indenyl, tetrahydronaphthyl or indanyl.

"Phenylene" means a bivalent phenyl group, including ortho, meta and para-substitution.

The statements wherein, for example, R, $R^1$, $R^2$ and $R^3$, are said to be independently selected from a group of substituents, mean that R, $R^1$, $R^2$ and $R^3$ are independently selected, but also that where an R, $R^1$, $R^2$ and $R^3$ variable occurs more than once in a molecule, each occurrence is independently selected (e.g., if R is- $OR^6$, wherein $R^6$ is hydrogen, $R^2$ can be $-OR^6$ wherein $R^6$ is lower alkyl). Those skilled in the art will recognize that the size and nature of the substituent(s) will affect the number of substituents that can be present.

Compounds of the invention have at least one asymmetrical carbon atom and therefore all isomers, including enantiomers, stereoisomers, rotamers, tautomers and racemates of the compounds of Formulae I-XII are contemplated as being part of this invention. The invention includes d and I isomers in both pure form and in admixture, including

racemic mixtures. Isomers can be prepared using conventional techniques, either by reacting optically pure or optically enriched starting materials or by separating isomers of a compound of the Formulas I-XII. Isomers may also include geometric isomers, e.g., when a double bond is present.

Those skilled in the art will appreciate that for some of the compounds of the Formulas I-XII, one isomer will show greater pharmacological activity than other isomers.

Compounds of the invention with an amino group can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

As used herein, "solvate" means a molecular or ionic complex of molecules or ions of solvent with those of solute (for example, one or more compounds of Formulae I-XII, isomers of the compounds of Formulae I-XII, or prodrugs of the compounds of Formulae I-XII). Non-limiting examples of useful solvents include polar, protic solvents such as water and/or alcohols (for example methanol).

Prodrugs of the compounds of Formulae I-XII are contemplated as being part of this invention. As used herein, "prodrug" means compounds that are drug precursors which, following administration to a patient, release the drug *in vivo* via some chemical or physiological process (e.g., a prodrug on being brought to the physiological pH or through enzyme action is converted to the desired drug form).

Preferred compounds of Formula (I) are those in which $Ar^1$ is phenyl or $R^4$-substituted phenyl, more preferably (4-$R^4$)-substituted phenyl. $Ar^2$ is preferably phenyl or $R^4$-substituted phenyl, more preferably (4-$R^4$)-substituted phenyl. $Ar^3$ is preferably $R^5$-substituted phenyl, more preferably (4-$R^5$)-substituted phenyl. When $Ar^1$ is (4-$R^4$)-substituted phenyl, $R^4$ is preferably a halogen. When $Ar^2$ and $Ar^3$ are $R^4$- and $R^5$-substituted phenyl, respectively, $R^4$ is preferably halogen or -$OR^6$ and $R^5$ is preferably -$OR^6$, wherein $R^6$ is lower alkyl or hydrogen. Especially preferred are compounds wherein each of $Ar^1$ and $Ar^2$ is 4-fluorophenyl and $Ar^3$ is 4-hydroxyphenyl or 4-methoxyphenyl.

X, Y and Z are each preferably -$CH_2$-. $R^1$ and $R^3$ are each preferably hydrogen. R and $R^2$ are preferably -$OR^6$ wherein $R^6$ is hydrogen, or a group readily metabolizable to a hydroxyl (such as -O(CO)$R^6$, -O(CO)O$R^9$ and -O(CO)N$R^6R^7$, defined above).

The sum of m, n, p, q and r is preferably 2, 3 or 4, more preferably 3. Preferred are compounds wherein m, n and r are each zero, q is 1 and p is 2.

Also preferred are compounds of Formula (I) in which p, q and n are each zero, r is 1 and m is 2 or 3. More preferred are compounds wherein m, n and r are each zero, q is 1, p is 2, Z is -$CH_2$- and R is -$OR^6$, especially when $R^6$ is hydrogen.

Also more preferred are compounds of Formula (I) wherein p, q and n are each zero, r is 1, m is 2, X is -$CH_2$- and $R^2$ is -$OR^6$, especially when $R^6$ is hydrogen.

Another group of preferred compounds of Formula (I) is that in which $Ar^1$ is phenyl or $R^4$-substituted phenyl, $Ar^2$ is phenyl or $R^4$-substituted phenyl and $Ar^3$ is $R^5$-substituted phenyl. Also preferred are compounds in which $Ar^1$ is phenyl or $R^4$-substituted phenyl, $Ar^2$ is phenyl or $R^4$-substituted phenyl, $Ar^3$ is $R^5$-substituted phenyl, and the sum of m, n, p, q and r is 2, 3 or 4, more preferably 3. More preferred are compounds wherein $Ar^1$ is phenyl or $R^4$-substituted phenyl, $Ar^2$ is phenyl or $R^4$-substituted phenyl, $Ar^3$ is $R^5$-substituted phenyl, and wherein m, n and r are each zero, q is 1 and p is 2, or wherein p, q and n are each zero, r is 1 and m is 2 or 3.

In a preferred embodiment, a sterol or 5$\alpha$-stanol inhibitor of Formula (I) useful in the compositions, therapeutic combinations and methods of the present invention is represented by Formula (II) (ezetimibe) below:

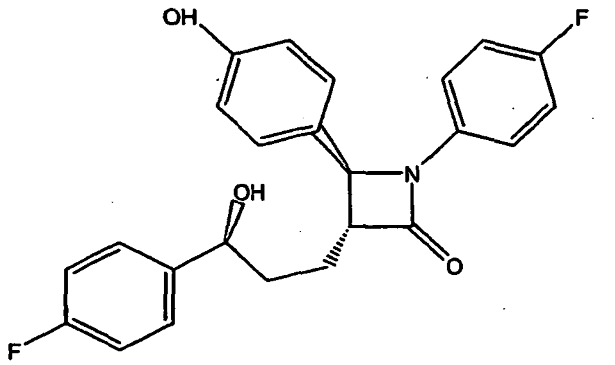

(II)

or a pharmaceutically acceptable salt or solvate thereof. The compound of Formula (II) can be in anhydrous or hydrated form.

Compounds of Formula I can be prepared by a variety of methods well known to those skilled in the art, for example such as are disclosed in U.S. Patents Nos. 5,631,365, 5,767,115, 5,846,966, 6,207,822, U.S. Patent Application No. 10/105,710 filed March 25, 2002, and PCT Patent Application WO 93/02048, each of which is incorporated herein by reference, and in the Example below. For example, suitable compounds of Formula I can be prepared by a method comprising the steps of:

(a) treating with a strong base a lactone of the Formula A or B:

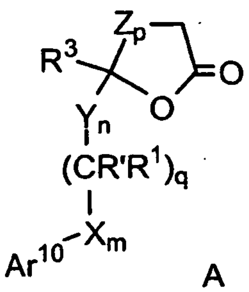

A

or

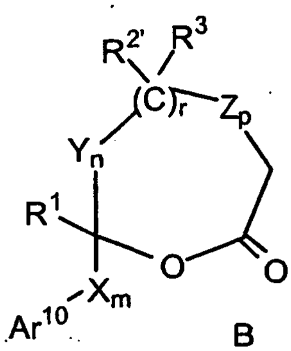

B

wherein R' and $R^{2'}$ are R and $R^2$, respectively, or are suitably protected hydroxy groups; $Ar^{10}$ is $Ar^1$, a suitably protected hydroxy-substituted aryl or a suitably protected amino-substituted aryl; and the remaining variables are as defined above for Formula I, provided that in lactone of formula B, when n and r are each zero, p is 1-4;
(b) reacting the product of step (a) with an imine of the formula

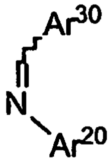

wherein $Ar^{20}$ is $Ar^2$, a suitably protected hydroxy-substituted aryl or a suitably protected amino-substituted aryl; and $Ar^{30}$ is $Ar^3$, a suitably protected hydroxy-substituted aryl or a suitably protected amino-substituted aryl;
c) quenching the reaction with an acid;
d) optionally removing the protecting groups from R', $R^{2'}$, $Ar^{10}$, $Ar^{20}$ and $Ar^{30}$, when present; and
e) optionally functionalizing hydroxy or amino substituents at R, $R^2$, $Ar^1$, $Ar^2$ and $Ar^3$.

Using the lactones shown above, compounds of Formula IA and IB are obtained as follows:

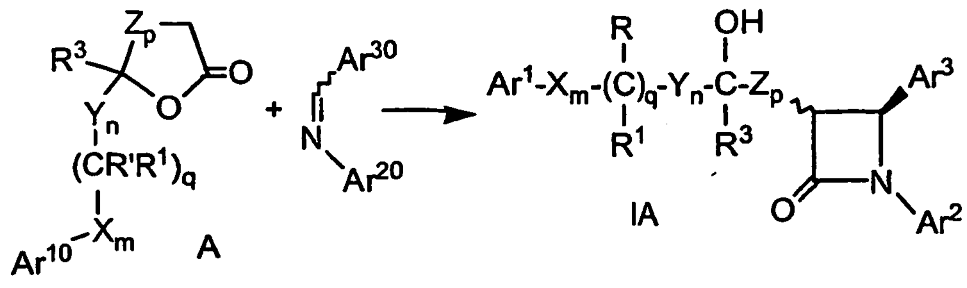

wherein the variables are as defined above; and

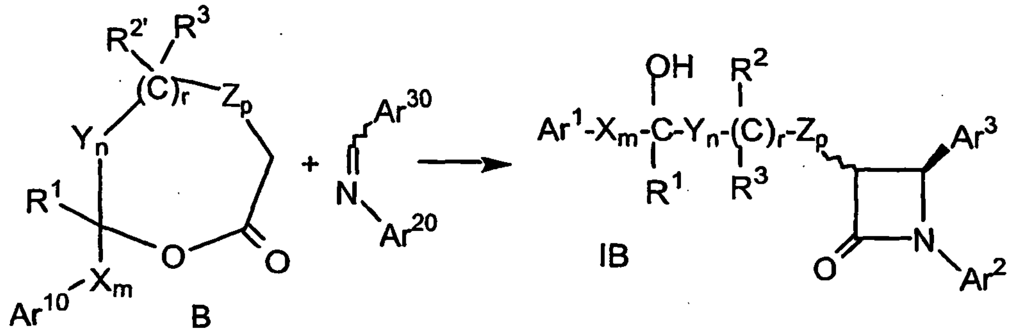

wherein the variables are as defined above.

Alternative sterol absorption inhibitors useful in the methods of the present invention are represented by Formula (III) below:

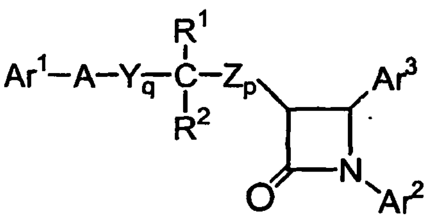

(III)

or a pharmaceutically acceptable salt thereof or a solvate thereof, wherein, in Formula (III) above:

$Ar^1$ is $R^3$-substituted aryl;
$Ar^2$ is $R^4$-substituted aryl;
$Ar^3$ is $R^5$-substituted aryl;
Y and Z are independently selected from the group consisting of $-CH_2-$, -CH(lower alkyl)- and -C(dilower alkyl)-;
A is selected from -O-, -S-, -S(O)- or $-S(O)_2-$;
$R^1$ is selected from the group consisting of $-OR^6$, $-O(CO)R^6$, $-O(CO)OR^9$ and $-O(CO)NR^6R^7$; $R^2$ is selected from the group consisting of hydrogen, lower alkyl and aryl; or $R^1$ and $R^2$ together are =O;
q is 1, 2 or 3;
p is 0, 1, 2, 3 or 4;
$R^5$ is 1-3 substituents independently selected from the group consisting of $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-lower alkyl,

$-NR^6SO_2$-aryl, $-CONR^6R^7$, $-COR^6$, $-SO_2NR^6R^7$, $S(O)_{0-2}$-alkyl, $S(O)_{0-2}$-aryl, $-O(CH_2)_{1-10}-COOR^6$, $-O(CH_2)_{1-10}CONR^6R^7$, o-halogeno, m-halogeno, o-lower alkyl, m-lower alkyl, -(lower alkylene)-$COOR^6$, and $-CH=CH-COOR^6$;

$R^3$ and $R^4$ are independently 1-3 substituents independently selected from the group consisting of $R^5$, hydrogen, p-lower alkyl, aryl, $-NO_2$, $-CF_3$ and p-halogeno;

$R^6$, $R^7$ and $R^8$ are independently selected from the group consisting of hydrogen, lower alkyl, aryl and aryl-substituted lower alkyl; and $R^9$ is lower alkyl, aryl or aryl-substituted lower alkyl.

Preferred compounds of Formula I include those in which $Ar^1$ is $R^3$-substituted phenyl, especially (4-$R^3$)-substituted phenyl. $Ar^2$ is preferably $R^4$-substituted phenyl, especially (4-$R^4$)-substituted phenyl. $Ar^3$ is preferably $R^5$-substituted phenyl, especially (4-$R^5$)-substituted phenyl. Mono-substitution of each of $Ar^1$, $Ar^2$ and $Ar^3$ is preferred.

Y and Z are each preferably $-CH_2$- $R^2$ is preferably hydrogen. $R^1$ is preferably $-OR^6$ wherein $R^6$ is hydrogen, or a group readily metabolizable to a hydroxyl (such as $-O(CO)R^6$, $-O(CO)OR^9$ and $-O(CO)NR^6R^7$, defined above). Also preferred are compounds wherein $R^1$ and $R^2$ together are =O.

The sum of q and p is preferably 1 or 2, more preferably 1. Preferred are compounds wherein p is zero and q is 1. More preferred are compounds wherein p is zero, q is 1, Y is $-CH_2$- and $R^1$ is $-OR^6$, especially when $R^6$ is hydrogen.

Another group of preferred compounds is that in which $Ar^1$ is $R^3$-substituted phenyl, $Ar^2$ is $R^4$-substituted phenyl and $Ar^3$ is $R^5$-substituted phenyl.

Also preferred are compounds wherein $Ar^1$ is $R^3$-substituted phenyl, $Ar^2$ is $R^4$-substituted phenyl, $Ar^3$ is $R^5$-substituted phenyl, and the sum of p and q is 1 or 2, especially 1. More preferred are compounds wherein $Ar^1$ is $R^3$-substituted phenyl, $Ar^2$ is $R^4$-substituted phenyl, $Ar^3$ is $R^5$-substituted phenyl, p is zero and q is 1.

A is preferably -O-.

$R^3$ is preferably $-COOR^6$, $-CONR^6R^7$, $-COR^6$, $-SO_2NR^6R^7$, $S(O)_{0-2}$-alkyl, $S(O)_{0-2}$-aryl, $NO_2$ or halogeno. A more preferred definition for $R^3$ is halogeno, especially fluoro or chloro.

$R^4$ is preferably hydrogen, lower alkyl, $-OR^6$, $-O(CO)R^6$, $-O(CO)OR^9$, $-O(CO)NR^6R^7$, $-NR^6R^7$, $COR^6$ or halogeno, wherein $R^6$ and $R^7$ are preferably independently hydrogen or lower alkyl, and $R^9$ is preferably lower alkyl. A more preferred definition for $R^4$ is hydrogen or halogeno, especially fluoro or chloro.

$R^5$ is preferably $-OR^6$, $-O(CO)R^6$, $-O(CO)OR^9$, $-O(CO)NR^6R^7$, $-NR^6R^7$, -(lower alkylene)-$COOR^6$ or $-CH=CH-COOR^6$, wherein $R^6$ and $R^7$ are preferably independently hydrogen or lower alkyl, and $R^9$ is preferably lower alkyl. A more preferred definition for $R^5$ is $-OR^6$, -(lower alkylene)-$COOR^6$ or $-CH=CH-COOR^6$, wherein $R^6$ is preferably hydrogen or lower alkyl.

Methods for making compounds of Formula III are well known to those skilled in the art. Non-limiting examples of suitable methods are disclosed in U.S. Patent No. 5,688,990, which is incorporated herein by reference.

In another embodiment, sterol absorption inhibitors useful in the methods of the present invention are represented by Formula (IV):

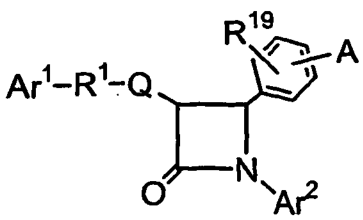

(IV)

or a pharmaceutically acceptable salt thereof or a solvate thereof, wherein, in Formula (IV) above:

A is selected from the group consisting of $R^2$-substituted heterocycloalkyl, $R^2$-substituted heteroaryl, $R^2$-substituted benzofused heterocycloalkyl, and $R^2$-substituted benzofused heteroaryl;

$Ar^1$ is aryl or $R^3$-substituted aryl;

$Ar^2$ is aryl or $R^4$-substituted aryl;

Q is a bond or, with the 3-position ring carbon of the azetidinone, forms the spiro group

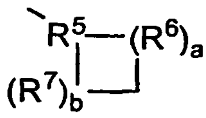;

and

$R^1$ is selected from the group consisting of:

-$(CH_2)_q$-, wherein q is 2-6, provided that when Q forms a spiro ring, q can also be zero or 1;
-$(CH_2)_e$-G-$(CH_2)_r$-, wherein G is -O-, -C(O)-, phenylene, -$NR^8$- or -$S(O)_{0-2}$-, e is 0-5 and r is 0-5, provided that the sum of e and r is 1-6; .
-($C_2$-$C_6$ alkenylene)-; and
-$(CH_2)_f$-V-$(CH_2)_g$-, wherein V is $C_3$-$C_6$ cycloalkylene, f is 1-5 and g is 0-5, provided that the sum of f and g is 1-6;

$R^5$ is selected from:

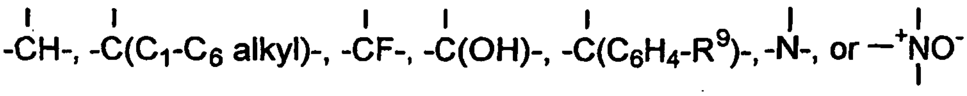;

$R^6$ and $R^7$ are independently selected from the group consisting of -$CH_2$-, -CH($C_1$-$C_6$ alkyl)-, -C(di-($C_1$-$C_6$) alkyl), -CH=CH- and -C($C_1$-$C_6$ alkyl)=CH-; or $R^5$ together with an adjacent $R^6$, or $R^5$ together with an adjacent $R^7$, form a -CH=CH- or a -CH=C($C_1$-$C_6$ alkyl)- group;

a and b are independently 0,1, 2 or 3, provided both are not zero; provided that when $R^6$ is -CH=CH- or -C($C_1$-$C_6$ alkyl)=CH-, a is 1; provided that when $R^7$ is -CH=CH- or -C($C_1$-$C_6$ alkyl)=CH-, b is 1; provided that when a is 2 or 3, the $R^6$'s can be the same or different; and provided that when b is 2 or 3, the $R^7$'s can be the same or different; and when Q is a bond, $R^1$ also can be selected from:

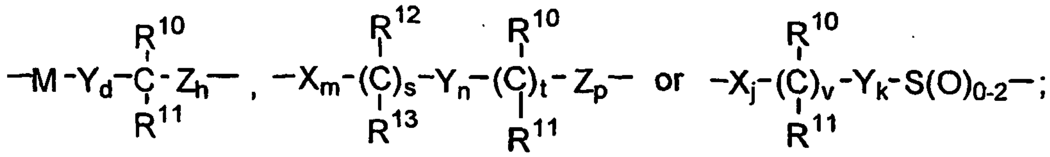

where M is -O-, -S-, -S(O)- or -$S(O)_2$-;

X, Y and Z are independently selected from the group consisting of -$CH_2$-, -CH($C_1$-$C_6$ alkyl)- and -C(di-($C_1$-$C_6$) alkyl);

$R^{10}$ and $R^{12}$ are independently selected from the group consisting of -$OR^{14}$, -O(CO)$R^{14}$, -O(CO)O$R^{16}$ and -O(CO)N$R^{14}R^{15}$;

$R^{11}$ and $R^{13}$ are independently selected from the group consisting of hydrogen, ($C_1$-$C_6$)alkyl and aryl; or $R^{10}$ and $R^{11}$ together are =O, or $R^{12}$ and $R^{13}$ together are =O;

d is 1, 2 or 3;

h is 0, 1, 2, 3 or 4;

s is 0 or 1; t is 0 or 1; m, n and p are independently 0-4; provided that at least one of s and t is 1, and the sum of m, n, p, s and t is 1-6; provided that when p is 0 and t is 1; the sum of m, s and n is 1-5; and provided that when p is 0 and s is 1, the sum of m, t and n is 1-5;

v is 0 or 1;

j and k are independently 1-5, provided that the sum of j, k and v is 1-5;

$R^2$ is 1-3 substituents on the ring carbon atoms selected from the group consisting of hydrogen, ($C_1$-$C_{10}$)alkyl, ($C_2$-$C_{10}$)alkenyl, ($C_2$-$C_{10}$)alkynyl, ($C_3$-$C_6$)cycloalkyl, ($C_3$-$C_6$)cycloalkenyl, $R^{17}$-substituted aryl, $R^{17}$-substituted benzyl, $R^{17}$-substituted benzyloxy, $R^{17}$-substituted aryloxy, halogeno, -$NR^{14}R^{15}$, $NR^{14}R^{15}$($C_1$-$C_6$ alkylene)-, $NR^{14}R^{15}$C(O)($C_1$-$C_6$ alkylene)-, -NHC(O)$R^{16}$, OH, $C_1$-$C_6$ alkoxy, -OC(O)$R^{16}$,
-$COR^{14}$, hydroxy($C_1$-$C_6$)alkyl, ($C_1$-$C_6$)alkoxy($C_1$-$C_6$)alkyl, $NO_2$, -$S(O)_{0-2}R^{16}$,-$SO_2NR^{14}R^{15}$ and -($C_1$-$C_6$ alkylene)$COOR^{14}$; when $R^2$ is a substituent on a heterocycloalkyl ring, $R^2$ is as defined, or is =O or

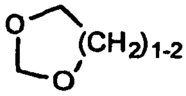 ;

and, where $R^2$ is a substituent on a substitutable ring nitrogen, it is hydrogen, $(C_1\text{-}C_6)$alkyl, aryl, $(C_1\text{-}C_6)$alkoxy, aryloxy, $(C_1\text{-}C_6)$alkylcarbonyl, arylcarbonyl, hydroxy, $-(CH_2)_{1\text{-}6}CONR^{18}R^{18}$,

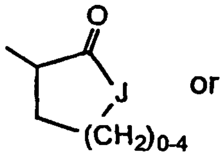 or 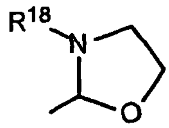 ;

wherein J is -O-, -NH-, $-NR^{18}-$ or $-CH_2-$;

$R^3$ and $R^4$ are independently selected from the group consisting of 1-3 substituents independently selected from the group consisting of $(C_1\text{-}C_6)$alkyl, $-OR^{14}$, $-O(CO)R^{14}$, $-O(CO)OR^{16}$, $-O(CH_2)_{1\text{-}5}OR^{14}$, $-O(CO)NR^{14}R^{15}$, $NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0\text{-}2}R^{16}$, $-O(CH_2)_{1\text{-}10}\text{-}COOR^{14}$, $-O(CH_2)_{1\text{-}10}CONR^{14}R^{15}$, $-(C_1\text{-}C_6$ alkylene)$-COOR^{14}$, $-CH{=}CH\text{-}COOR^{14}$, $-CF_3$, -CN,$-NO_2$ and halogen;

$R^8$ is hydrogen, $(C_1\text{-}C_6)$alkyl, aryl $(C_1\text{-}C_6)$alkyl, $-C(O)R^{14}$ or $-COOR^{14}$;

$R^9$ and $R^{17}$ are independently 1-3 groups independently selected from the group consisting of hydrogen, $(C_1\text{-}C_6)$ alkyl, $(C_1\text{-}C_6)$alkoxy, -COOH, $NO_2$, $-NR^{14}R^{15}$, OH and halogeno;

$R^{14}$ and $R^{15}$ are independently selected from the group consisting of hydrogen, $(C_1\text{-}C_6)$alkyl, aryl and aryl-substituted $(C_1\text{-}C_6)$alkyl;

$R^{16}$ is $(C_1\text{-}C_6)$alkyl, aryl or $R^{17}$ -substituted aryl;

$R^{18}$ is hydrogen or $(C_1\text{-}C_6)$alkyl; and

$R^{19}$ is hydrogen, hydroxy or $(C_1\text{-}C_6)$alkoxy.

As used in Formula (IV) above, "A" is preferably an $R^2$-substituted, 6-membered heterocycloalkyl ring containing 1 or 2 nitrogen atoms. Preferred heterocycloalkyl rings are piperidinyl, piperazinyl and morpholinyl groups. The ring "A" is preferably joined to the phenyl ring through a ring nitrogen. Preferred $R^2$ substituents are hydrogen and lower alkyl. $R^{19}$ is preferably hydrogen.

$Ar^2$ is preferably phenyl or $R^4$-phenyl, especially $(4\text{-}R^4)$-substituted phenyl. Preferred definitions of $R^4$ are lower alkoxy, especially methoxy, and halogeno, especially fluoro.

$Ar^1$ is preferably phenyl or $R^3$-substituted phenyl, especially $(4\text{-}R^3)$-substituted phenyl.

There are several preferred definitions for the $-R^1\text{-}Q-$ combination of variables:

Q is a bond and $R^1$ is lower alkylene, preferably propylene;

Q is a spiro group as defined above, wherein preferably $R^6$ and $R^7$ are each ethylene and $R^5$ is

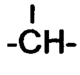 or 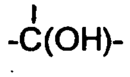 ;

Q is a bond and $R^1$ is

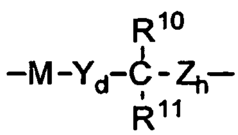

wherein the variables are chosen such that $R^1$ is $-O-CH_2-CH(OH)-$;

Q is a bond and $R^1$ is

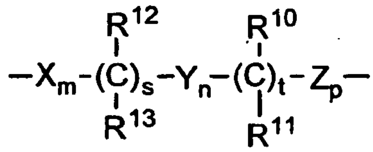

wherein the variables are chosen such that $R^1$ is $-CH(OH)-(CH_2)_2-$; and

Q is a bond and $R^1$ is

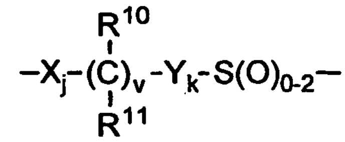

wherein the variables are chosen such that $R^1$ is $-CH(OH)-CH_2-S(O)-_{0-2}-$.

Methods for making compounds of Formula IV are well known to those skilled in the art. Non-limiting examples of suitable methods are disclosed in U.S. Patent No. 5,656,624, which is incorporated herein by reference.

In another embodiment, sterol absorption inhibitors useful in the methods of the present invention are represented by Formula (V):

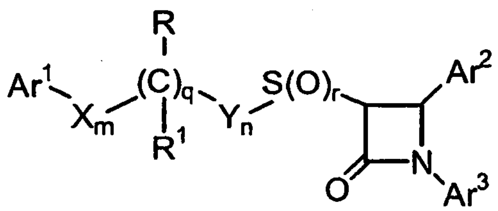

(V)

or a pharmaceutically acceptable salt thereof or a solvate thereof, wherein, in Formula (V) above:

$Ar^1$ is aryl, $R^{10}$-substituted aryl or heteroaryl;
$Ar^2$ is aryl or $R^4$-substituted aryl;
$Ar^3$ is aryl or $R^5$-substituted aryl;
X and Y are independently selected from the group consisting of $-CH_2-$, -CH(lower alkyl)- and -C(dilower alkyl)-;
R is $-OR^6$, $-O(CO)R^6$, $-O(CO)OR^9$ or $-O(CO)NR^6R^7$; $R^1$ is hydrogen, lower alkyl or aryl; or R and $R^1$ together are =O;
q is 0 or 1;
r is 0, 1 or 2;
m and n are independently 0, 1, 2, 3, 4 or 5; provided that the sum of m, n and q is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, halogen, - (lower alkylene)$COOR^6$ and $-CH=CH-COOR^6$;

$R^6$, $R^7$ and $R^8$ are independently selected from the group consisting of hydrogen, lower alkyl, aryl and aryl-substituted lower alkyl;

$R^9$ is lower alkyl, aryl or aryl-substituted lower alkyl; and

$R^{10}$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, $-CF_3$, -CN, $-NO_2$ and halogen.

Within the scope of Formula V, there are included two preferred structures. In Formula VA, q is zero and the remaining variables are as defined above, and in Formula VB, q is 1 and the remaining variables are as defined above:

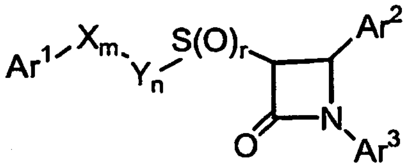

VA

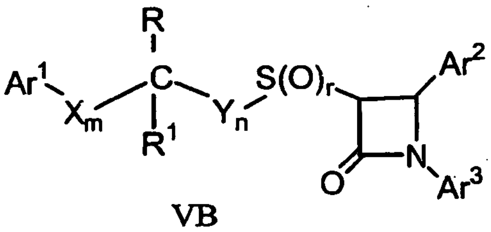

VB

$R^4$, $R^5$ and $R^{10}$ are each preferably 1-3 independently selected substituents as set forth above. Preferred are compounds of Formula (V) wherein $Ar^1$ is phenyl, $R^{10}$-substituted phenyl or thienyl, especially (4-$R^{10}$)-substituted phenyl or thienyl. $A^2$ is preferably $R^4$-substituted phenyl, especially (4-$R^4$)-substituted phenyl. $Ar^3$ is preferably phenyl or $R^5$-substituted phenyl, especially (4-$R^5$)-substituted phenyl. When $Ar^1$ is $R^{10}$-substituted phenyl, $R^{10}$ is preferably halogeno, especially fluoro. When $Ar^2$ is $R^4$-substituted phenyl, $R^4$ is preferably $-OR^6$, especially wherein $R^6$ is hydrogen or lower alkyl. When $Ar^3$ is $R^5$-substituted phenyl, $R^5$ is preferably halogeno, especially fluoro. Especially preferred are compounds of Formula (V) wherein $Ar^1$ is phenyl, 4-fluorophenyl or thienyl, $Ar^2$ is 4-(alkoxy or hydroxy)phenyl, and $Ar^3$ is phenyl or 4-fluorophenyl.

X and Y are each preferably $-CH_2-$. The sum of m, n and q is preferably 2, 3 or 4, more preferably 2. When q is 1, n is preferably 1 to 5.

Preferences for X, Y, $Ar^1$, $Ar^2$ and $Ar^3$ are the same in each of Formulae (VA) and (VB).

In compounds of Formula (VA), the sum of m and n is preferably 2, 3 or 4, more preferably 2. Also preferred are compounds wherein the sum of m and n is 2, and r is 0 or 1.

In compounds of Formula (VB), the sum of m and n is preferably 1, 2 or 3, more preferably 1. Especially preferred are compounds wherein m is zero and n is 1. $R^1$ is preferably hydrogen and R is preferably $-OR^6$ wherein $R^6$ is hydrogen, or a group readily metabolizable to a hydroxyl (such as $-O(CO)R^6$, $-O(CO)OR^9$ and $-O(CO)NR^6R^7$, defined above), or R and $R^1$ together form a =O group.

Methods for making compounds of Formula V are well known to those skilled in the art. Non-limiting examples of suitable methods are disclosed in U.S. Patent No. 5,624,920, which is incorporated herein by reference.

In another embodiment, sterol absorption inhibitors useful in the methods of the present invention are represented by Formula (VI):

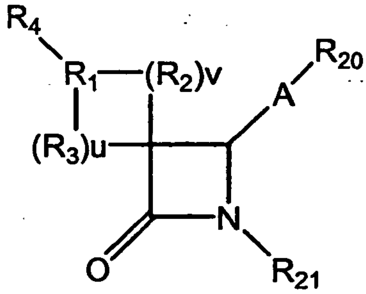

(VI)

or a pharmaceutically acceptable salt thereof or a solvate thereof, wherein:

$R_1$ is

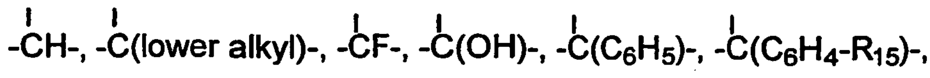

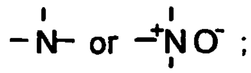

$R_2$ and $R_3$ are independently selected from the group consisting of: $-CH_2-$, -CH(lower alkyl)-, -C(di-lower alkyl)-, -CH=CH- and -C(lower alkyl)=CH-; or $R_1$ together with an adjacent $R_2$, or $R_1$ together with an adjacent $R_3$, form a -CH=CH- or a -CH=C(lower alkyl)- group;

u and v are independently 0,1, 2 or 3, provided both are not zero; provided that when $R_2$ is -CH=CH- or -C(lower alkyl)=CH-, v is 1; provided that when $R_3$ is-CH=CH- or -C(lower alkyl)=CH-, u is 1; provided that when v is 2 or 3, the $R_2$'s can be the same or different; and provided that when u is 2 or 3, the $R_3$'s can be the same or different;

$R_4$ is selected from $B-(CH_2)_mC(O)-$, wherein m is 0, 1, 2, 3,4 or 5; $B-(CH_2)_q-$, wherein q is 0, 1, 2, 3, 4, 5 or 6; $B-(CH_2)_e-Z-(CH_2)_r-$, wherein Z is -O-, -C(O)-, phenylene, $-N(R_8)-$ or $-S(O)_{0-2}-$, e is 0, 1, 2, 3, 4 or 5 and r is 0, 1, 2, 3, 4 or 5, provided that the sum of e and r is 0, 1, 2, 3, 4, 5 or 6; B-($C_2$-$C_6$ alkenylene)-; B-($C_4$-$C_6$ alkadienylene)-; $B-(CH_2)_t-Z-$($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6; $B-(CH_2)_f-V-(CH_2)_g-$, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6; $B-(CH_2)_t-V-$($C_2$-$C_6$ alkenylene)- or B-($C_2$-$C_6$ alkenylene $V-(CH_2)_t-$, wherein V and t are as defined above, provided that the sum of t and the number of carbon atoms in the alkenylene chain is 2, 3, 4, 5 or 6;

$B-(CH_2)_a-Z-(CH_2)_b-V-(CH_2)_d-$, wherein Z and V are as defined above and a, b and d are independently 0, 1, 2, 3, 4, 5 or 6, provided that the sum of a, b and d is 0, 1, 2, 3, 4, 5 or 6; or $T-(CH_2)_s-$, wherein T is cycloalkyl of 3-6 carbon atoms and s is 0, 1, 2, 3, 4, 5 or 6; or

$R_1$ and $R_4$ together form the group

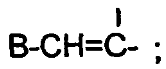

B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or

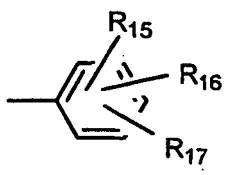

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, $-CF_3$, $-OCF_3$, benzyl, $R_7$-benzyl, benzyloxy, $R_7$-benzyloxy, phenoxy, $R_7$-phenoxy, dioxolanyl, $NO_2$, $-N(R_8)(R_9)$, $N(R_8)(R_9)$-lower alkylene-, $N(R_8)(R_9)$-lower alkylenyloxy-, OH, halogeno, -CN, $-N_3$, $-NHC(O)OR_{10}$, $-NHC(O)R_{10}$, $R_{11}O_2SNH-$, $(R_{11}O_2S)_2N-$, $-S(O)_2NH_2$, $-S(O)_{0-2}R_8$, tert-butyldimethyl-silyloxymethyl, $-C(O)R_{12}$, $-COOR_{19}$, $-CON(R_8)(R_9)$, $-CH=CHC(O)R_{12}$, -lower alkylene-$C(O)R_{12}$, $R_{10}C(O)$(lower alkylenyloxy)-, N $(R_8)(R_9)C(O)$(lower alkylenyloxy)- and

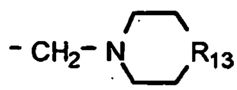

for substitution on ring carbon atoms, and the substituents on the substituted heteroaryl ring nitrogen atoms, when present, are selected from the group consisting of lower alkyl, lower alkoxy, $-C(O)OR_{10}$, $-C(O)R_{10}$, OH, $N(R_8)(R_9)$-lower alkylene-, $N(R_8)(R_9)$-lower alkylenyloxy-, $-S(O)_2NH_2$ and 2-(trimethylsilyl)-ethoxymethyl;

$R_7$ is 1-3 groups independently selected from the group consisting of lower alkyl, lower alkoxy, -COOH, $NO_2$, $-N(R_8)(R_9)$, OH, and halogeno;

$R_8$ and $R_9$ are independently selected from H or lower alkyl;

$R_{10}$ is selected from lower alkyl, phenyl, $R_7$-phenyl, benzyl or $R_7$-benzyl;

$R_{11}$ is selected from OH, lower alkyl, phenyl, benzyl, $R_7$-phenyl or $R_7$-benzyl;

$R_{12}$ is selected from H, OH, alkoxy, phenoxy, benzyloxy,

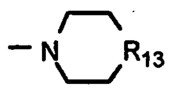

,

$-N(R_8)(R_9)$, lower alkyl, phenyl or $R_7$-phenyl;

$R_{13}$ is selected from -O-, $-CH_2-$, -NH-, -N(lower alkyl)- or $-NC(O)R_{19}$;

$R_{15}$, $R_{16}$ and $R_{17}$ are independently selected from the group consisting of H and the groups defined for W; or $R_{15}$ is hydrogen and $R_{16}$ and $R_{17}$, together with adjacent carbon atoms to which they are attached, form a dioxolanyl ring;

$R_{19}$ is H, lower alkyl, phenyl or phenyl lower alkyl; and

$R_{20}$ and $R_{21}$ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above.

One group of preferred compounds of Formula VI is that in which $R_{21}$ is selected from phenyl, W-substituted phenyl, indanyl, benzofuranyl, benzodioxolyl, tetrahydronaphthyl, pyridyl, pyrazinyl, pyrimidinyl, quinolyl or cyclopropyl, wherein W is lower alkyl, lower alkoxy, OH, halogeno, $-N(R_8)(R_9)$, $-NHC(O)OR_{10}$, $-NHC(O)R_{10}$, $NO_2$, -CN, $-N_3$, -SH, $-S(O)_{0-2}$-(lower alkyl), $-COOR_{19}$, $-CON(R_8)(R_9)$, $-COR_{12}$, phenoxy, benzyloxy, $-OCF_3$, $-CH=C(O)R_{12}$ or tert-butyldimethylsilyloxy, wherein $R_8$, $R_9$, $R_{10}$, $R_{12}$ and $R_{19}$ are as defined for Formula IV. When W is 2 or 3 substituents, the substituents can be the same or different.

Another group of preferred compound of Formula VI is that in which $R_{20}$ is phenyl or W-substituted phenyl, wherein preferred meanings of W are as defined above for preferred definitions of $R_{21}$.

More preferred are compounds of Formula VI wherein $R_{20}$ is phenyl or W-substituted phenyl and $R_{21}$ is phenyl, W-substituted phenyl, indanyl, benzofuranyl, benzodioxolyl, tetrahydronaphthyl, pyridyl, pyrazinyl, pyrimidinyl, quinolyl or cyclopropyl; W is lower alkyl, lower alkoxy, OH, halogeno, $-N(R_8)(R_9)$, $-NHC(O)OR_{10}$, $-NHC(O)R_{10}$, $NO_2$, -CN, $-N_3$, -SH, $-S(O)_{0-2}$-(lower alkyl), $-COOR_{19}$, $-CON(R_8)(R_9)$, $-COR_{12}$, phenoxy, benzyloxy, $-CH=CHC(O)R_{12}$, $-OCF_3$ or tert-butyldimethyl-silyloxy, wherein when W is 2 or 3 substituents, the substituents can be the same or different, and wherein $R_8$, $R_9$, $R_{10}$, $R_{12}$ and $R_{19}$ are as defined in Formula VI.

Also preferred are compounds of Formula VI wherein $R_1$ is

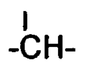 or 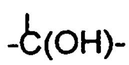 .

Another group of preferred compounds of Formula VI is in which $R_2$ and $R_3$ are each $-CH_2-$ and the sum of u and v is 2, 3 or 4, with u=v=2 being more preferred.

$R_4$ is preferably $B-(CH_2)q-$ or $B-(CH_2)_e-Z-(CH_2)_r-$, wherein B, Z, q, e and r are as defined above. B is preferably

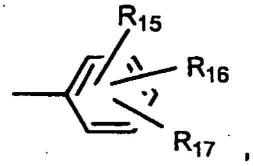

wherein $R_{16}$ and $R_{17}$ are each hydrogen and wherein $R_{15}$ is preferably H, OH, lower alkoxy, especially methoxy, or halogeno, especially chloro.

Preferably Z is -O-, e is 0, and r is 0.

Preferably q is 0-2.

$R_{20}$ is preferably phenyl or W-substituted phenyl.

Preferred W substituents for $R_{20}$ are lower alkoxy, especially methoxy and ethoxy, OH, and $-C(O)R_{12}$, wherein $R_{12}$ is preferably lower alkoxy.

Preferably $R_{21}$ is selected from phenyl, lower alkoxy-substituted phenyl and F-phenyl.

Especially preferred are compounds of Formula VI wherein $R_1$ is

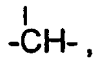

or

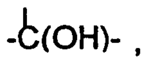

$R_2$ and $R_3$ are each $-CH_2-$, u=v=2, $R_4$ is $B-(CH_2)_q-$, wherein B is phenyl or phenyl substituted by lower alkoxy or chloro, q is 0-2, $R_{20}$ is phenyl, OH-phenyl, lower alkoxy-substituted phenyl or lower alkoxycarbonyl-substituted phenyl, and $R_{21}$ is phenyl, lower alkoxy-substituted phenyl or
F-phenyl.

Methods for making compounds of Formula VI are well known to those skilled in the art. Non-limiting examples of suitable methods are disclosed in U.S. Patent No. 5,698,548, which is incorporated herein by reference.

In another embodiment, sterol absorption inhibitors useful in the methods of the present invention are represented by Formulas (VIIA) and (VIIB):

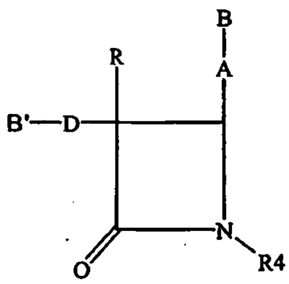

(VIIA)

and

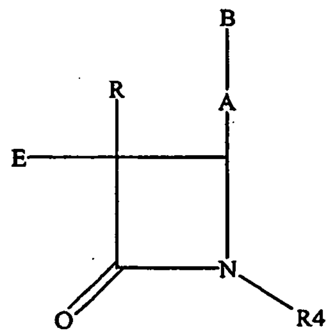

(VIIB)

or a pharmaceutically acceptable salt or solvate thereof,
wherein:

A is -CH=CH-, -C≡C- or $-(CH_2)_p-$ wherein p is 0, 1 or 2;
B is

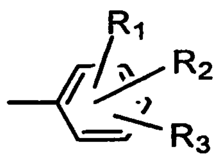

B' is

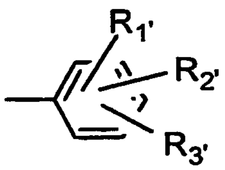

D is $-(CH_2)_mC(O)-$ or $-(CH_2)_q-$ wherein m is 1, 2, 3 or 4 and q is 2, 3 or 4;
E is $C_{10}$ to $C_{20}$ alkyl or -C(O)-($C_9$ to $C_{19}$)-alkyl, wherein the alkyl is straight or branched, saturated or containing one or more double bonds;
R is hydrogen, $C_1$-$C_{15}$ alkyl, straight or branched, saturated or containing one or more double bonds, or $B-(CH_2)_r$-, wherein r is 0, 1, 2, or 3;
$R_1$, $R_2$, $R_3$, $R_{1'}$, $R_{2'}$, and $R_{3'}$ are independently selected from the group consisting of hydrogen, lower alkyl, lower alkoxy, carboxy, $NO_2$, $NH_2$, OH, halogeno, lower alkylamino, dilower alkylamino, $-NHC(O)OR_5$, $R_6O_2SNH$- and $-S(O)_2NH_2$;
$R_4$ is

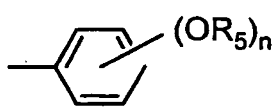

wherein n is 0, 1, 2 or 3;
$R_5$ is lower alkyl; and

$R_6$ is OH, lower alkyl, phenyl, benzyl or substituted phenyl wherein the substituents are 1-3 groups independently selected from the group consisting of lower alkyl, lower alkoxy, carboxy, $NO_2$, $NH_2$, OH, halogeno, lower alkylamino and dilower alkylamino; or a pharmaceutically acceptable salt thereof or a prodrug thereof.

Preferred are compounds of Formula (VIIA) wherein R is hydrogen, saturated or mono-unsaturated $C_1$ -$C_{10}$ alkyl or phenyl. Another group of preferred compounds of Formula (VIIA) is that in which D is propyl (i.e., -$(CH_2)$q- and q is 3). A third group of preferred compounds of Formula (VIIA) is that wherein $R_4$ is p-methoxyphenyl or 2,4,6-trimethoxyphenyl. Still another group of preferred compounds of Formula (VIIA) is that wherein A is ethylene or a bond (i.e., -($CH_2$ )p- wherein p is zero). $R_{1'}$, $R_{2'}$, and $R_{3'}$ are preferably each hydrogen, and preferably $R_1$ is hydrogen, hydroxy, nitro, lower alkoxy, amino or t-butoxycarbonyl-amino and $R_2$ and $R_3$ are each hydrogen.

More preferred are compounds of Formula (VIIA) wherein $R_{1'}$, $R_{2'}$, and $R_{3'}$ are each hydrogen; $R_1$ is hydrogen, hydroxy, nitro, lower alkoxy, amino or t-butoxycarbonyl-amino and $R_2$ and $R_3$ are each hydrogen; R is hydrogen, ethyl or phenyl; D is propyl; $R_4$ is p-methoxyphenyl or 2,4,6-trimethoxyphenyl; and A is ethylene or a bond.

Preferred compounds of Formula (VIIA), wherein B' is phenyl, are shown in the following table:

| D | R | A | B | R4 |
|---|---|---|---|---|
| -$(CH_2)_3$- | H | --- | p-MeO-phenyl | p-MeO-phenyl |
| -$CH_2C(O)$- | phenyl | --- | phenyl | p-MeO-phenyl |
| -$(CH_2)_3$- | H | --- | phenyl | p-MeO-phenyl |
| -$(CH_2)_3$- | H | --- | p-OH-phenyl | p-MeO-phenyl |
| -$(CH_2)_3$- | H | ethylene | p-MeO-phenyl | p-MeO-phenyl |
| -$(CH_2)_3$- | H | --- | 3-MeO-phenyl | p-MeO-phenyl |
| -$(CH_2)_3$- | ethyl | --- | phenyl | p-MeO-phenyl |
| -$(CH_2)_3$- | phenyl | --- | phenyl | p-MeO-phenyl |
| -$(CH_2)_3$- | ethyl | --- | phenyl | 2,4,6-tri-MeO-phenyl |
| -$(CH_2)_3$- | methyl | --- | phenyl | p-MeO-phenyl |
| -$(CH_2)_3$- | H | --- | p-$NH_2$-phenyl | p-MeO-phenyl |

The first-listed compound in the above table having the (3R,4S) absolute stereochemistry is more preferred.

Preferred compounds of Formula (VIIB) are those wherein R is hydrogen, methyl, ethyl, phenyl or phenylpropyl. Another group of preferred compounds of Formula (VIIB) is that wherein $R_4$ is p-methoxyphenyl or 2,4,6-trimethoxyphenyl. Still another group of preferred compounds of Formula (VIB) is that wherein A is ethylene or a bond. Yet another group of preferred compounds of Formula (VIIB) is that wherein E is decyl, oleoyl or 7-Z-hexadecenyl. Preferably $R_1$, $R_2$ and $R_3$ are each hydrogen.

More preferred compounds of Formula (VIIB) are those wherein R is hydrogen, methyl, ethyl, phenyl or phenylpropyl; $R_4$ is p-methoxyphenyl or 2,4,6-trimethoxyphenyl; A is ethylene or a bond; E is decyl, oleoyl or 7-Z-hexadecenyl; and $R_1$, $R_2$ and $R_3$ are each hydrogen.

A preferred compound of Formula (VIIB) is that wherein E is decyl, R is hydrogen, B-A is phenyl and $R_4$ is p-methoxyphenyl.

In another embodiment, sterol absorption inhibitors useful in the methods of the present invention are represented by Formula (VIII):

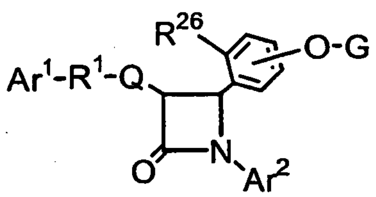

(VIII)

or a pharmaceutically acceptable salt thereof or a solvate thereof, wherein, in Formula (VIII) above,

$R^{26}$ is H or $OG^1$ ;

G and $G^1$ are independently selected from the group consisting of H,

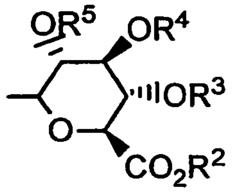 , 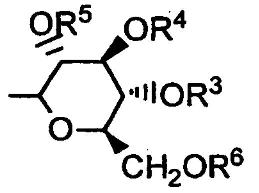 , 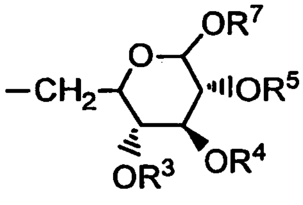

and

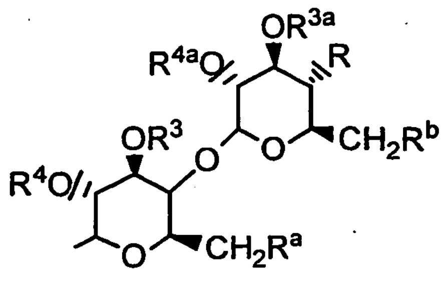 ;

provided that when $R^{26}$ is H or OH, G is not H;

R, $R^a$ and $R^b$ are independently selected from the group consisting of H, -OH, halogeno, $-NH_2$, azido, $(C_1-C_6)$alkoxy $(C_1-C_6)$-alkoxy or $-W-R^{30}$;

W is independently selected from the group consisting of -NH-C(O)-,- O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_1-C_6)$alkyl, aryl and aryl$(C_1-C_6)$alkyl;

$R^3$, $R^4$, $R^5$, $R^7$, $R^{3a}$ and $R^{4a}$ are independently selected from the group consisting of H, $(C_1-C_6)$alkyl, aryl$(C_1-C_6)$ alkyl, $-C(O)(C_1-C_6)$alkyl and -C(O)aryl;

$R^{30}$ is selected from the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3-C_7)$cycloalkyl and $R^{32}$-substituted-$(C_3$-C7)cycloalkyl$(C_1-C_6)$alkyl;

$R^{31}$ is selected from the group consisting of H and $(C_1-C_4)$alkyl; T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl,

$-C(O)-N((C_1-C_4)alkyl)_2$, $-C(O)(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is a bond or, with the 3-position ring carbon of the azetidinone, forms the spiro group

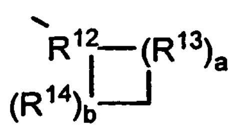 ;

and

$R^1$ is selected from the group consisting of

$-(CH_2)_q-$, wherein q is 2-6, provided that when Q forms a spiro ring, q can also be zero or 1;

$-(CH_2)_e-E-(CH_2)_r-$, wherein E is -O-, -C(O)-, phenylene, $-NR^{22}-$ or $-S(O)_{0-2}-$, e is 0-5 and r is 0-5, provided that

the sum of e and r is 1-6;
$-(C_2-C_6)$alkenylene-; and
$-(CH_2)_f-V-(CH_2)_g-$, wherein V is $C_3-C_6$ cycloalkylene, f is 1-5 and g is 0-5, provided that the sum of f and g is 1-6;

$R^{12}$ is

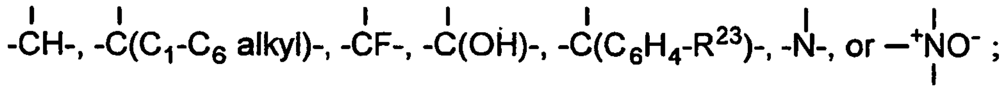

$R^{13}$ and $R^{14}$ are independently selected from the group consisting of $-CH_2-$, $-CH(C_1-C_6$ alkyl)-, -C(di-$(C_1-C_6)$ alkyl), -CH=CH- and $-C(C_1-C_6$ alkyl)=CH-; or $R^{12}$ together with an adjacent $R^{13}$, or $R^{12}$ together with an adjacent $R^{14}$, form a -CH=CH- or a $-CH=C(C_1-C_6$ alkyl)- group;
a and b are independently 0, 1, 2 or 3, provided both are not zero;
provided that when $R^{13}$ is -CH=CH- or $-C(C_1-C_6$ alkyl)=CH-, a is 1;
provided that when $R^{14}$ is -CH=CH- or $-C(C_1-C_6$ alkyl)=CH-, b is 1;
provided that when a is 2 or 3, the $R^{13}$'s can be the same or different; and
provided that when b is 2 or 3, the $R^{14}$'s can be the same or different;
and when Q is a bond, $R^1$ also can be:

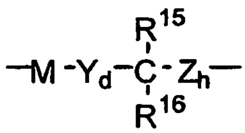 , 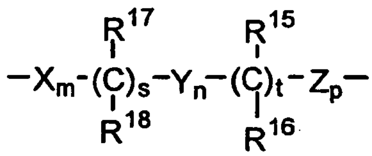 or 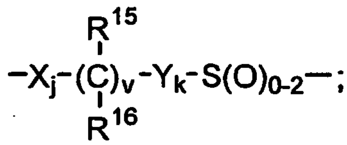 ;

M is -O-, -S-, -S(O)- or $-S(O)_2-$;
X, Y and Z are independently selected from the group consisting of $-CH_2-$, $-CH(C_1-C_6)$alkyl- and -C(di-$(C_1-C_6)$alkyl);
$R^{10}$ and $R^{11}$ are independently selected from the group consisting of 1-3 substituents independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO^2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene)-$COOR^{19}$, -CH=CH-$COOR^{19}$, $-CF_3$, -CN, $-NO_2$ and halogen;
$R^{15}$ and $R^{17}$are independently selected from the group consisting of $-OR^{19}$, $-O(CO)R^{19}$, $-O(CO)OR^{21}$ and -O(CO) $NR^{19}R^{20}$;
$R^{16}$ and $R^{18}$ are independently selected from the group consisting of H, $(C_1-C_6)$alkyl and aryl; or $R^{15}$ and $R^{16}$ together are =O, or $R^{17}$ and $R^{18}$ together are =O;
d is 1,2 or 3;
h is 0, 1, 2, 3 or 4;
s is 0 or 1; t is 0 or 1; m, n and p are independently 0-4;
provided that at least one of s and t is 1, and the sum of m, n, p, s and t is 1-6;
provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
and when Q is a bond and $R^1$ is

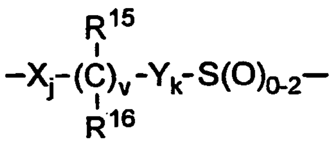 ,

$Ar^1$ can also be pyridyl, isoxazolyl, furanyl, pyrrolyl, thienyl, imidazolyl, pyrazolyl, thiazolyl, pyrazinyl, pyrimidinyl or pyridazinyl;

$R^{19}$ and $R^{20}$ are independently selected from the group consisting of H, $(C_1-C_6)$alkyl, aryl and aryl-substituted $(C_1-C_6)$ alkyl;
$R^{21}$ is $(C_1-C_6)$alkyl, aryl or $R^{24}$-substituted aryl;
$R^{22}$ is H, $(C_1-C_6)$alkyl, aryl $(C_1-C_6)$alkyl, $-C(O)R^{19}$ or $-COOR^{19}$;
$R^{23}$ and $R^{24}$ are independently 1-3 groups independently selected from the group consisting of H, $(C_1-C_6)$alkyl, $(C_1-C_6)$alkoxy, -COOH, $NO_2$, $-NR^{19}R^{20}$, -OH and halogeno; and
$R^{25}$ is H, -OH or $(C_1-C_6)$alkoxy.

$Ar^2$ is preferably phenyl or $R^{11}$-phenyl, especially (4-$R^{11}$)-substituted phenyl. Preferred definitions of $R^{11}$ are lower alkoxy, especially methoxy, and halogeno, especially fluoro.

$Ar^1$ is preferably phenyl or $R^{10}$-substituted phenyl, especially (4-$R^{10}$)-substituted phenyl. Preferably $R^{10}$ is halogeno, and more preferably fluoro.

There are several preferred definitions for the $-R^1$-Q- combination of variables:

Q is a bond and $R^1$ is lower alkylene, preferably propylene;

Q is a spiro group as defined above, wherein preferably $R^{13}$ and $R^{14}$ are each ethylene and $R^{12}$ is

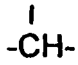 or 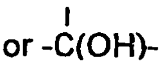 ,

and $R^1$ is $-(CH_2)_q$ wherein q is 0-6;

Q is a bond and $R^1$ is

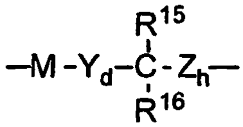

wherein the variables are chosen such that $R^1$ is $-O-CH_2-CH(OH)-$;

Q is a bond and $R^1$ is

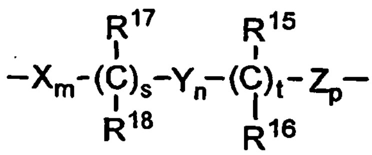

wherein the variables are chosen such that $R^1$ is $-CH(OH)-(CH_2)_2-$; and

Q is a bond and $R^1$ is

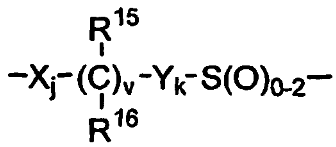

wherein the variables are chosen such that $R^1$ is $-CH(OH)-CH_2-S(O)_{0-2}-$.

A preferred compound of Formula (VIII) therefore, is one wherein G and $G^1$ are as defined above and in which

the remaining variables have the following definitions:

Ar$^1$ is phenyl or R$^{10}$-substituted phenyl, wherein R$^{10}$ is halogeno;
Ar$^2$ is phenyl or R$^{11}$-phenyl, wherein R$^{11}$ is 1 to 3 substituents independently selected from the group consisting of $C_1$-$C_6$ alkoxy and halogeno;
Q is a bond and R$^1$ is lower alkylene; Q, with the 3-position ring carbon of the azetidinone, forms the group

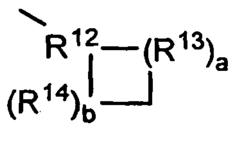

wherein preferably R$^{13}$ and R$^{14}$ are each ethylene and a and b are each 1, and wherein R$^{12}$ is

-CH- or -C(OH)- ;

Q is a bond and R$^1$ is -O-$CH_2$-CH(OH)-; Q is a bond and R$^1$ is -CH(OH)-$(CH_2)_2$-; or Q is a bond and R$^1$ is -CH(OH)-$CH_2$-$S(O)_{0-2}$-.

Preferred variables for G and G$^1$ groups of the formulae

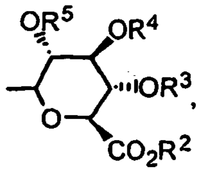,
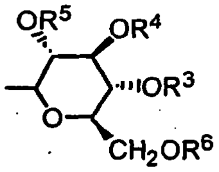
and
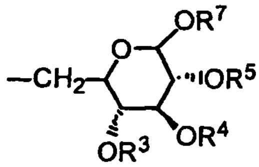

are as follows:

R$^2$, R$^3$, R$^4$, R$^5$, R$^6$ and R$^7$ are independently selected from the group consisting of H, ($C_1$-$C_6$)alkyl, benzyl and acetyl.

Preferred variables for group G or G$^1$ of the formula:

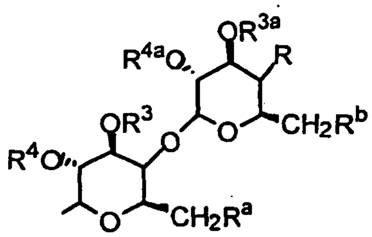

are as follows:

R$^3$, R$^{3a}$, R$^4$ and R$^{4a}$ are selected from the group consisting of H, ($C_1$-$C_6$)alkyl, benzyl and acetyl;
R, R$^a$ and R$^b$ are independently selected from the group consisting of H, -OH, halogeno, -$NH_2$, azido, ($C_1$-$C_6$)alkoxy ($C_1$-$C_6$)alkoxy and -W-R$^{30}$,

wherein W is -O-C(O)- or -O-C(O)-NR$^{31}$-, R$^{31}$ is H and R$^{30}$ is ($C_1$-$C_6$)alkyl, -C(O)-($C_1$-$C_4$)alkoxy-($C_1$-$C_6$)alkyl, T , T-($C_1$-$C_6$) alkyl, or T or T-($C_1$-$C_6$)alkyl wherein T is substituted by one or two halogeno or ($C_1$-$C_6$)alkyl groups.
Preferred R$^{30}$ substituents are selected from the group consisting of: 2-fluorophenyl, 2,4-difluoro-phenyl, 2,6-dichlorophenyl, 2-methylphenyl, 2-thienylmethyl, 2-methoxy-carbonylethyl, thiazol-2-yl-methyl, 2-furyl, 2-methoxycarbonylbutyl and phenyl.

Preferred combinations of R, $R^a$ and $R^b$ are as follows:

1) R, $R^a$ and $R^b$ are independently -OH or -O-C(O)-NH-$R^{30}$, especially wherein $R^a$ is -OH and R and $R^b$ are -O-C(O)-NH-$R^{30}$ and $R^{30}$ is selected from the preferred substituents identified above, or wherein R and $R^a$ are each -OH and $R^b$ is-O-C(O)-NH-$R^{30}$ wherein $R^{30}$ is 2-fluorophenyl, 2,4-difluoro-Phenyl, 2,6-dichlorophenyl;

2) $R^a$ is -OH, halogeno, azido or ($C_1$-$C_6$)-alkoxy($C_1$-$C_6$)alkoxy, $R^b$ is H, halogeno, azido or ($C_1$-$C_6$)alkoxy ($C_1$-$C_6$)-alkoxy, and R is -O-C(O)-NH-$R^{30}$, especially compounds wherein $R^a$ is -OH, $R^b$ is H and $R^{30}$ is 2-fluorophenyl;

3) R, $R^a$ and $R^b$ are independently -OH or -O-C(O)-$R^{30}$ and $R^{30}$ is ($C_1$-$C_6$)alkyl, T , or T substituted by one or two halogeno or ($C_1$-$C_6$)alkyl groups, especially compounds wherein R is -OH and $R^a$ and $R^b$ are -O-C(O)-$R^{30}$ wherein $R^{30}$ is 2-furyl; and

4) R, $R^a$ and $R^b$ are independently -OH or halogeno. Three additional classes of preferred compounds are those wherein the $C^{1'}$ anomeric oxy is beta, wherein the $C^{2'}$ anomeric oxy is beta, and wherein the R group is alpha. G and $G^1$ are preferably selected from:

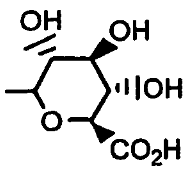 , 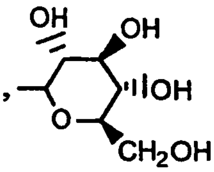 , 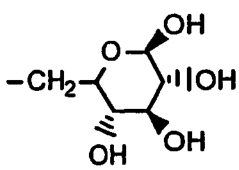 , 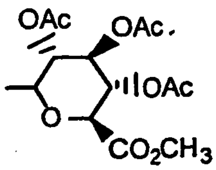 ,

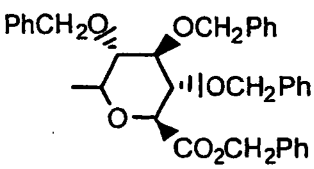 , 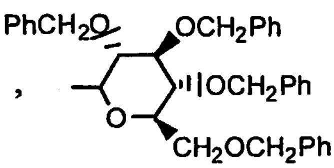 , 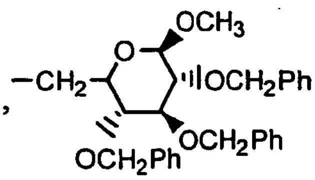 ,

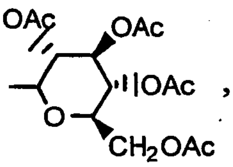 , 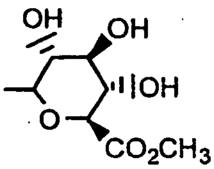 , 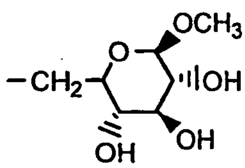 ,

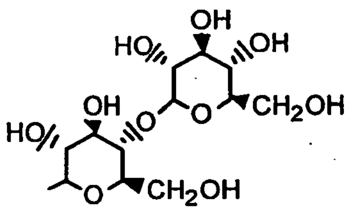 , 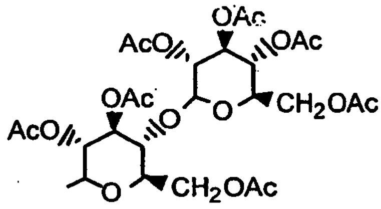

and

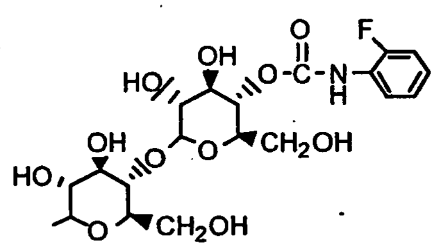

wherein Ac is acetyl and Ph is phenyl.

Preferably, $R^{26}$ is H or OH, more preferably H. The -O-G substituent is preferably in the 4-position of the phenyl ring to which it is attached.

In another embodiment, sterol inhibitors useful in the compositions and methods of the present invention are represented by Formula (IX) below:

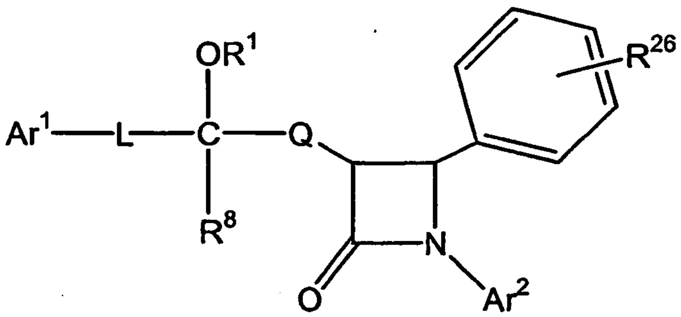

(IX)

or a pharmaceutically acceptable salt or solvate thereof, wherein in Formula (IX):

$R^1$ is selected from the group consisting of H, G, $G^1$, $G^2$, $-SO_3H$ and $-PO_3H$;

G is selected from the group consisting of: H,

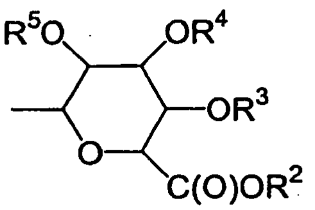 , 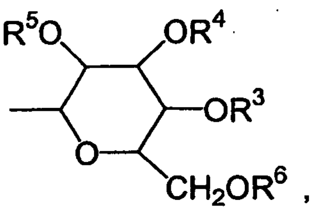 , 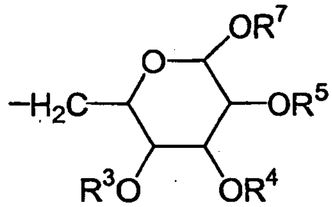 ,

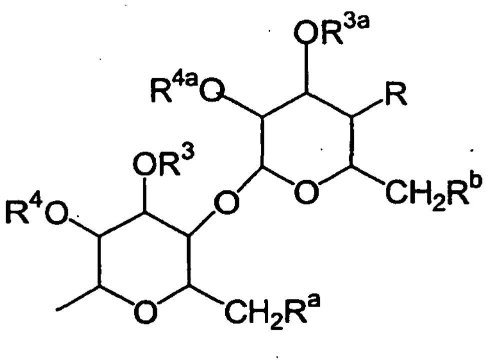

and

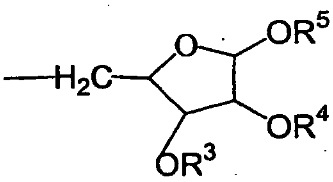

.

(sugar derivatives)

wherein R, $R^a$ and $R^b$ are each independently selected from the group consisting of H, -OH, halo, $-NH_2$, azido, $(C_1-C_6)$alkoxy$(C_1-C_6)$alkoxy or -W-$R^{30}$;

W is independently selected from the group consisting of -NH-C(O)-, -O-C(O)-, -O-C(O)-N($R^{31}$)-, -NH-C(O)-N($R^{31}$)- and -O-C(S)-N($R^{31}$)-;

$R^2$ and $R^6$ are each independently selected from the group consisting of H, $(C_1-C_6)$alkyl, acetyl, aryl and aryl$(C_1-C_6)$ alkyl;

R3, $R^4$, $R^5$, $R^7$, $R^{3a}$ and $R^{4a}$ are each independently selected from the group consisting of H, $(C_1-C_6)$alkyl, acetyl, aryl$(C_1-C_6)$alkyl, -C(O)$(C_1-C_6)$alkyl and-C(O)aryl;

$R^{30}$ is independently selected from the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3$-C7)cycloalkyl and $R^{32}$-substituted-$(C_3$-C7)cycloalkyl$(C_1-C_6)$alkyl;

$R^{31}$ is independently selected from the group consisting of H and $(C_1-C_4)$alkyl;

T is independently selected from the group consisting of phenyl, furyl, thienyl, pyrrolyl, oxazolyl, isoxazolyl, thiazolyl, isothiazolyl, benzothiazolyl, thiadiazolyl, pyrazolyl, imidazolyl and pyridyl;

$R^{32}$ is independently selected from 1-3 substituents which are each independently selected from the group consisting of H, halo, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, -C(O)-NH$(C_1-C_4)$alkyl, -C(O)-N($(C_1-C_4)$alkyl$)_2$, -C(O)-$(C_1-C_4)$ alkyl, -C(O)-$(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$ alkoxycarbonyl-substituted pyrrolidinyl, piperidinyl, N-methylpiperazinyl, indolinyl or morpholinyl group;

$G^1$ is represented by the structure:

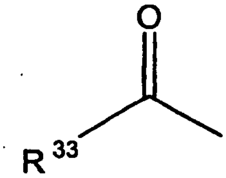

wherein $R^{33}$ is independently selected from the group consisting of unsubstituted alkyl, $R^{34}$-substituted alkyl, ($R^{35}$)($R^{36}$)alkyl-,

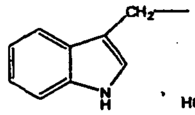, 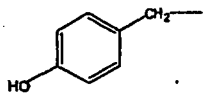, 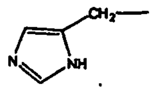, 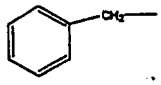,

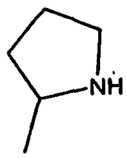 and 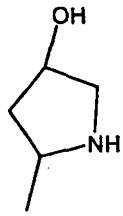 ;

$R^{34}$ is one to three substituents, each $R^{34}$ being independently selected from the group consisting of HOOC-, HS-, $(CH_3)S$-, $H_2N$-, $(NH_2)(NH)C(NH)$-, $(NH_2)C(O)$- and $HOOCCH(NH_3^+)CH_2SS$-;

$R^{35}$ is independently selected from the group consisting of H and $NH_2$-;

$R^{36}$ is independently selected from the group consisting of H, unsubstituted alkyl, $R^{34}$-substituted alkyl, unsubstituted cycloalkyl and $R^{34}$-substituted cycloalkyl;

$G^2$ is represented by the structure:

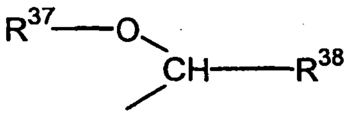

wherein $R^{37}$ and $R^{38}$ are each independently selected from the group consisting of $(C_1\text{-}C_6)$alkyl and aryl;

$R^{26}$ is one to five substituents, each $R^{26}$ being independently selected from the group consisting of:

a) H;
b) -OH;
c) $-OCH_3$;
d) fluorine;
e) chlorine;
f) -O-G;
g) $-O-G^1$;
h) $-O-G^2$;
i) $-SO_3H$; and
j) $-PO_3H$;

provided that when $R^1$ is H, $R^{26}$ is not H, -OH, $-OCH_3$ or -O-G;

$Ar^1$ is aryl, $R^{10}$-substituted aryl, heteroaryl or $R^{10}$-substituted heteroaryl;

$Ar^2$ is aryl, $R^{11}$-substituted aryl, heteroaryl or $R^{11}$-substituted heteroaryl;

L is selected from the group consisting of:

a) a covalent bond;
b) $-(CH_2)_q-$, wherein q is 1-6;

c) $-(CH_2)_e-E-(CH_2)_r-$, wherein E is -O-, -C(O)-, phenylene, $-NR^{22}-$ or $-S(O)_{0-2}-$, e is 0-5 and r is 0-5, provided that the sum of e and r is 1-6;
d) $-(C_2-C_6)$alkenylene-;
e) $-(CH_2)_f-V-(CH_2)_g-$, wherein V is $C_3-C_6$cycloalkylene, f is 1-5 and g is 0-5, provided that the sum of f and g is 1-6; and
f)

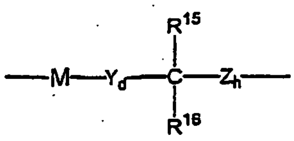
,
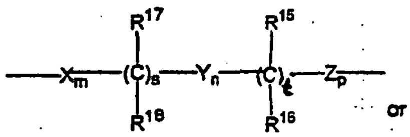
or
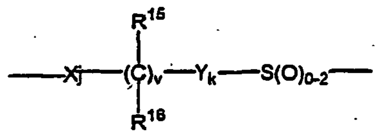
;

wherein M is -O-, -S-, -S(O)- or $-S(O)_2-$;

X, Y and Z are each independently selected from the group consisting of $-CH_2-$, $-CH(C_1-C_6)$alkyl- and $-C(di-(C_1-C_6)$ alkyl)-;

$R^8$ is selected from the group consisting of H and alkyl;

$R^{10}$ and $R^{11}$ are each independently selected from the group consisting of 1-3 substituents which are each independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene)-$COOR^{19}$, $-CH=CH-COOR^{19}$, $-CF_3$, -CN, $-NO_2$ and halo;

$R^{15}$ and $R^{17}$ are each independently selected from the group consisting of $-OR^{19}$, $-OC(O)R^{19}$, $-OC(O)OR^{21}$, $-OC(O)NR^{19}R^{20}$;

$R^{16}$ and $R^{18}$ are each independently selected from the group consisting of H, $(C_1-C_6)$alkyl and aryl;

or $R^{15}$ and $R^{16}$ together are =O, or $R^{17}$ and $R^{18}$ together are =O;

d is 1, 2 or 3;

h is 0, 1, 2, 3 or 4;

s is 0 or 1;

t is 0 or 1;

m, n and p are each independently selected from 0-4;

provided that at least one of s and t is 1, and the sum of m, n, p, s and t is 1-6; provided that when p is 0 and t is 1, the sum of m, n and p is 1-5; and provided that when p is 0 and s is 1, the sum of m, t and n is 1-5;

v is 0 or 1;

j and k are each independently 1-5, provided that the sum of j, k and v is 1-5;

Q is a bond, $-(CH_2)_q-$, wherein q is 1-6, or, with the 3-position ring carbon of the azetidinone, forms the spiro group

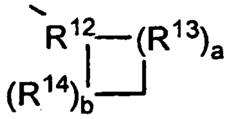

;

wherein $R^{12}$ is

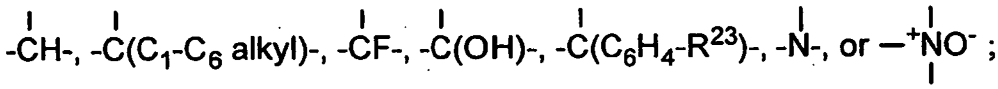

$R^{13}$ and $R^{14}$ are each independently selected from the group consisting of $-CH_2-$, $-CH(C_1-C_6$ alkyl)-, $-C(di-(C_1-C_6)$ alkyl), -CH=CH- and $-C(C_1-C_6$ alkyl)=CH-; or $R^{12}$ together with an adjacent $R^{13}$, or $R^{12}$ together with an adjacent $R^{14}$, form a-CH=CH- or a $-CH=C(C_1-C_6$ alkyl)- group;

a and b are each independently 0, 1, 2 or 3, provided both are not zero; provided that when $R^{13}$ is -CH=CH- or $-C(C_1-C_6$ alkyl)=CH-, a is 1; provided that when $R^{14}$ is -CH=CH- or $-C(C_1-C_6$ alkyl)=CH-, b is 1; provided that when a is 2 or 3, the $R^{13}$'s can be the same or different; and provided that when b is 2 or 3, the $R^{14}$'s can be the same or different; and when Q is a bond and L is

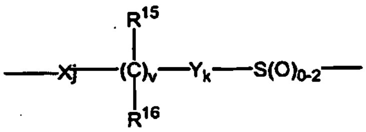

then $Ar^1$ can also be pyridyl, isoxazolyl, furanyl, pyrrolyl, thienyl, imidazolyl, pyrazolyl, thiazolyl, pyrazinyl, pyrimidinyl or pyridazinyl;

$R^{19}$ and $R^{20}$ are each independently selected from the group consisting of H, $(C_1-C_6)$alkyl, aryl and aryl-substituted $(C_1-C_6)$alkyl;

$R^{21}$ is $(C_1-C_6)$alkyl, aryl or $R^{24}$-substituted aryl;

$R^{22}$ is H, $(C_1-C_6)$alkyl, aryl $(C_1-C_6)$alkyl, $-C(O)R^{19}$ or $-COOR^{19}$;

$R^{23}$ and $R^{24}$ are each independently selected from the group consisting of 1-3 substituents which are each independently selected from the group consisting of H, $(C_1-C_6)$alkyl, $(C_1-C_6)$alkoxy, -COOH, $NO_2$, $-NR^{19}R^{20}$, -OH and halo; and

$R^{25}$ is H, -OH or $(C_1-C_6)$alkoxy.

Examples of compounds of Formula (IX) which are useful in the methods and combinations of the present invention and methods for making such compounds are disclosed in U.S. Patent Application Serial No. 10/166,942, filed June 11, 2002, incorporated herein by reference.

An example of a useful compound of this invention is one represented by the formula X:

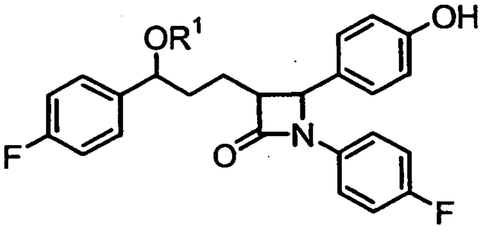

X

wherein $R^1$ is defined as above.

A more preferred compound is one represented by formula XI:

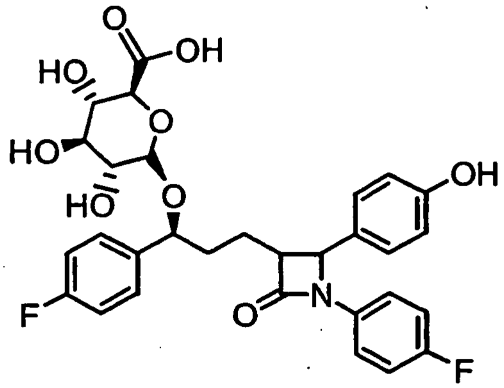

(XI).

Another useful compound is represented by Formula XII:

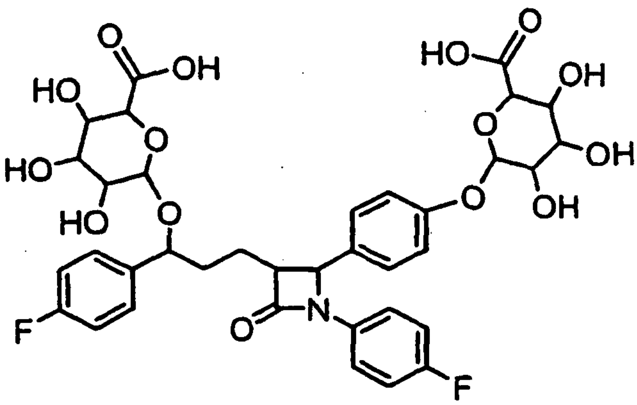

XII

The compounds of Formulae I-XII can be prepared by known methods, including the methods discussed above and, for example, WO 93/02048 describes the preparation of compounds wherein $-R^1-Q-$ is alkylene, alkenylene or alkylene interrupted by a hetero atom, phenylene or cycloalkylene; WO 94/17038 describes the preparation of compounds wherein Q is a spirocyctic group; WO 95/08532 describes the preparation of compounds wherein $-R^1-Q-$ is a hydroxy-substituted alkylene group; PCT/US95/03196 describes compounds wherein $-R^1-Q-$ is a hydroxy-substituted alkylene attached to the $Ar^1$ moiety through an -0- or $S(O)_{0-2}$-group; and U.S. Serial No. 08/463,619, filed June 5, 1995, describes the preparation of compounds wherein $-R^1-Q-$ is a hydroxy-substituted alkylene group attached the azetidinone ring by a $-S(O)_{0-2}-$ group.

The daily dose of the sterol or 5α-stanol absorption inhibitor(s) administered to the subject can range from about 0.1 to about 1000 mg per day, preferably about 0.25 to about 50 mg/day, and more preferably about 10 mg per

day, given in a single dose or 2-4 divided doses. The exact dose, however, is determined by the attending clinician and is dependent on the potency of the compound administered, the age, weight, condition and response of the patient.

For administration of pharmaceutically acceptable salts of the above compounds, the weights indicated above refer to the weight of the acid equivalent or the base equivalent of the therapeutic compound derived from the salt.

In another embodiment, the use of the present invention for treating or preventing vascular inflammation or for reducing blood levels of c-reactive protein can further comprise one or more peroxisome proliferator-activated receptor activator(s) coadministered with or in combination with the and sterol absorption inhibitor(s). These activators act as agonists for the peroxisome proliferator-activated receptors (PPAR). Three subtypes of PPAR have been identified, and these are designated as peroxisome proliferator-activated receptor alpha (PPARα), peroxisome proliferator-activated receptor gamma (PPARγ) and peroxisome proliferator-activated receptor delta (PPARδ). It should be noted that PPARδ is also referred to in the literature as PPAR and as NUC1, and each of these names refers to the same receptor.

PPARα regulates the metabolism of lipids. PPARα is activated by fibrates and a number of medium and long-chain fatty acids, and it is involved in stimulating β-oxidation of fatty acids. The PPARγ receptor subtypes are involved in activating the program of adipocyte differentiation and are not involved in stimulating peroxisome proliferation in the liver. PPARδ has been identified as being useful in.increasing high density lipoprotein (HDL) levels in humans. See, e.g., WO 97/28149.

PPARα activator compounds are useful for, among other things, lowering triglycerides, moderately lowering LDL levels and increasing HDL levels. Useful examples of PPARα activators include fibrates.

Non-limiting examples of suitable fibric acid derivatives ("fibrates") include clofibrate (such as ethyl 2-(p-chlorophenoxy)-2-methyl-propionate, for example ATROMID-S® Capsules which are commercially available from Wyeth-Ayerst); gemfibrozil (such as 5-(2,5-dimethylphenoxy)-2,2-dimethylpentanoic acid, for example LOPID® tablets which are commercially available from Parke Davis); ciprofibrate (C.A.S. Registry No. 52214-84-3, see U.S. Patent No. 3,948,973 which is incorporated herein by reference); bezafibrate (C.A.S. Registry No. 41859-67-0, see U.S. Patent No. 3,781,328 which is incorporated herein by reference); clinofibrate (C.A.S. Registry No. 30299-08-2, see U.S. Patent No. 3,716,583 which is incorporated herein by reference); binifibrate (C.A.S. Registry No. 69047-39-8, see BE 884722 which is incorporated herein by reference); lifibrol (C.A.S. Registry No. 96609-16-4); fenofibrate (such as TRICOR® micronized fenofibrate (2-[4-(4-chlorobenzoyl) phenoxy]-2-methyl-propanoic acid, 1-methylethyl ester) which is commercially available from Abbott Laboratories or LIPANTHYL® micronized fenofibrate which is commercially available from Labortoire Founier, France) and mixtures thereof. These compounds can be used in a variety of forms, including but not limited to acid form, salt form, racemates, enantiomers, zwitterions and tautomers.

Other examples of PPARα activators useful with the practice of the present invention include suitable fluorophenyl compounds as disclosed in U.S. No. 6,028,109 which is incorporated herein by reference; certain substituted phenylpropionic compounds as disclosed in WO 00/75103 which is incorporated herein by reference; and PPARα activator compounds as disclosed in WO 98/43081 which is incorporated herein by reference.

Non-limiting examples of suitable PPARγ activator include derivatives of glitazones or thiazolidinediones, such as, troglitazone (such as REZULIN® troglitazone (-5-[[4-[3,4-dihydro-6-hydroxy-2,5,7,8-tetramethyl-2H-1-benzopyran-2-yl)methoxy]phenyl] methyl]-2,4-thiazolidinedione) commercially available from Parke-Davis); rosiglitazone (such as AVANDIA® rosiglitazone maleate (-5-[[4-[2-(methyl-2-pyridinylamino)ethoxy] phenyl] methyl]-2,4-thiazolidinedione, -2-butenedioate) commercially available from SmithKline Beecham) and pioglitazone (such as ACTOS™ pioglitazone hydrochloride (5-[[4-[2-(5-ethyl-2-pyridinyl)ethoxy]phenyl]methyl]-2,4-] thiazolidinedione monohydrochloride) commercially available from Takeda Pharmaceuticals). Other useful thiazolidinediones include ciglitazone, englitazone, darglitazone and BRL 49653 as disclosed in WO 98/05331 which is incorporated herein by reference; PPARγ activator compounds disclosed in WO 00/76488 which is incorporated herein by reference; and PPARγ activator compounds disclosed in U.S. Patent No. 5,994,554 which is incorporated herein by reference.

Other useful classes of PPARγ activator compounds include certain acetylphenols as disclosed in U.S. Patent No. 5,859,051 which is incorporated herein by reference; certain quinoline phenyl compounds as disclosed in WO 99/20275 which is incorporated herein by reference; aryl compounds as disclosed by WO 99/38845 which is incorporated herein by reference; certain 1,4-disubstituted phenyl compounds as disclosed in WO 00/63161; certain aryl compounds as disclosed in WO 01/00579 which is incorporated herein by reference; benzoic acid compounds as disclosed in WO 01/12612 & WO 01/12187 which are incorporated herein by reference; and substituted 4-hydroxy-phenylalconic acid compounds as disclosed in WO 97/31907 which is incorporated herein by reference.

PPARδ compounds are useful for, among other things, lowering triglyceride levels or raising HDL levels. Non-limiting examples of PPARδ activators include suitable thiazole and oxazole derivates, such as C.A.S. Registry No. 317318-32-4, as disclosed in WO 01/00603 which is incorporated herein by reference); certain fluoro, chloro or thio phenoxy phenylacetic acids as disclosed in WO 97/28149 which is incorporated herein by reference; suitable non-β-oxidizable fatty acid analogues as disclosed in U.S. Patent No. 5,093,365 which is incorporated herein by reference; and PPARδ compounds as disclosed in WO 99/04815 which is incorporated herein by reference.

Moreover, compounds that have multiple functionality for activating various combinations of PPARα, PPARγ

and PPARδ are also useful with the practice of the present invention. Non-limiting examples include certain substituted aryl compounds as disclosed in U.S. Patent No. 6,248,781; WO 00/23416; WO 00/23415; WO 00/23425; WO 00/23445; WO 00/23451; and WO 00/63153, all of which are incorporated herein by reference, are described as being useful PPARα and/or PPARγ activator compounds. Other non-limiting examples of useful PPARα and/or PPARγ activator compounds include activator compounds as disclosed in WO 97/25042 which is incorporated herein by reference; activator compounds as disclosed in WO 00/63190 which is incorporated herein by reference; activator compounds as disclosed in WO 01/21181 which is incorporated herein by reference; biaryl-oxa(thia)zole compounds as disclosed in WO 01/16120 which is incorporated herein by reference; compounds as disclosed in WO 00/63196 and WO 00/63209 which are incorporated herein by reference; substituted 5-aryl-2,4-thiazolidinediones compounds as disclosed in U.S. Patent No. 6,008,237 which is incorporated herein by reference; arylthiazolidinedione and aryloxazolidinedione compounds as disclosed in WO 00/78312 and WO 00/78313G which are incorporated herein by reference; GW2331 or (2-(4-[difluorophenyl]-1heptylureido)ethyl]phenoxy)-2-methylbutyric compounds as disclosed in WO 98/05331 which is incorporated herein by reference; aryl compounds as disclosed in U.S. Patent No. 6,166,049 which is incorporated herein by reference; oxazole compounds as disclosed in WO 01/17994 which is incorporated herein by reference; and dithiolane compounds as disclosed in WO 01/25225 and WO 01/25226 which are incorporated herein by reference.

Other useful PPAR activator compounds include substituted benzylthiazolidine-2,4-dione compounds as disclosed in WO 01/14349, WO 01/14350 and WO/01/04351 which are incorporated herein by reference; mercaptocarboxylic compounds as disclosed in WO 00/50392 which is incorporated herein by reference; ascofuranone compounds as disclosed in WO 00/53563 which is incorporated herein by reference; carboxylic compounds as disclosed in WO 99/46232 which is incorporated herein by reference; compounds as disclosed in WO 99/12534 which is incorporated herein by reference; benzene compounds as disclosed in WO 99/15520 which is incorporated herein by reference; o-anisamide compounds as disclosed in WO 01/21578 which is incorporated herein by reference; and PPAR activator compounds as disclosed in WO 01/40192 which is incorporated herein by reference.

The peroxisome proliferator-activated receptor(s) activator(s) can be administered in a therapeutically effective amount to treat the specified condition, for example in a daily dose preferably ranging from about 50 to about 3000 mg per day, and more preferably about 50 to about 2000 mg per day, given in a single dose or 2-4 divided doses. The exact dose, however, is determined by the attending clinician and is dependent on such factors as the potency of the compound administered, the age, weight, condition and response of the patient.

In another embodiment of the present invention, the use, compositions or therapeutic combinations can further comprise one or more pharmacological or therapeutic agents or drugs such as lipid-lowering agents discussed below.

Non-limiting examples of cholesterol biosynthesis inhibitors for use in the methods for treating or preventing vascular inflammation or for reducing blood levels of c-reactive protein include competitive inhibitors of HMG CoA reductase, the rate-limiting step in cholesterol biosynthesis, squalene synthase inhibitors, squalene epoxidase inhibitors and mixtures thereof. Non-limiting examples of suitable HMG CoA reductase inhibitors include statins such as lovastatin (for example MEVACOR® which is available from Merck & Co.), pravastatin (for example PRAVACHOL® which is available from Bristol Meyers Squibb), fluvastatin, simvastatin (for example ZOCOR® which is available from Merck & Co.), atorvastatin, cerivastatin, Cl-981 and pitavastatin (such as NK-104 of Negma Kowa of Japan); HMG CoA synthetase inhibitor, for example L-659,699 ((E,E)-11-[3'R-(hydroxy-methyl)-4'-oxo-2'R-oxetanyl]-3,5,7R-trimethyl-2,4-undecadienoic acid); squalene synthesis inhibitors, for example squalestatin 1; and squalene epoxidase inhibitors, for example, NB-598 ((E)-N-ethyl-N-(6,6-dimethyl-2-hepten-4-ynyl)-3-[(3,3'-bithiophen-5-yl)methoxy]benzene-methanamine hydrochloride) and other sterol biosynthesis inhibitors such as DMP-565. Preferred HMG CoA reductase inhibitors include lovastatin, pravastatin and simvastatin. The most preferred HMG CoA reductase inhibitor is simvastatin.

Generally, a total daily dosage of cholesterol biosynthesis inhibitor(s) can range from about 0.1 to about 160 mg per day, and preferably about 0.2 to about 80 mg/day in single or 2-3 divided doses.

In another preferred embodiment, the use for treating or preventing vascular inflammation or for reducing blood levels of c-reactive protein composition or treatment comprises the compound of Formula (II) in combination with one or more peroxisome proliferator-activated receptor(s) activator(s) and one or more cholesterol biosynthesis inhibitors. In this embodiment, preferably the peroxisome proliferator-activated receptor activator(s) is a fibric acid derivative is selected from gemfibrozil, clofibrate and/or fenofibrate. Preferably the cholesterol biosynthesis inhibitor comprises one or more HMG CoA reductase inhibitors, such as, for example, lovastatin, pravastatin and/or simvastatin. More preferably, the method comprises the compound of Formula (II) in combination with simvastatin and gemfibrozil or fenofibrate.

In another alternative embodiment, the use of the present invention for treating or preventing vascular inflammation or for reducing blood levels of c-reactive protein of the present invention can further comprise one or more bile acid sequestrants (insoluble anion exchange resins), coadministered with or in combination with the fibric acid derivative (s) and sterol absorption inhibitor(s) discussed above.

Bile acid sequestrants bind bile acids in the intestine, interrupting the enterohepatic circulation of bile acids and causing an increase in the faecal excretion of steroids. Use of bile acid sequestrants is desirable because of their non-systemic mode of action. Bile acid sequestrants can lower intrahepatic cholesterol and promote the synthesis of

apo B/E (LDL) receptors which bind LDL from plasma to further reduce cholesterol levels in the blood.

Non-limiting examples of suitable bile acid sequestrants include cholestyramine (a styrene-divinylbenzene copolymer containing quaternary ammonium cationic groups capable of binding bile acids, such as QUESTRAN® or QUESTRAN LIGHT® cholestyramine which are available from Bristol-Myers Squibb), colestipol (a copolymer of diethylenetriamine and 1-chloro-2,3-epoxypropane, such as COLESTID® tablets which are available from Pharmacia), colesevelam hydrochloride (such as WelChol® Tablets (poly(allylamine hydrochloride) cross-linked with epichlorohydrin and alkylated with 1-bromodecane and (6-bromohexyl)-trimethylammonium bromide) which are available from Sankyo), water soluble derivatives such as 3,3-ioene, N-(cycloalkyl) alkylamines and poliglusam, insoluble quaternized polystyrenes, saponins and mixtures thereof. Other useful bile acid sequestrants are disclosed in PCT Patent Applications Nos. WO 97/11345 and WO 98/57652, and U.S. Patents Nos. 3,692,895 and 5,703,188 which are incorporated herein by reference. Suitable inorganic cholesterol sequestrants include bismuth salicylate plus montmorillonite clay, aluminum hydroxide and calcium carbonate antacids.

Generally, a total daily dosage of bile acid sequestrant(s) can range from about 1 to about 50 grams per day, and preferably about 2 to about 16 grams per day in single or 2-4 divided doses.

In an alternative embodiment, the use of the present invention for treating or preventing vascular inflammation or for reducing blood levels of c-reactive protein further comprise one or more ileal bile acid transport ("IBAT") inhibitors (or apical sodium co-dependent bile acid transport ("ASBT") inhibitors) coadministered with or in combination with the peroxisome proliferator-activated receptor activator(s) and sterol absorption inhibitor(s) discussed above. The IBAT inhibitors can inhibit bile acid transport to reduce LDL cholesterol levels. Non-limiting examples of suitable IBAT inhibitors include benzothiepines such as therapeutic compounds comprising a 2,3,4,5-tetrahydro-1-benzothiepine 1,1-dioxide structure such as are disclosed in PCT Patent Application WO 00/38727 which is incorporated herein by reference.

Generally, a total daily dosage of IBAT inhibitor(s) can range from about 0.01 to about 1000 mg/day, and preferably about 0.1 to about 50 mg/day in single or 2-4 divided doses.

In another alternative embodiment, the use of the present invention for treating or preventing vascular inflammation or for reducing blood levels of c-reactive protein can further comprise nicotinic acid (niacin) and/or derivatives thereof coadministered with or in combination with the peroxisome proliferator-activated receptor activator(s) and sterol absorption inhibitor(s) discussed above.

As used herein, "nicotinic acid derivative" means a compound comprising a pyridine-3-carboxylate structure or a pyrazine-2-carboxylate structure, including acid forms, salts, esters, zwitterions and tautomers, where available. Examples of nicotinic acid derivatives include niceritrol, nicofuranose and acipimox (5-methyl pyrazine-2-carboxylic acid 4-oxide). Nicotinic acid and its derivatives inhibit hepatic production of VLDL and its metabolite LDL and increases HDL and apo A-1 levels. An example of a suitable nicotinic acid product is NIASPAN® (niacin extended-release tablets) which are available from Kos.

Generally, a total daily dosage of nicotinic acid or a derivative thereof can range from about 500 to about 10,000 mg/day, preferably about 1000 to about 8000 mg/day, and more preferably about 3000 to about 6000 mg/day in single or divided doses.

In another alternative embodiment, the use of the present invention for treating or preventing vascular inflammation or for reducing blood levels of c-reactive protein can further comprise one or more AcylCoA:Cholesterol *O*-acyltransferase ("ACAT") Inhibitors, which can reduce LDL and VLDL levels, coadministered with or in combination with the peroxisome proliferator-activated receptor activator(s) and sterol absorption inhibitor(s) discussed above. ACAT is an enzyme responsible for esterifying excess intracellular cholesterol and may reduce the synthesis of VLDL; which is a product of cholesterol esterification, and overproduction of apo B-100-containing lipoproteins.

Non-limiting examples of useful ACAT inhibitors include avasimibe ([[2,4,6-tris(1-methylethyl)phenyl]acetyl] sulfamic acid, 2,6-bis(1-methylethyl)phenyl ester, formerly known as CI-1011), HL-004, lecimibide (DuP-128) and CL-277082 (*N*-(2,4-difluorophenyl)-*N*-[[4-(2,2-dimethylpropyl)phenyl]methyl]-*N*-heptylurea). See P. Chang et al., "Current, New and Future Treatments in Dyslipidaemia and Atherosclerosis", Drugs 2000 Jul;60(1); 55-93, which is incorporated by reference herein.

Generally, a total daily dosage of ACAT inhibitor(s) can range from about 0.1 to about 1000 mg/day in single or 2-4 divided doses.

In another alternative embodiment, the use of the present invention for treating or preventing vascular inflammation or for reducing blood levels of c-reactive protein can further comprise one or more Cholesteryl Ester Transfer Protein ("CETP") Inhibitors coadministered with or in combination with the peroxisome proliferator-activated receptor activator(s) and sterol absorption inhibitor(s) discussed above. CETP is responsible for the exchange or transfer of cholesteryl ester carrying HDL and triglycerides in VLDL.

Non-limiting examples of suitable CETP inhibitors are disclosed in PCT Patent Application No. WO 00/38721 and U.S. Patent No. 6,147,090, which are incorporated herein by reference. Pancreatic cholesteryl ester hydrolase (pCEH) inhibitors such as WAY-121898 also can be coadministered with or in combination with the peroxisome proliferator-activated receptor(s) activator and sterol absorption inhibitor(s) discussed above.

Generally, a total daily dosage of CETP inhibitor(s) can range from about 0.01 to about 1000 mg/day, and preferably about 0.5 to about 20 mg/kg body weight/day in single or divided doses.

In another alternative embodiment, the use of the present invention for treating or preventing vascular inflammation or for reducing blood levels of c-reactive protein can further comprise probucol or derivatives thereof (such as AGI-1067 and other derivatives disclosed in U.S. Patents Nos. 6,121,319 and 6,147,250), which can reduce LDL levels, coadministered with or in combination with the peroxisome proliferator-activated receptor activator(s) and sterol absorption inhibitor(s) discussed above.

Generally, a total daily dosage of probucol or derivatives thereof can range from about 10 to about 2000 mg/day, and preferably about 500 to about 1500 mg/day in single or 2-4 divided doses.

In another alternative embodiment, the use of the present invention for treating or preventing vascular inflammation or for reducing blood levels of c-reactive protein can further comprise low-density lipoprotein (LDL) receptor activators, coadministered with or in combination with the peroxisome proliferator-activated receptor activator(s) and sterol absorption inhibitor(s) discussed above. Non-limiting examples of suitable LDL-receptor activators include HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arterioscler. Thromb. 1993; 13:1005-12.

Generally, a total daily dosage of LDL receptor activator(s) can range from about 1 to about 1000 mg/day in single or 2-4 divided doses.

In another alternative embodiment, the use of the present invention for treating or preventing vascular inflammation or for reducing blood levels of c-reactive protein can further comprise fish oil, which contains Omega 3 fatty acids (3-PUFA), which can reduce VLDL and triglyceride levels, coadministered with or in combination with the peroxisome proliferator-activated receptor activator(s) and sterol absorption inhibitor(s) discussed above. Generally, a total daily dosage of fish oil or Omega 3 fatty acids can range from about 1 to about 30 grams per day in single or 2-4 divided doses.

In another alternative embodiment, the use of the present invention for treating or preventing vascular inflammation or for reducing blood levels of c-reactive protein can further comprise natural water soluble fibers, such as psyllium, guar, oat and pectin, which can reduce cholesterol levels, coadministered with or in combination with the peroxisome proliferator-activated receptor activator(s) and sterol absorption inhibitor(s) discussed above. Generally, a total daily dosage of natural water soluble fibers can range from about 0.1 to about 10 grams per day in single or 2-4 divided doses.

In another alternative embodiment, the use of the present invention for treating or preventing vascular inflammation or for reducing blood levels of c-reactive protein can further comprise plant sterols, plant stanols and/or fatty acid esters of plant stanols, such as sitostanol ester used in BENECOL® margarine, which can reduce cholesterol levels, coadministered with or in combination with the peroxisome proliferator-activated receptor activator(s) and sterol absorption inhibitor(s) discussed above. Generally, a total daily dosage of plant sterols, plant stanols and/or fatty acid esters of plant stanols can range from about 0.5 to about 20 grams per day in single or 2-4 divided doses.

In another alternative embodiment, the use of the present invention for treating or preventing vascular inflammation or for reducing blood levels of c-reactive protein can further comprise antioxidants, such as probucol, tocopherol, ascorbic acid, β-carotene and selenium, or vitamins such as vitamin $B_6$ or vitamin $B_{12}$, coadministered with or in combination with the peroxisome proliferator-activated receptor activator(s) and sterol absorption inhibitor(s) discussed above. Generally, a total daily dosage of antioxidants or vitamins can range from about 0.05 to about 10 grams per day in single or 2-4 divided doses.

In another alternative embodiment, the use of the present invention for treating or preventing vascular inflammation or for reducing blood levels of c-reactive protein can further comprise monocyte and macrophage inhibitors such as polyunsaturated fatty acids (PUFA), thyroid hormones including throxine analogues such as CGS-26214 (a thyroxine compound with a fluorinated ring), gene therapy and use of recombinant proteins such as recombinant apo E, coadministered with or in combination with the peroxisome proliferator-activated receptor activator(s) and sterol absorption inhibitor(s) discussed above. Generally, a total daily dosage of these agents can range from about 0.01 to about 1000 mg/day in single or 2-4 divided doses.

Also useful with the present invention are compositions or therapeutic combinations that further comprise hormone replacement agents and compositions. Useful hormone agents and compositions for hormone replacement therapy of the present invention include androgens, estrogens, progestins, their pharmaceutically acceptable salts and derivatives. Combinations of these agents and compositions are also useful.

The dosage of androgen and estrogen combinations vary, desirably from about 1 mg to about 4 mg androgen and from about 1 mg to about 3 mg estrogen. Examples include, but are not limited to, androgen and estrogen combinations such as the combination of esterified estrogens (sodium estrone sulfate and sodium equilin sulfate) and methyltestosterone (17-hydroxy-17-methyl-, (17B)- androst-4-en-3-one) available from Solvay Pharmaceuticals, Inc., Marietta, GA, under the tradename Estratest.

Estrogens and estrogen combinations may vary in dosage from about 0.01 mg up to 8 mg, desirably from about

0.3 mg to about 3.0 mg. Examples of useful estrogens and estrogen combinations include:

(a) the blend of nine (9) synthetic estrogenic substances including sodium estrone sulfate, sodium equilin sulfate, sodium 17 α -dihydroequilin sulfate, sodium 17 α -estradiol sulfate, sodium 17 β -dihydroequilin sulfate, sodium 17 α -dihydroequilenin sulfate, sodium 17 β -dihydroequilenin sulfate, sodium equilenin sulfate and sodium 17 β -estradiol sulfate; available from Duramed Pharmaceuticals, Inc., Cincinnati, OH, under the tradename Cenestin;
(b) ethinyl estradiol (19-or-17α-pregna-1,3,5(10)-trien-20-yne-3,17-diol; available by Schering Plough Corporation, Kenilworth, NJ, under the tradename Estinyl;
(c) esterified estrogen combinations such as sodium estrone sulfate and sodium equilin sulfate; available from Solvay under the tradename Estratab and from Monarch Pharmaceuticals, Bristol, TN, under the tradename Menest;
(d) estropipate (piperazine estra-1,3,5(10)-trien-17-one, 3-(sulfooxy)-estrone sulfate); available from Pharmacia & Upjohn, Peapack, NJ, under the tradename Ogen and from Women First Health Care, Inc., San Diego, CA, under the tradename Ortho-Est; and
(e) conjugated estrogens (17α-dihydroequilin, 17α-estradiol, and 17β-dihydroequilin); available from Wyeth-Ayerst Pharmaceuticals, Philadelphia, PA, under the tradename Premarin.

Progestins and estrogens may also be administered with a variety of dosages, generally from about .05 to about 2.0 mg progestin and about .001 mg to about 2 mg estrogen, desirably from about .1 mg to about 1 mg progestin and about 0:01 mg to about .5 mg estrogen. Examples of progestin and estrogen combinations that may vary in dosage and regimen include:

(a) the combination of estradiol (estra-1, 3, 5 (10)-triene-3, 17 β-diol hemihydrate) and norethindrone (17 β-acetoxy-19-nor-17 α-pregn-4-en-20-yn-3-one); which is available from Pharmacia & Upjohn, Peapack, NJ, under the tradename Activella;
(b) the combination of levonorgestrel (d(-)-13 β-ethyl-17 α-ethinyl-17 β-hydroxygon- 4-en-3-one) and ethinyl estradial; available from Wyeth-Ayerst under the tradename Alesse, from Watson Laboratories, Inc., Corona, CA, under the tradenames Levora and Trivora, Monarch Pharmaceuticals, under the tradename Nordette, and from Wyeth-Ayerst under the tradename Triphasil;
(c) the combination of ethynodiol diacetate (19-nor-17 α-pregn-4-en-20-yne-3 β, 17-diol diacetate) and ethinyl estradiol; available from G.D. Searle & Co., Chicago, IL, under the tradename Demulen and from Watson under the tradename Zovia;
(d) the combination of desogestrel (13-ethyl-11- methylene-18,19-dinor-17 α-pregn- 4-en- 20-yn-17-ol) and ethinyl estradiol; available from Organon under the tradenames Desogen and Mircette, and from Ortho-McNeil Pharmaceutical, Raritan, NJ, under the tradename Ortho-Cept;
(e) the combination of norethindrone and ethinyl estradiol; available from Parke-Davis, Morris Plains, NJ, under the tradenames Estrostep and femhrt, from Watson under the tradenames Microgestin, Necon, and Tri-Norinyl, from Ortho-McNeil under the tradenames Modicon and Ortho-Novum, and from Warner Chilcott Laboratories, Rockaway, NJ, under the tradename Ovcon;
(f) the combination of norgestrel ((±)-13-ethyl-17-hydroxy-18, 1-9-dinor-17 α-preg-4-en-20-yn-3-one) and ethinyl estradiol; available from Wyeth-Ayerst under the tradenames Ovral and Lo/Ovral, and from Watson under the tradenames Ogestrel and Low-Ogestrel;
(g) the combination of norethindrone, ethinyl estradiol, and mestranol (3-methoxy-19-nor-17α-pregna-1,3,5(10)-trien-20-yn-17-ol); available from Watson under the tradenames Brevicon and Norinyl;
(h) the combination of 17 β-estradiol (estra-1,3,5(10)-triene-3,17β-diol) and micronized norgestimate (17 α-17-(Acetyloxyl)-13-ethyl-18, 19-dinorpregn-4-en-20-yn-3-one3-oxime); available from Ortho-McNeil under the tradename Ortho-Prefest;
(i) the combination of norgestimate (18,19-dinor-17-pregn-4-en-20-yn-3-one, 17-(acetyloxy)-13-ethyl-,oxime, (17 (α)-(+)-) and ethinyl estradiol; available from Ortho-McNeil under the tradenames Ortho Cyclen and Ortho Tri-Cyclen; and
(j) the combination of conjugated estrogens (sodium estrone sulfate and sodium equilin sulfate) and medroxyprogesterone acetate (20-dione, 17-(acetyloxy)-6-methyl-, (6(α))- pregn-4-ene-3); available from Wyeth-Ayerst under the tradenames Premphase and Prempro.

In general, a dosage of progestins may vary from about .05 mg to about 10 mg or up to about 200 mg if microsized progesterone is administered. Examples of progestins include norethindrone; available from ESI Lederle, Inc., Philadelphia, PA, under the tradename Aygestin, from Ortho-McNeil under the tradename Micronor, and from Watson under the tradename Nor-QD; norgestrel; available from Wyeth-Ayerst under the tradename Ovrette; micronized progesterone (pregn-4-ene-3, 20-dione); available from Solvay under the tradename Prometrium; and medroxyprogesterone acetate; available from Pharmacia & Upjohn under the tradename Provera.

The compositions, therapeutic combinations or use of the present invention can further comprise one or more obesity control medications. Useful obesity control medications include, but are not limited to, drugs that reduce energy intake or suppress appetite, drugs that increase energy expenditure and nutrient-partitioning agents. Suitable obesity control medications include, but are not limited to, noradrenergic agents (such as diethylpropion, mazindol, phenylpropanolamine, phentermine, phendimetrazine, phendamine tartrate, methamphetamine, phendimetrazine and tartrate); serotonergic agents (such as sibutramine, fenfluramine, dexfenfluramine, fluoxetine, fluvoxamine and paroxtine); thermogenic agents (such as ephedrine, caffeine, theophylline, and selective β3-adrenergic agonists); an alpha-blocking agent; a kainite or AMPA receptor antagonist; a leptin-lipolysis stimulated receptor; a phosphodiesterase enzyme inhibitor; a compound having nucleotide sequences of the mahogany gene; a fibroblast growth factor-10 polypeptide; a monoamine oxidase inhibitor (such as befloxatone, moclobemide, brofaromine, phenoxathine, esuprone, befol, toloxatone, pirlindol, amiflamine, sercloremine, bazinaprine, lazabemide, milacemide and caroxazone); a compound for increasing lipid metabolism (such as evodiamine compounds); and a lipase inhibitor (such as orlistat). Generally, a total dosage of the above-described obesity control medications can range from 1 to 3,000 mg/day, desirably from about 1 to 1,000 mg/day and more desirably from about 1 to 200 mg/day in single or 2-4 divided doses.

The compositions, therapeutic combinations or use of the present invention can further comprise one or more blood modifiers. Useful blood modifiers include but are not limited to anti-coagulants (argatroban, bivalirudin, dalteparin sodium, desirudin, dicumarol, lyapolate sodium, nafamostat mesylate, phenprocoumon, tinzaparin sodium, warfarin sodium); antithrombotic (anagrelide hydrochloride, bivalirudin, cilostazol, dalteparin sodium, danaparoid sodium, dazoxiben hydrochloride, efegatran sulfate, enoxaparin sodium, fluretofen, ifetroban, ifetroban sodium, lamifiban, lotrafiban hydrochloride, napsagatran, orbofiban acetate, roxifiban acetate, sibrafiban, tinzaparin sodium, trifenagrel, abciximab, zolimomab aritox); fibrinogen receptor antagonists (roxifiban acetate, fradafiban, orbofiban, lotrafiban hydrochloride, tirofiban, xemilofiban, monoclonal antibody 7E3, sibrafiban); platelet inhibitors (cilostazol, clopidogrel bisulfate, epoprostenol, epoprostenol sodium, ticlopidine hydrochloride, aspirin, ibuprofen, naproxen, sulindae, idomethacin, mefenamate, droxicam, diclofenac, sulfinpyrazone, piroxicam, dipyridamole); platelet aggregation inhibitors (acadesine, beraprost, beraprost sodium, ciprostene calcium, itazigrel, lifarizine, lotrafiban hydrochloride, orbofiban acetate, oxagrelate, fradafiban, orbofiban, tirofiban, xemilofiban); hemorrheologic agents (pentoxifylline); lipoprotein associated coagulation inhibitor; Factor VIIa inhibitors (4H-31-benzoxazin-4-ones, 4H-3,1-benzoxazin-4-thiones, quinazolin-4-ones, quinazolin-4-thiones, benzothiazin-4-ones, imidazolyl-boronic acid-derived peptide analogues TFPI-derived peptides, naphthalene-2-sulfonic acid {1-[3-(aminoiminomethyl)-benzyl]-2-oxo-pyrrolidin-3-(S)-yl} amide trifluoroacetate, dibenzofuran-2-sulfonic acid {1-[3-(aminomethyl)-benzyl]-5-oxo-pyrrolidin-3-yl}-amide, tolulene-4-sulfonic acid {1-[3-(aminoiminomethyl)-benzyl]-2-oxo-pyrrolid-in-3-(S)-yl}-amide trifluoroacetate, 3,4-dihydro-1H-isoquinoline-2-sulfonic acid {1-[3-(aminoiminomethyl)-benzyl]-2-oxo-pyrrolin-3-(S)-yl}-amide trifluoroacetate); Factor Xa inhibitors (disubstituted pyrazolines, disubstituted triazolines, substituted n-[(aminoiminomethyl)phenyl] propylamides, substituted n-[(aminomethyl)pheny] propylamides, tissue factor pathway inhibitor (TFPI), low molecular weight heparins, heparinoids, benzimidazolines, benzoxazolinones, benzopiperazinones, indanones, dibasic (amidinoaryl) propanoic acid derivatives, amidinophenyl-pyrrolidines, amidinophenyl-pyrrolines, amidinophenyl-isoxazolidines, amidinoindoles, amidinoazoles, bis-arlysulfonylaminobenzamide derivatives, peptidic Factor Xa inhibitors).

The compositions, therapeutic combinations or use of the present invention can further comprise one or more cardiovascular agents. Useful cardiovascular agents include but are not limited to calcium channel blockers (clentiazem maleate, amlodipine besylate, isradipine, nimodipine, felodipine, nilvadipine, nifedipine, teludipine hydrochloride, diltiazem hydrochloride, belfosdil, verapamil hydrochloride, fostedil); adrenergic blockers (fenspiride hydrochloride, labetalol hydrochloride, proroxan, alfuzosin hydrochloride, acebutolol, acebutolol hydrochloride, alprenolol hydrochloride, atenolol, bunolol hydrochloride, carteolol hydrochloride, celiprolol hydrochloride, cetamolol hydrochloride, cicloprolol hydrochloride, dexpropranolol hydrochloride, diacetolol hydrochloride, dilevalol hydrochloride, esmolol hydrochloride, exaprolol hydrochloride, flestolol suffite, labetalol hydrochloride, levobetaxolol hydrochloride, levobunolol hydrochloride, metalol hydrochloride, metoprolol, metoprolol tartrate, nadolol, pamatolol sulfate, penbutolol sulfate, practolol, propranolol hydrochloride, sotalol hydrochloride, timolol, timolol maleate, tiprenolol hydrochloride, tolamolol, bisoprolol, bisoprolol fumarate, nebivolol); adrenergic stimulants; angiotensin converting enzyme (ACE) inhibitors (benazepril hydrochloride, benazeprilat, captopril, delapril hydrochloride, fosinopril sodium, libenzapril, moexipril hydrochloride, pentopril, perindopril, quinapril hydrochloride, quinaprilat, ramipril, spirapril hydrochloride, spiraprilat, teprotide, enalapril maleate, lisinopril, zofenopril calcium, perindopril erbumine); antihypertensive agents (althiazide, benzthiazide, captopril, carvedilol, chlorothiazide sodium, clonidine hydrochloride, cyclothiazide, delapril hydrochloride, dilevalol hydrochloride, doxazosin mesylate, fosinopril sodium, guanfacine hydrochloride, methyldopa, metoprolol succinate, moexipril hydrochloride, monatepil maleate, pelanserin hydrochloride, phenoxybenzamine hydrochloride, prazosin hydrochloride, primidolol, quinapril hydrochloride, quinaprilat, ramipril, terazosin hydrochloride, candesartan, candesartan cilexetil, telmisartan, amlodipine besylate, amlodipine maleate, bevantolol hydrochloride); angiotensin II receptor antagonists (candesartan, irbesartan, losartan potassium, candesartan cilexetil, telmisartan); anti-anginal agents (amlodipine besylate,

amlodipine maleate, betaxolol hydrochloride, bevantolol hydrochloride, butoprozine hydrochloride, carvedilol, cinepazet maleate, metoprolol succinate, molsidomine, monatepil maleate, primidolol, ranolazine hydrochoride, tosifen, verapamil hydrochloride); coronary vasodilators (fostedil, azaclorzine hydrochloride, chromonar hydrochloride, clonitrate, diltiazem hydrochloride, dipyridamole, droprenilamine, erythrityl tetranitrate, isosorbide dinitrate, isosorbide mononitrate, lidoflazine, mioflazine hydrochloride, mixidine, molsidomine, nicorandil, nifedipine, nisoldipine, nitroglycerine, oxprenolol hydrochloride, pentrinitrol, perhexiline maleate, prenylamine, propatyl nitrate, terodiline hydrochloride, tolamolol, verapamil); diuretics (the combination product of hydrochlorothiazide and spironolactone and the combination product of hydrochlorothiazide and triamterene).

The compositions, therapeutic combinations or use of the present invention can further comprise one or more antidiabetic medications for reducing blood glucose levels in a human. Useful antidiabetic medications include, but are not limited to, drugs that reduce energy intake or suppress appetite, drugs that increase energy expenditure and nutrient-partitioning agents. Suitable antidiabetic medications include, but are not limited to, sulfonylurea (such as acetohexamide, chlorpropamide, gliamilide, gliclazide, glimepiride, glipizide, glyburide, glibenclamide, tolazamide, and tolbutamide), meglitinide (such as repaglinide and nateglinide), biguanide (such as metformin and buformin), thiazolidinedione (such as troglitazone, rosiglitazone, pioglitazone, ciglitazone, englitazone, and darglitazone), alpha-glucosidase inhibitor (such as acarbose, miglitol, camiglibose, and voglibose), certain peptides (such as amlintide, pramlintide, exendin, and GLP-1 agonistic peptides), and orally administrable insulin or insulin composition for intestinal delivery thereof. Generally, a total dosage of the above-described antidiabetic medications can range from 0.1 to 1,000 mg/day in single or 2-4 divided doses.

Mixtures of any of the pharmacological or therapeutic agents described above can be used in the compositions and therapeutic combinations of these other embodiments of the present invention.

The compositions and therapeutic combinations of the present invention can be administered to a mammal in need of such treatment in a therapeutically effective amount to treat vascular inflammation or to reduce c-reactive protein levels. The compositions and treatments can be administered by any suitable means that produce contact of these compounds with the site of action in the body, for example in the plasma, liver or small intestine of a mammal.

The daily dosage for the various compositions and therapeutic combinations described above can be administered to a patient in a single dose or in multiple subdoses, as desired. Subdoses can be administered 2 to 6 times per day, for example. Sustained release dosages can be used. Where the sterol absorption inhibitor(s) and other therapeutic agent are administered in separate dosages, the number of doses of each component given per day may not necessarily be the same, e.g., one component may have a greater duration of activity and will therefore need to be administered less frequently.

The compositions, therapeutic combinations or medicaments of the present invention can further comprise one or more pharmaceutically acceptable carriers, one or more excipients and/or one or more additives. The pharmaceutical compositions can comprise about 1 to about 99 weight percent of active ingredient (one or more compounds of Formula I-XII), and preferably about 5 to about 95 percent active ingredient.

Useful pharmaceutically acceptable carriers can be either solid, liquid or gas. Non-limiting examples of pharmaceutically acceptable carriers include solids and/or liquids such as magnesium carbonate, magnesium stearate, talc, sugar, lactose, ethanol, glycerol, water and the like. The amount of carrier in the treatment composition or therapeutic combination can range from about 5 to about 99 weight percent of the total weight of the treatment composition or therapeutic combination. Non-limiting examples of suitable pharmaceutically acceptable excipients and additives include non-toxic compatible fillers, binders such as starch, polyvinyl pyrrolidone or cellulose ethers, disintegrants such as sodium starch glycolate, crosslinked polyvinyl pyrrolidone or croscarmellose sodium, buffers, preservatives, anti-oxidants, lubricants, flavorings, thickeners, coloring agents, wetting agents such as sodium lauryl sulfate, emulsifiers and the like. The amount of excipient or additive can range from about 0.1 to about 95 weight percent of the total weight of the treatment composition or therapeutic combination. One skilled in the art would understand that the amount of carrier (s), excipients and additives (if present) can vary. Further examples of pharmaceutical acceptable carriers and methods of manufacture for various compositions can be found in A. Gennaro (ed.), Remington: The Science and Practice of Pharmacy, 20$^{th}$ Edition, (2000), Lippincott Williams & Wilkins, Baltimore, MD.

Useful solid form preparations include powders, tablets, dispersible granules, capsules, cachets and suppositories. An example of a preparation of a preferred solid form dosage formulation is provided below.

Useful liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also useful are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can

take the form of creams, lotions, aerosols and/or emulsions and can be included in a transdermal patch of the matrix or reservoir type as are conventional in the art for this purpose.

Preferably the compound is administered orally.

## ANALYTICAL PROCEDURES FOR CRP MEASUREMENT

Procedure: For Measuring C-Reactive Protein (CRP) in Serum Using a Behring Nephelometer II

Principle

Polystyrene particles coated with antibodies to CRP are agglutinated when mixed with samples containing CRP. The intensity of the light scattered in the nephelometer is proportional to the concentration of CRP in the sample. Results are evaluated by comparison with a standard of known concentrations of CRP (and ASL and RF). Preservative: Sodium Azide (<1 g/L).

The N/T Rheumatology Controls are supplied ready to use and are stable until the date on the label when stored at 2-8°C. Once opened, they are stable for 14 days when stored tightly capped at 2-8°C. Reagents N-CRP Latex Mono Catalog # OQIY 20/21 (suspension of polystyrene particles coated with mouse monoclonal antibodies to CRP), N-Supplementary Reagent/Precipitation Catalog # OUMU (phosphate buffer solution containing sodium chloride), N Rheumatology Standard SL Catalog # OQKZ13 (contiaining concentration of CRP listed on package insert), N-Diluent Catalog # 61 (phosphate buffered saline), and N-Reaction buffer catalog # OUMS61 (solution of polyethylene glycol and sodium chloride in phosphate buffer) are available from Behring Diagnostics, Inc.

The Behring Nephelometer II is available from Behring Diagnostics, Inc., Somerville, NJ.

Testing Procedures

Vortex samples:

Select the "ROUTINE" menu and select "ENTER JOB LIST".
Enter the appropriate patient identification as Study\Sequence\ID.
Select Assay No. 71 for CRP measurement.
When all samples to be run have been requested, exit "ROUTINE" menu.
Select "LOADING" icon. Load reagents and samples onto the analyzer in the appropriate positions.
Select "LAB JOURNAL" Review controls.

To interface: highlight results to be sent. Select the checkmark icon. A checkmark will appear next to the results to be sent. An H will appear to the right when the results have been sent to the host. Select the printer icon to print results.

For any CRP value that gives a concentration <O.2 mg/L the sample is reordered using the high sensitivity assay #99.

The results are calculated automatically using a logit-log function.

Reference Range: 0.0-8.4mg/L

Reference range established by analyzing 216 randomly chosen serum samples from a patient population of varying age and gender.

Reporting Results:

CRP values greater than or equal to 0.2 mg/L are reported to one decimal.

CRP values less than 0.2 mg/L which have been reanalyzed using the high sensitivity method are reported to 3 decimals.

Procedural Notes

Reagents must not be used beyond their expiration dates. Reagents and samples do not need to be run at room temperature. The apparatus permits the direct use of reagents and samples stored at 2-8°C.

Limitations on Procedure

The sensitivity of the assay is determined by the lower limit of the reference curve and thus depends on the CRP concentration of the standard. Therefore it is possible to have a different sensitivity for each reference curve.
The measuring range is designed to measure CRP concentrations up to 1100 mg/L using the dilution of 1:2000.
Samples with CRP concentrations greater than 1100 mg/L should be diluted with N-Diluent prior to being assayed on the nephelometer. This dilution factor must be used to correct CRP concentration.
Hemoglobin up to 350 mg/dl showed no significant interference.
Highly lipemic samples (triglyceride >500 mg/dl) which cannot be clarified by centrifugation (10 minutes at 15000 g) must be excluded from the assay.

**FORMULATION**

The following formulation exemplifies one of the dosage forms of this invention. In the formulation, the term "Active Compound I" designates a sterol or 5α-stanol absorption inhibitor such as are described herein above.

EXAMPLE

Tablets

| No. | Ingredient | mg/tablet |
|---|---|---|
| 1 | Active Compound I | 10 |
| 2 | Lactose monohydrate NF | 55 |
| 3 | Microcrystalline cellulose NF | 20 |
| 4 | Povidone USP (K29-32) | 4 |
| 5 | Croscarmellose sodium NF | 8 |
| 6 | Sodium lauryl sulfate NF | 2 |
| 7 | Magnesium stearate NF | 1 |
| | Total | 100 |

In the present invention, the above-described tablet can be coadministered with a tablet, capsule, etc. comprising a dosage of Active Compound II, for example a therapeutic agent such as a cardiovascular agent or blood modifier as described above.

Method of Manufacture

Mix Item No. 4 with purified water in suitable mixer to form binder solution. Spray the binder solution and then water over Items 1, 2 and 6 and a portion of item 5 in a fluidized bed processor to granulate the ingredients. Continue fluidization to dry the damp granules. Screen the dried granule and blend with Item No. 3 and the remainder of Item No. 5. Add Item No. 7 and mix. Compress the mixture to appropriate size and weight on a suitable tablet machine.
For coadministration in separate tablets or capsules, representative formulations comprising a cholesterol absorption inhibitor such as are discussed above are well known in the art and representative formulations comprising other cardiovascular agents or blood modifiers such as are discussed above are well known in the art. It is contemplated that where the two active ingredients are administered as a single composition, the dosage forms disclosed above for sterol absorption inhibitors may readily be modified using the knowledge of one skilled in the art.
Since the present invention relates to treating vascular inflammation or to controlling or reducing the level of c-reactive protein by treatment with a combination of active ingredients wherein the active ingredients may be administered separately, the invention also relates to combining separate pharmaceutical compositions in kit form. That is, a kit is contemplated wherein two separate units are combined: a pharmaceutical composition comprising at least one sterol absorption inhibitor as described above and one or more therapeutic agents such as cardiovascular agents, blood modifiers or other active ingredients as discussed above. The kit will preferably include directions for the administration of the separate components. The kit form is particularly advantageous when the separate components must be administered in different dosage forms (e.g., oral and parenteral) or are administered at different dosage intervals.
The treatment compositions and therapeutic combinations of the present invention can treat vascular inflammation and/or control or reduce the level of c-reactive protein in the blood and can be useful in the treatment as well as

prevention of vascular conditions, such as atherosclerosis, hypercholesterolemia and sitosterolemia, stroke, obesity and lower plasma levels of sterols and/or 5α-stanols in a subject, in particular in humans, such as phytosterols (such as sitosterol, campesterol, stigmasterol and avenosterol), 5α-stanols (such as cholestanol, 5α-campestanol, 5α-sitostanol), cholesterol and mixtures thereof. The plasma concentration can be reduced by administering to a subject in need of such treatment an effective amount of at least one treatment composition or therapeutic combination comprising at least one sterol absorption inhibitor described above. The reduction in plasma concentration of sterols can range from about 1 to about 70 percent, and preferably about 10 to about 50 percent. Methods of measuring serum total blood cholesterol and total LDL cholesterol are well known to those skilled in the art and for example include those disclosed in PCT WO 99/38498 at page 11, incorporated by reference herein. Methods of determining levels of other sterols in serum are disclosed in H. Gylling et al., "Serum Sterols During Stanol Ester Feeding in a Mildly Hypercholesterolemic Population", J. Lipid Res. 40: 593-600 (1999), incorporated by reference herein.

Illustrating the invention are the following example of preparation of a compound of formula (II) which, however, are not to be considered as limiting the invention to their details. Unless otherwise indicated, all parts and percentages in the following examples, as well as throughout the specification, are by weight.

## EXAMPLE

PREPARATION OF COMPOUND OF FORMULA (II)

Step 1): To a solution of (S)-4-phenyl-2-oxazolidinone (41 g, 0.25 mol) in $CH_2Cl_2$ (200 ml), was added 4-dimethylaminopyridine (2.5 g, 0.02 mol) and triethylamine (84.7 ml, 0.61 mol) and the reaction mixture was cooled to 0°C. Methyl-4-(chloroformyl)butyrate (50 g, 0.3 mol) was added as a solution in $CH_2Cl_2$ (375 ml) dropwise over 1 h, and the reaction was allowed to warm to 22°C. After 17 h, water and $H_2SO_4$ (2N, 100 ml), was added the layers were separated, and the organic layer was washed sequentially with NaOH (10%), NaCl (sat'd) and water. The organic layer was dried over $MgSO_4$ and concentrated to obtain a semicrystalline product.

Step 2): To a solution of $TiCl_4$ (18.2 ml, 0.165 mol) in $CH_2Cl_2$ (600 ml) at 0°C, was added titanium isopropoxide (16.5 ml, 0.055 mol). After 15 min, the product of Step 1 (49.0 g, 0.17 mol) was added as a solution in $CH_2Cl_2$ (100 ml). After 5 min., diisopropylethylamine (DIPEA) (65.2 ml, 0.37 mol) was added and the reaction mixture was stirred at 0°C for 1 h, the reaction mixture was cooled to -20°C, and 4-benzyloxybenzylidine(4-fluoro)aniline (114.3 g, 0.37 mol) was added as a solid. The reaction mixture was stirred vigorously for 4 h at -20°C, then acetic acid was added as a solution in $CH_2Cl_2$ dropwise over 15 min, the reaction mixture was allowed to warm to 0°C, and $H_2SO_4$ (2N) was added. The reaction mixture was stirred an additional 1 h, the layers were separated, washed with water, separated and the organic layer was dried. The crude product was crystallized from ethanol/water to obtain the pure intermediate.

Step 3): To a solution of the product of Step 2 (8.9 g, 14.9 mmol) in toluene (100 ml) at 50°C, was added N,O-bis (trimethylsilyl)acetamide (BSA) (7.50 ml, 30.3 mmol). After 0.5 h, solid TBAF (0.39 g, 1.5 mmol) was added and the reaction mixture stirred at 50°C for an additional 3 h. The reaction mixture was cooled to 22°C, $CH_3OH$ (10 ml), was added. The reaction mixture was washed with HCl (1 N), $NaHCO_3$ (1 N) and NaCl (sat'd.), and the organic layer was dried over $MgSO_4$.

Step 4): To a solution of the product of Step 3 (0.94 g, 2.2 mmol) in $CH_3OH$ (3 ml), was added water (1 ml) and $LiOH\cdot H_2O$ (102 mg, 2.4 mmole). The reaction mixture was stirred at 22°C for 1 h and then additional $LiOH\cdot H_2O$ (54 mg, 1.3 mmole) was added. After a total of 2 h, HCl (1 N) and EtOAc was added, the layers were separated, the organic layer was dried and concentrated in *vacuo.* To a solution of the resultant product (0.91 g, 2.2 mmol) in $CH_2Cl_2$ at 22°C, was added ClCOCOCl (0.29 ml, 3.3 mmol) and the mixture stirred for 16 h. The solvent was removed in *vacuo.*

Step 5): To an efficiently stirred suspension of 4-fluorophenylzinc chloride (4.4 mmol) prepared from 4-fluorophenylmagnesium bromide (1 M in THF, 4.4 ml, 4.4 mmol) and $ZnCl_2$ (0.6 g, 4.4 mmol) at 4°C, was added tetrakis (triphenylphosphine)palladium (0.25 g, 0.21 mmol) followed by the product of Step 4 (0.94 g, 2.2 mmol) as a solution in THF (2 ml). The reaction was stirred for 1 h at 0°C and then for 0.5 h at 22°C. HCl (1 N, 5 ml) was added and the mixture was extracted with EtOAc. The organic layer was concentrated to an oil and purified by silica gel chromatography to obtain 1-(4-fluorophenyl)-4(S)-(4-hydroxyphenyl)-3(R)-(3-oxo-3-phenylpropyl)-2-azetidinone:

HRMS calc'd for $C_{24}H_{19}F_2NO_3$ = 408.1429, found 408.1411.

Step 6): To the product of Step 5 (0.95 g, 1.91 mmol) in THF (3 ml), was added (R)-tetrahydro-1-methyl-3,3-diphenyl-1H,3H-pyrrolo-[1,2-c][1,3,2] oxazaborole (120 mg, 0.43 mmol) and the mixture was cooled to -20°C. After 5 min, borohydride-dimethylsulfide complex (2M in THF, 0.85 ml, 1.7 mmol) was added dropwise over 0.5 h. After a total

of 1.5 h , $CH_3OH$ was added followed by HCl (1 N) and the reaction mixture was extracted with EtOAc to obtain 1-(4-fluorophenyl)-3(R)-[3(S)-(4-fluorophenyl)-3-hydroxypropyl)]-4(S)-[4-(phenylmethoxy)phenyl]-2-azetidinone (compound 6A-1) as an oil. $^1H$ in $CDCl_3$ d H3 = 4.68. J = 2.3 Hz. CI ($M^+H$) 500.

Use of (S)-tetra-hydro-1-methyl-3,3-diphenyl-1H,3H-pyrrolo-[1,2-c][1,3,2] oxazaborole gives the corresponding 3 (R)-hydroxypropyl azetidinone (compound 6B-1). $^1H$ in $CDCl_3$ d H3 = 4.69. J = 2.3 Hz. CI ($M^+H$) 500.

To a solution of compound 6A-1 (0.4 g, 0.8 mmol) in ethanol (2 ml), was added 10% Pd/C (0.03 g) and the reaction mixture was stirred under a pressure (60 psi) of $H_2$ gas for 16 h. The reaction mixture was filtered and the solvent was concentrated to obtain compound 6A. $M_p$ 164-166°C; CI ($M^+H$) 410. $[\alpha]_D^{25} = -28.1°$ (c 3, $CH_3OH$) . Elemental analysis calc'd for $C_{24}H_{21}F_2NO_3$: C 70.41; H 5.17; N 3.42; found C 70.25; H 5.19; N 3.54.

Similarly treat compound 6B-1 to obtain compound 6B.

Mp 129.5-132.5°C; CI ($M^+H$) 410. Elemental analysis calc'd for $C_{24}H_{21}F_2NO_3$: C 70.41; H 5.17; N 3.42; found C 70.30; H 5.14; N 3.52.

Step 6' (Alternative): To a solution of the product of Step 5 (0.14 g, 0.3 mmol) in ethanol (2 ml), was added 10% Pd/C (0.03 g) and the reaction was stirred under a pressure (60 psi) of $H_2$ gas for 16 h. The reaction mixture was filtered and the solvent was concentrated to afford a 1:1 mixture of compounds 6A and 6B.

## Study in Patients with Hypercholesterolemia

An analysis of findings from a double-blind, randomized, placebo-controlled study in patients with primary hypercholesterolemia (n=668) was performed. After following NCEP diet guidelines, a drug washout and 4 week, single-blind, placebo lead-in period, patients with baseline LDL-C ≥145 to ≤250 mg/dl and TG≤350 mg/dl were randomized to one of the following administered daily for 12 consecutive weeks: a tablet formulation as described above having 10 milligrams of the compound of Formula (II) "Composition A"; SIMVASTATIN 10, 20,40 or 80 mg (available from Merck & Co., Inc.); coadministration of Composition A + SIMVASTATIN 10, 20,40 or 80mg; or placebo.

The results of the study are presented in Table 1 below.

**Table 1**

***P<0.05 for Composition A + SIMVASTATIN vs. SIMVASTATIN at each dose level; #P=0.09 vs. SIMVASTATIN 20**

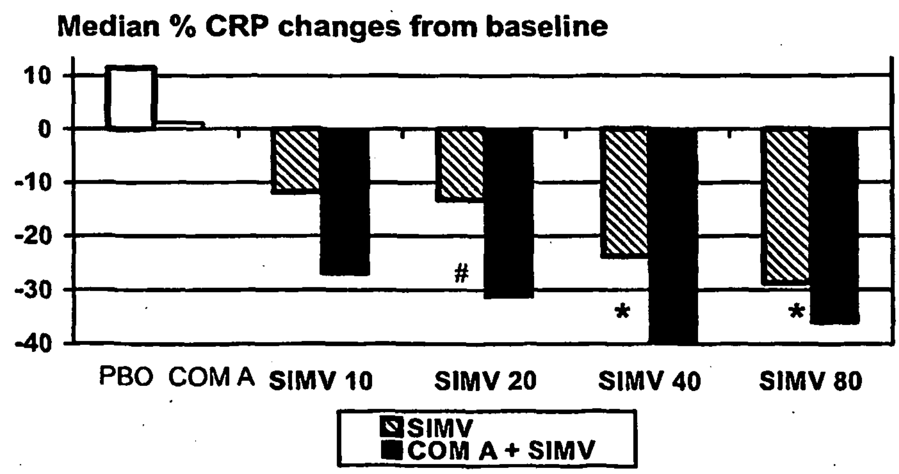


Pooled subjects treated with Composition A + SIMVASTATIN had reduced LDL-C from baseline by 49.9% vs. pooled subjects treated with SIMVASTATIN alone (36.1%, P<.01) and co-administration of Composition A + SIMVASTATIN was superior to statin alone at each SIMVASTATIN dose. Overall, median percent reductions in CRP from baseline were almost 2X greater with pooled Composition A + SIMVASTATIN vs. pooled SIMVASTATIN alone (-34.8% vs -18.2%, P<.01). Median CRP was reduced in pooled Composition A + SIMVASTATIN to 0.180mg/dL and with SIMVASTATIN to 0.215mg/dL (P=.03). CRP reductions by Composition A + SIMVASTATIN were comparable to SIMVASTATIN 80.

**Claims**

1. Use of a combination of (1) at least one sterol absorption inhibitor or at least one 5α-stanol absorption inhibitor and (2) at least one cholesterol biosynthesis inhibitor for the manufacture of a medicament for the treatment or prevention of vascular inflammation in a subject having a blood level of c-reactive protein of greater than 0.4 mg/dL.

2. The use according to claim 1, wherein the at least one sterol absorption inhibitor is represented by Formula (1):

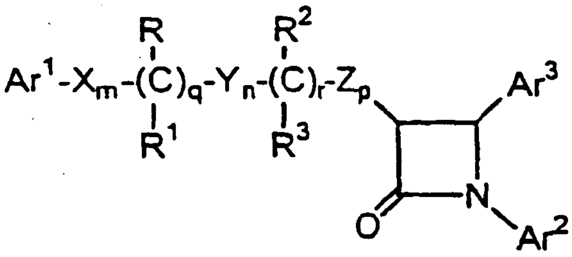

(I)

or a pharmaceutically acceptable salt thereof or a solvate thereof, wherein:

$Ar^1$ and $Ar^2$ are independently selected from the group consisting of aryl and $R^4$ -substituted aryl;
$Ar^3$ is aryl or $R^5$ -substituted aryl;
X, Y and Z are independently selected from the group consisting of $-CH_2-$, -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting or lower alkyl, $-OR^6$, $-O(CO)R^6$, $-O(CO)OR^9$, $-C(CH_2)_{1-5}OR^6$, $-O(CO)NR^6R^7$, $-NR^6R^7$,$-NR^7(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$ $S(O)_{0-2}R^9$,$-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$,$-O(CO)R^6$, $-O(CO)OR^9$, $-O(CH_2)_{1-5}OR^6$, $-O(CO)NR^6R^7$, $NR^6R^7$, $-NR^6(CO)R^7$, $-NR^6(CO)OR^9$,$-NR^6(CO)NR^7R^8$,$-NR^6SO_2R^9$,$-COOR^6$, $-CONR^6R^7$, $-COR^6$, $-SO_2NR^6R^7$, $S(O)_{0-2}R^9$, $-O(CH_2)_{10}-COOR^6$, $-O(CH_2)_{1-10}CONR^6R^7$, -(lower alkylene) $COOR^6$ and $-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl.

3. The use according to claim 2, wherein the sterol absorption inhibitor is represented by Formula (II) below:

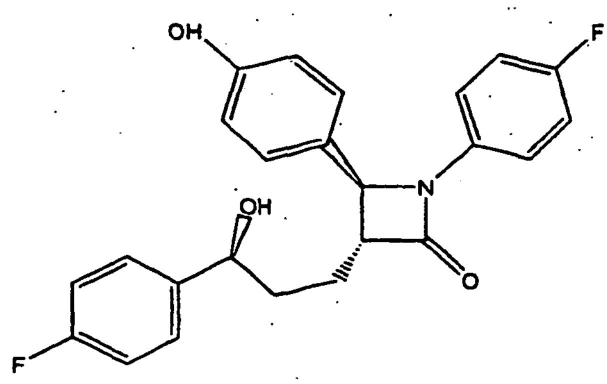

(II).

4. The use according to claim 1, wherein the at least one sterol absorption inhibitor is represented by Formula (III):

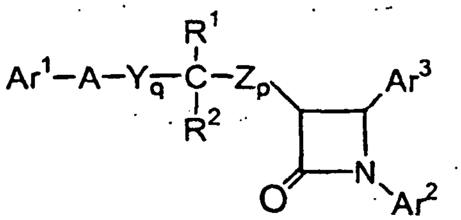

(III)

or a pharmaceutically acceptable salt thereof or a solvate thereof, wherein, in Formula (III) above:

$Ar^1$ is $R^3$-substituted aryl;
$Ar^2$ is $R^4$-substituted aryl;
$Ar^3$ is $R^5$-substituted aryl;
Y and Z are independently selected from the group consisting of $-CH_2-$, -CH(lower alkyl)-and -C(dilower alkyl),
A is selected from -O-, -S-, -S(O)- or $-S(O)_2-$;
$R^1$ is selected from the group consisting of $-OR^6$, $-O(CO)R^6$ $-O(CO)OR^9$ and $-O(CO)NR^6R^7$ ; $R^2$ is selected from the group consisting of hydrogen, lower alkyl and aryl; or $R^1$ and $R^2$ together are =O;
q is 1, 2 or 3;
p is 0,1,2,3 or 4;
$R^5$ is 1-3 substituents independently selected from the group consisting of $-OR^6$, $-O(CO)R^6$,$-O(CO)OR^9$ , $-O(CH_2)_{1-5}OR^9$, $-O(CO)NR^6R^7$, $-NR^6R^7$,$-NR^6(CO)R^7$, $-NR^6(CO)OR^9$, $-NR^6(CO)NR^7R^8$, $-NR^6SO_2$-lower alkyl, $-NR^6SO_2$-aryl; $-CONR^6R^7$, $-COR^6$ $-SO_2NR^6R^7$, $S(O)_{0-2}$-alkyl, $S(O)_{0-2}$-aryl, $-O(CH_2)_{1-10}-COOR^6$, $-O(CH_2)_{1-10}CONR^6R^7$, o-halogeno, m-halogeno, o-lower alkyl, m-lower alkyl, -(lower lkylene)-$COOR^6$, and $-CH=CH-COOR^6$;
$R^3$ and $R^4$ are independently 1-3 substituents independently selected from then group consisting of $R^5$, hydrogen, p-lower alkyl, aryl, $-NO_2$, $-CF_3$ and p-halogeno;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl.

5. The use according to claim 1, wherein the at least one sterol absorption inhibitor is represented by Formula (IV):

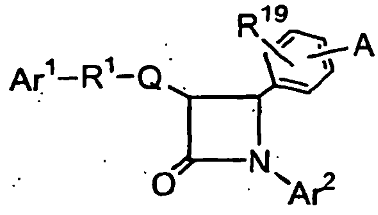

(IV)

or a pharmaceutically acceptable salt thereof or a solvate thereof, wherein, in Formula (IV) above:

A is selected from the group consisting of $R^2$-substituted heterocycloalkyl, $R^2$-substituted heteroaryl, $R^2$-substituted benzofused heterocycloalkyl, and $R^2$-substituted benzofused heteroaryl;
$Ar^1$ is aryl or $R^3$-substituted aryl;
$Ar^2$ is aryl or $R^4$-substituted aryl;
Q is a bond or, with the 3-position ring carbon of the azetidinone, forms the spiro group

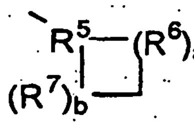

;

and
$R^1$ is selected from the group consisting of:

$-(CH_2)_q-$, wherein q is 2-6, provided that when Q forms a spiro ring, q can also be zero or 1;
$-(CH_2)_e-G-(CH_2)_r-$, wherein G is -O-, -C(O)-, phenylene, $-NR^8-$ or- $S(O)_{0-2}-$, e is 0-5 and r is 0-5, provided that the sum of e and r is 1-6;
$-(C_2-C_6$ alkenylene)-; and
$-(CH_2)_f-V-(CH_2)_g$ , wherein V is $C_3-C_6$ cycloalkylene, f is 1-5 and g is 0-5, provided that the sum of f and g is 1-6;

$R^5$ is selected from:

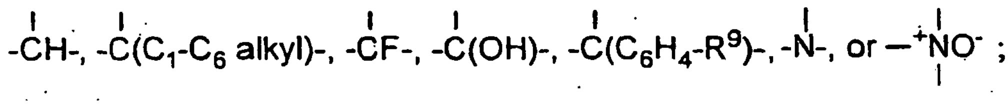

$R^6$ and $R^7$ are independently selected from the group consisting of $-CH_2-$, $-CH(C_1-C_6$ alkyl)-, $-C(di-(C_1-C_6)$ alkyl), -CH=CH- and $-C(C_1-C_6$ alkyl)=CH-; or $R^5$ together with an adjacent $R^6$ or $R^5$ together with an adjacent $R^7$, form a -CH=CH- or a $-CH=C(C_1-C_6$, alkyl)- group;
a and b are independently 0, 1, 2 or 3, provided both are not zero; provided that when $R^6$ is -CH=CH- or $-C(C_1-C_6$ alkyl)=CH-, a is 1; provided that when $R^7$ is -CH=CH- or $-C(C_1-C_6$ alkyl)=CH-, b is 1; provided that when a is 2 or 3, the $R^6$'s can be the same or different; and provided that when b is 2 or 3, the $R^7$'s can be the same or different;
and when Q is a bond, $R^1$ also can be selected from:

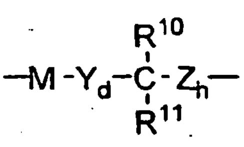

,

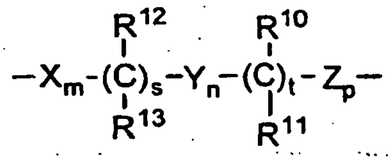

or

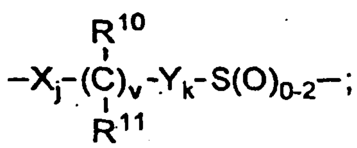

;

where M is -O-, -S-, -S(O)- or $-S(O)_2-$;
X, Y and Z are independently selected from the group consisting of $-CH_2-$, $-CH(C_1-C_6$ alkyl)- and $-C(di-(C_1-C_6)$ alkyl);
$R^{10}$ and $R^{12}$ are independently selected from the group consisting of $-OR^{14}$ $-O(CO)R^{14}$,$-O(CO)OR^{16}$ and -O (CO)$NR^{14}$ $R^{15}$;
$R^{11}$ and $R^{13}$ are independently selected from the group consisting of hydrogen, $(C_1-C_6)$alkyl, and aryl; or $R^{10}$ and $R^{11}$ together are =O, or $R^{12}$ and $R^{13}$ together are =O;
d is 1,2 or 3;
h is 0, 1, 2, 3 or 4;
s is 0 or 1; t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
$R^2$ is 1-3 substituents on the ring carbon atoms selected from the group consisting of hydrogen, $(C_1-C_{10})$alkyl, $(C_2-C_{10})$alkenyl, $(C_2-C_{10})$alkynyl, $(C_3-C_6)$cycoalkyl, $(C_3-C_6)$cycloalkenyl, $R^{17}$-substituted aryl, $R^{17}$ -substituted benzyl, $R^{17}$-substituted benzyloxy, $R^{17}$-substituted aryloxy, halogeno, $-NR^{14}R^{15}$, $NR^{14}R^{15}(C_1-C_6$ alkylene)-, $NR^{14}R^{15}C(O)(C_1-C_6$ alkylene)-$NHC(O)R^{16}$, OH; $C_1-C_6$ alkoxy, $-OC(O)R^{16}$, $COR^{14}$ hydroxy$(C_1-C_6)$alkyl, $(C_1-C_6)$alkoxy$(C_1-C_6)$alkyl, $NO_2$, $-S(O)_{0-2}R^{16}$, $-SO_2NR^{14}R^{15}$ and $-(C_1-C_6$ alkylene)$COOR^{14}$; when $R^2$ is a substituent on a heterocycloalkyl ring, $R^2$ is as defined, or is =O or

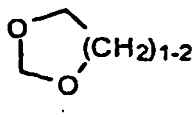 ;

and, where $R^2$ is a substituent on a substitutable ring nitrogen, it is hydrogen, $(C_1-C_6)$alkyl, aryl, $(C_1-C_6)$alkoxy, aryloxy, $(C_1-C_6)$alkylcarbonyl, arylcarbonyl, hydroxy $-(CH_2)_{1-6}CONR^{18}R^{18}$,

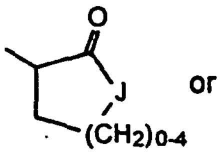 or 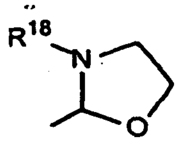 ;

wherein J is -O-, -NH-, $-NR^{18}$- or$-CH_2-$;
$R^3$ and $R^4$ are independently selected from the group consisting of 1-3 substituents independently selected from the group consisting of $(C_1-C_6)$alkyl, $-OR^{14}$ , $-O(CO)R^{14}$ , $-O(CO)OR^{16}$, $-O(CH_2)_{1-5}OR^{14}$ , -O(CO)$NR^{14}R^{15}$ , $-NR^{14}$ $R^{15}$ , $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$ $-COR^{14}$ , $-SO_2NR^{14}R^{15}$ , $S(O)_{0-2}R^{16}$ , $-O(CH_2)_{1-10}-COOR^{14}$,- $O(CH_2)_{1-10}CONR^{14}R^{15}$, $-(C_1-C_6$ alkylene)-$COOR^{14}$, -CH=CH-$COOR^{14}$, $-CF_3$, -CN,$-NO_2$ and halogen;
$R^8$ is hydrogen, $(C_1-C_6)$alkyl, aryl $(C_1-C_6)$alkyl, $-C(O)R^{14}$or $-COOR^{14}$;
$R^9$ and $R^{17}$ are independently 1-3 groups independently selected from the group consisting of hydrogen, $(C_1-C_6)$ alkyl, $(C_1-C_6)$alkoxy, -COOH, $NO_2$, $-NR^{14}R^{15}$, OH and halogeno;
$R^{14}$ and $R^{15}$ are independently selected from the group consisting of hydrogen, $(C_1-C_6)$alkyl, aryl and aryl-substituted $(C_1-C_6)$alkyl;
$R^{16}$ is $(C_1-C_6)$alkyl, aryl or $R^{17}$ -substituted aryl;
$R^{18}$ is hydrogen or $(C_1-C_6)$alkyl; and
$R^{19}$ is hydrogen, hydroxy or $(C_1-C_6)$alkoxy.

**6.** The use according to claim 1, wherein the at least one sterol absorption inhibitor is.represented by Formula (V):

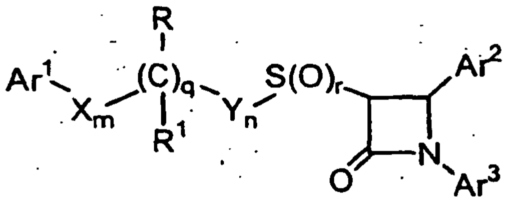

(V)

or a pharmaceutically acceptable salt thereof or a solvate thereof, wherein, in Formula (V) above:

$Ar^1$ is aryl, $R^{10}$-substituted aryl or heteroaryl;
$Ar^2$ is aryl or $R^4$-substituted aryl;
$Ar^3$ is aryl or $R^5$-substituted aryl;
X and Y are independently selected from the group consisting of $-CH_2-$, -CH(lower alkyl)- and -C(dilower alkyl)-;
R is $-OR^6$, $-O(CO)R^6$, $O(CO)OR^9$ or $-O(CO)NR^6R^7$; $R^1$ is hydrogen, lower alkyl or aryl; or R and $R^1$ together are =O;
q is 0 or1;
r is 0, 1 or 2;
m and n are independently 0, 1, 2, 3, 4 or 5; provided that the sum of m, n and q is 1,2,3,4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and-$CH=CH-COOR^6$;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $SO_2NR^6R^7$, $S(O)_{0-2}R^9$, $-O(CH_2)_{1-10}-COOR^6$, $-O(CH_2)_{1-10}CON^6R^7$ $-CF_3$, -CN, $-NO_2$, halogen,
-(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl;
$R^9$ is lower alkyl, aryl or aryl-substituted lower alkyl; and
$R^{10}$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, $-CF_3$, -CN, $-NO_2$ and halogen.

**7.** The use according to claim 1, where the at least one sterol absorption inhibitor is represented by Formula (VI):

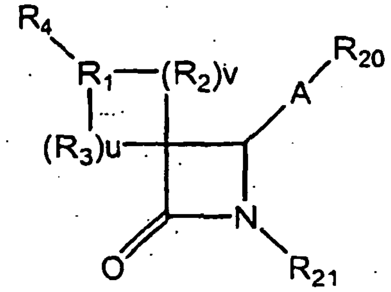

(VI)

or a pharmaceutically acceptable salt thereof or a solvate thereof, wherein:

$R_1$ is

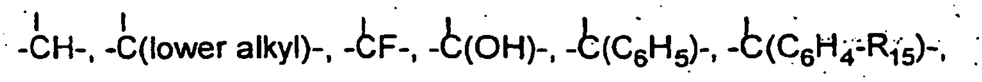

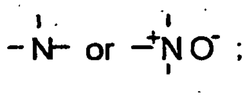

$R_2$ and $R_3$ are independently selected from the group consisting of: $-CH_2-$, -CH(lower alkyl)-, -C(di-lower alkyl)-, -CH=CH- and -C(lower alkyl)=CH-; or $R_1$ together with an adjacent $R_2$, or $R_1$ together with an adjacent $R_3$, form a -CH=CH- or a -CH=C(lower alkyl)- group;
u and v are independently 0, 1, 2 or 3, provided both are not zero; provided that when $R_2$ is -CH=CH- or-C(lower alkyl)=CH-, v is 1; provided that when $R_3$ is -CH=CH- or -C(lower alkyl)=CH-, u is 1; provided that when v is 2 or 3, the $R_2$'s can be the same or different; and provided that when u is 2 or 3, the $R_3$'s can be the same or different;
$R_4$ is selected from $B-(CH_2)_mC(O)-$, wherein m is 0, 1, 2, 3, 4 or 5;
$B-(CH_2)_q-$, wherein q is 0, 1, 2, 3, 4, 5, or 6;
$B-(CH_2)_e-Z-(CH_2)_r$, wherein Z is -O-, -C(O)-, phenylene, $-N(R_8)$-or-$S(O)_{0-2}-$, e is 0, 1, 2, 3, 4 or 5 and r is 0,1, 2, 3, 4 or 5, provided that the sum of e and r is 0,1, 2, 3, 4, . 5 or 6;
B-($C_2$-$C_6$ alkenylene)-;
B-($C_4$-$C_6$ alkadienylene)-;
$B-(CH_2)_t-Z-$($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6;
$B-(CH_2)_f-V-(CH_2)_g-$, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6;
$B-(CH_2)_t-V-$($C_2$-$C_6$ alkenylene)- or
B-($C_2$-$C_6$ alkenylene)-$V-(CH_2)_t-$, wherein V and t are as defined above, provided that the sum of t and the number of carbon atoms in the alkenylene chain is 2, 3, 4, 5 or 6;
$B-(CH_2)_a-Z-(CH_2)_b-V-(CH_2)_d-$, wherein Z and V are as defined above and a, b and d are independently 0, 1, 2, 3, 4, 5 or 6, provided that the sum of a, b and d is 0, 1, 2, 3, 4, 5 or 6; or $T-(CH_2)_s-$, wherein T is cycloalkyl of 3-6 carbon atoms and s is 0, 1, 2, 3, 4, 5 or 6; or
$R_1$ and $R_4$ together form the group

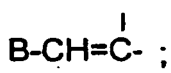

B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or

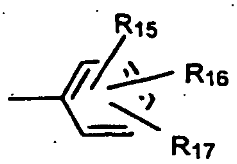

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, $-CF_3$, $-OCF_3$, benzyl, $R_7$-benzyl, benzyloxy, $R_7$-benzyloxy, phenoxy, $R_7$-phenoxy, dioxolanyl, $NO_2$,$-N(R_8)(R_9)$, $N(R_8)(R_9)$-lower alkylene-, $N(R_8)(R_9)$-lower alkylenyloxy-, OH, halogeno, -CN, $-N_3$, $-NHC(O)OR_{10}$, $-NHC(O)R_{10}$, $R_{11}O_2SNH-$, $(R_{11}O_2S)_2N-$, $-S(O)_2NH_2$, $-S(O)_{0-2}R_8$, tert-butyldimethyl-silyloxymethyl, $-C(O)R_{12}$, $-COOR_{19}$, $-CON(R_8)(R_9)$, $-CH=CHC(O)R_{12}$,- lower alkylene-$C(O)R_{12}$, $R_{10}C(O)$(lower alkylenyloxy)-, $N(R_8)(R_9)C(O)$(lower alkylenyloxy)- and

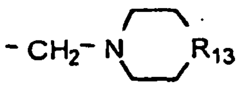

for substitution on ring carbon atoms, and the substituents on the substituted heteroaryl ring nitrogen atoms, when present, are selected from the group consisting of lower alkyl, lower alkoxy, $-C(O)OR_{10}$, $-C(O)R_{10}$, OH, $N(R_8)(R_9)$-lower alkylene-,$N(R_8)(R_9)$-lower alkylenyloxy-, $-S(O)_2NH_2$ and 2-(trimethylsilyl)-ethoxymethyl;
$R_7$ is 1-3 groups independently selected from the group consisting of lower alkyl, lower alkoxy, -COOH, $NO_2$, $-N(R_8)(R_9)$, OH, and halogeno;
$R_8$ and $R_9$ are independently selected from H or lower alkyl;
$R_{10}$ is selected from lower alkyl, phenyl, $R_7$-phenyl, benzyl or $R_7$-benzyl;
$R_{11}$ is selected from OH, lower alkyl, phenyl, benzyl, $R_7$-phenyl or $R_7$-benzyl;
$R_{12}$ is selected from H, OH, alkoxy, phenoxy, benzyloxy,

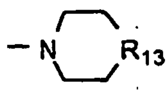

,

$-N(R_8)(R_9)$, lower alkyl, phenyl or $R_7$-phenyl;
$R_{13}$ is selected from -O-, $-CH_2-$, -NH-, -N(lower alkyl)- or $-NC(O)R_{19}$;
$R_{15}$, $R_{16}$ and $R_{17}$ are independently selected from the group consisting of H and the groups defined for W; or $R_{15}$ is hydrogen and $R_{16}$ and $R_{17}$, together with adjacent carbon atoms to which they are attached, form a dioxolanyl ring;
$R_{19}$ is H, lower alkyl, phenyl or phenyl lower alkyl; and
$R_{20}$ and $R_{21}$ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above.

**8.** The use according to claim 1, wherein the at least one sterol absorption inhibitor is represented by Formula (VIIA) or (VIIB):

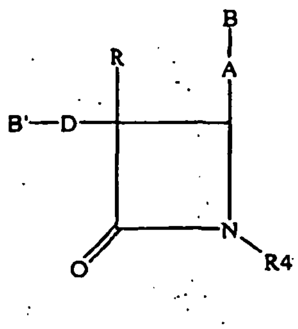

(VIIA)

or

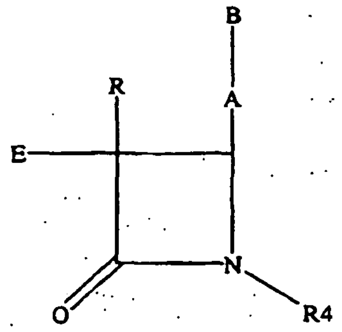

(VIIB)

or a pharmaceutically acceptable salt or solvate thereof,
wherein:

A is -CH=CH-, -C≡C- or $-(CH_2)_p-$ wherein p is 0, 1 or 2;
B is

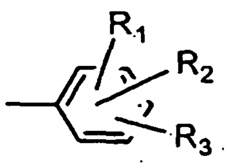

B' is

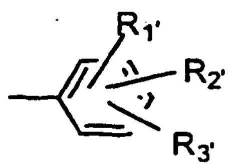

D is $-(CH_2)_mC(O)-$ or $-(CH_2)_q-$ wherein m is 1, 2, 3 or 4 and q is 2, 3 or 4;
E is $C_{10}$ to $C_{20}$ alkyl or -C(O)-($C_9$ to $C_{19}$)-alkyl, wherein the alkyl is straight or branched, saturated or containing one or more double bonds;
R is hydrogen, $C_1$-$C_{15}$ alkyl, straight or branched, saturated or containing one or more double bonds, or B-$(CH_2)_r$-, wherein r is 0, 1, 2, or 3;
$R_1$, $R_2$, $R_3$, $R_{1'}$, $R_{2'}$, and $R_{3'}$ are independently selected from the group consisting of hydrogen, lower alkyl, lower alkoxy, carboxy, $NO_2$, $NH_2$, OH, halogeno, lower alkylamino, dilower alkylamino, -NHC(O)$OR_5$, $R_6O_2SNH$- and $-S(O)_2NH_2$;
$R_4$ is

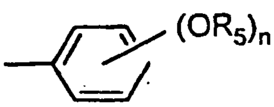

wherein n is 0, 1, 2 or 3;
$R_5$ is lower alkyl; and
$R_6$ is OH, lower alkyl, phenyl, benzyl or substituted phenyl wherein the substituents are 1-3 groups independently

selected from the group consisting of lower alkyl, lower alkoxy, carboxy, $NO_2$, $NH_2$, OH, halogeno, lower alkylamino and dilower alkylamino.

**9.** The use according to claim 1, wherein the at least one sterol absorption inhibitor is represented by Formula (VIII):

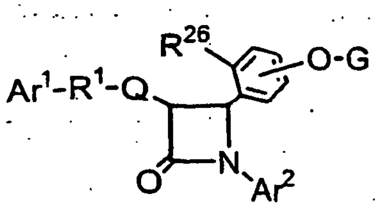

(VIII)

or a pharmaceutically acceptable salt thereof or a solvate thereof, wherein, in Formula (VIII) above,

$R^{26}$ is H or $OG^1$;
G and $G^1$ are independently selected from the group consisting of H,

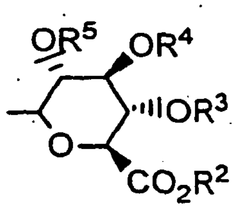
,
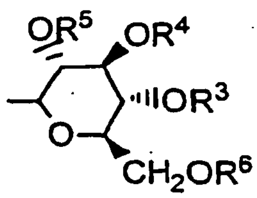
,
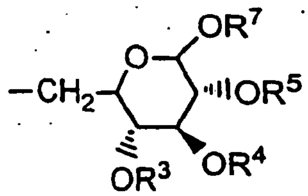

and

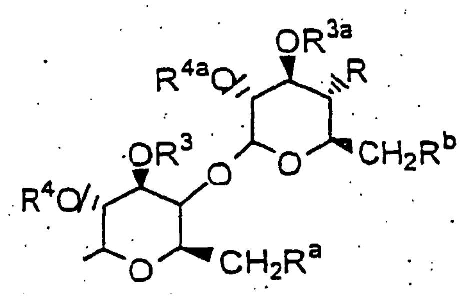
;

provided that when $R^{26}$ is H or OH, G is not H;
R, $R^a$ and $R^b$ are independently selected from the group consisting of H, -OH, halogeno, $-NH_2$, azido, $(C_1-C_6)$alkoxy$(C_1-C_6)$-alkoxy or-W-$R^{30}$;
W is independently selected from the group consisting of -NH-C(O)-, -O-C(O)-, -O-C(O)-N($R^{31}$)-, -NH-C(O)-N($R^{31}$)- and -O-C(S)-N($R^{31}$)-;
$R^2$ and $R^6$ are independently selected from the group consisting of H, $(C_1-C_6)$alkyl, aryl and aryl$(C_1-C_6)$alkyl;
$R^3$, $R^4$, $R^5$, $R^7$, $R^{3a}$ and $R^{4a}$ are independently selected from the group consisting of H, $(C_1-C_6)$alkyl, aryl$(C_1-C_6)$alkyl, -C(O)$(C_1-C_6)$alkyl and -C(O)aryl;
$R^{30}$ is selected from the group consisting of $R^{32}$-substituted T, $R^{32}$-substituted-T-$(C_1-C_6)$alkyl, $R^{32}$-substituted-$(C_2-C_4)$alkonyl, $R^{32}$-substituted-$(C_1-C_6)$alkyl, $R^{32}$-substituted-$(C_3-C_7)$cycloalkyl and $R^{32}$-substituted-$(C_3-C_7)$cycloalkyl$(C_1-C_6)$alkyl;
R31 is selected from the group consisting of H and $(C_1-C_4)$alkyl;
T is selected from the group consisting of phenyl, furyl, thienyl, pyrrolyl, oxazolyl, isoxazolyl, thiazolyl, isothiazolyl, benzothiazolyl, thiadiazolyl, pyrazolyl, imidazolyl and pyridyl;
$R^{32}$ is independently selected from 1-3 substituents independently selected from the group consisting of halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl,

$(C_1-C_4)$alkylsulfinyl, $(C_1-C_4)$alkylsulfonyl, $-N(CH_3)_2$, $-C(O)-NH(C_1-C_4)$alkyl, $-C(O)-N((C_1-C_4)alkyl)_2$, -C(O)-$(C_1-C_4)$alkyl, -C(O)-$(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$ -substituted aryl;

Q is a bond or, with the 3-position ring carbon of the azetidinone, forms the spiro group

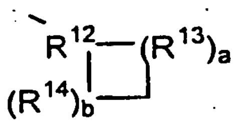 ;

and

$R^1$ is selected from the group consisting of

- $-(CH_2)_q-$, wherein q is 2-6, provided that when Q forms a spiro ring, q can also be zero or 1;
- $-(CH_2)_e-E-(CH_2)_r-$, wherein E is -O-, -C(O)-, phenylene; $-NR^{22-}$ or $-S(O)_{0-2}-$, e is 0-5 and r is 0-5, provided that the sum of e and r is 1-6;
- $-(C_2-C_6)$alkenylene-; and
- $-(CH_2)_f-V-(CH_2)_g-$, wherein V is $C_3-C_6$ cycloalkylene, f is 1-5 and g is 0-5, provided that the sum of f and g is 1-6;

$R^{12}$ is

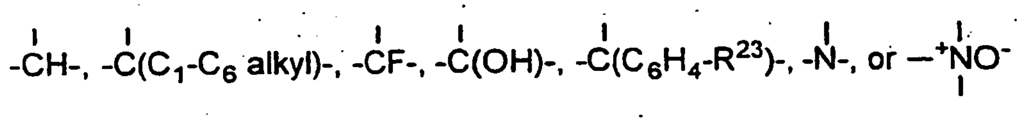

$R^{13}$ and $R^{14}$ are independently selected from the group consisting of $-CH_2-$, $-CH(C_1-C_6$ alkyl)-, -C(di-$(C_1-C_6)$ alkyl), -CH=CH- and $-C(C_1-C_6$ alkyl)=CH-; or $R^{12}$ together with an adjacent $R^{13}$, or $R^{12}$ together with an adjacent $R^{14}$, form a -CH=CH- or a $-CH=C(C_1-C_6$ alkyl)- group;

a and b are independently 0, 1, 2 or 3, provided both are not zero;

provided that when $R^{13}$ is -CH=CH- or $-C(C_1-C_6$ alkyl)=CH-, a is 1;

provided that when $R^{14}$ is -CH=CH- or $-C(C_1-C_6$ alkyl)=CH-, b is 1;

provided that when a is 2 or 3, the $R^{13}$'s can be the same or different; and

provided that when b is 2 or 3, the $R^{14}$'s can be the same or different;

and when Q is a bond, $R^1$ also can be:

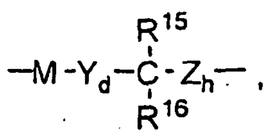 , 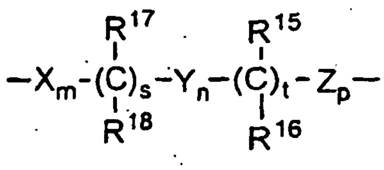 or 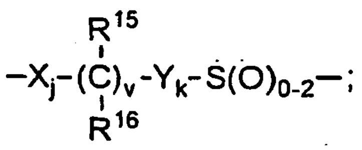

M is -O-, -S-, -S(O)- or $-S(O)_2-$;

X, Y and Z are independently selected from the group consisting of $-CH_2-$, $-CH(C_1-C_6)$alkyl- and -C(di-$(C_1-C_6)$ alkyl);

$R^{10}$ and $R^{11}$ are independently selected from the group consisting of 1-3 substituent independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene)-$COOR^{19}$, $-CH=CH-COOR^{19}$, $-CF_3$, -CN, $-NO_2$ and halogen;

$R^{15}$ and $R^{17}$ are independently selected from the group consisting of $-OR^{19}$, $-O(CO)R^{19}$, $-O(CO)OR^{21}$ and -O

(CO)$NR^{19}R^{20}$;
$R^{16}$ and $R^{18}$ are independently selected from the group consisting of H, $(C_1-C_6)$alkyl and aryl; or $R^{15}$ and $R^{16}$ together are =O, or $R^{17}$ and $R^{18}$ together are =O;
d is 1, 2 or 3;
h is 0, 1, 2, 3 or 4;
s is 0 or 1; t is 0 or 1; m, n and p are independently 0-4;
provided that at least one of s and t is 1, and the sum of m, n, p, s and t is 1-6;
provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
and when Q is a bond and $R^1$ is

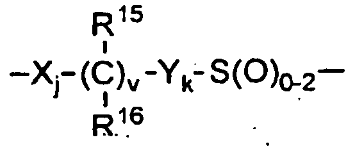
,

$Ar^1$ can also be pyridyl, isoxazolyl, furanyl, pyrrolyl, thienyl, imidazolyl, pyrazolyl, thiazolyl, pyrazinyl, pyrimidinyl or pyridazinyl;
$R^{19}$ and $R^{20}$ are independently selected from the group consisting of H, $(C_1-C_6)$alkyl, aryl and aryl-substituted $(C_1-C_6)$alkyl;
$R^{21}$ is $(C_1-C_6)$alkyl, aryl or $R^{24}$-substituted aryl;
$R^{22}$ is H, $(C_1-C_6)$alkyl, aryl $(C_1-C_6)$alkyl, -C(O)$R^{19}$ or -COOR$^{19}$;
R23 and $R^{24}$ are independently 1-3 groups independently selected from the group consisting of H, $(C_1-C_6)$alkyl, $(C_1-C_6)$alkoxy, -COOH, $NO_2$, -$NR^{19}R^{20}$, -OH and halogeno; and
$R^{25}$ is H, -OH or $(C_1-C_6)$alkoxy.

**10.** The use according to claim 1, wherein the at least one sterol absorption inhibitor is represented by Formula (IX):

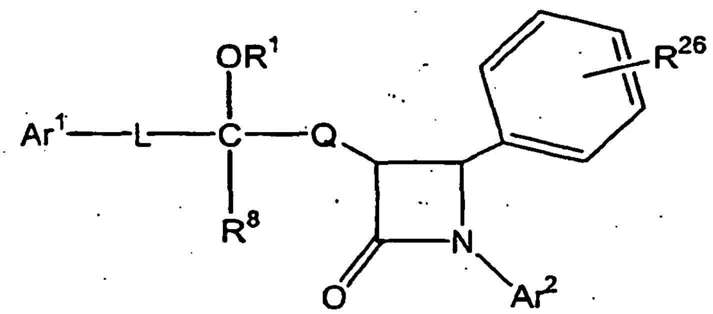
(IX)

or a pharmaceutically acceptable salt or solvate thereof, wherein in Formula (IX):

$R^1$ is selected from the group consisting of H, G, $G^1$, $G^2$, -$SO_3$H and -$PO_3$H;
G is selected from the group consisting of: H,

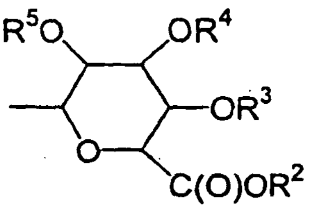 , 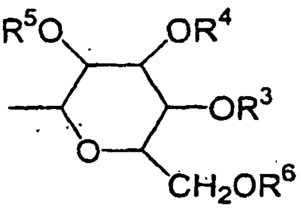 , 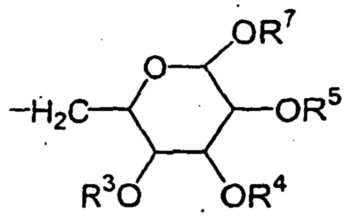 ,

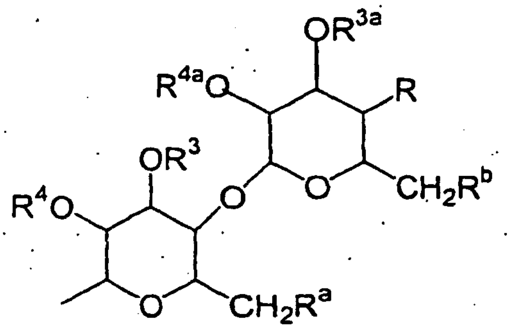 and 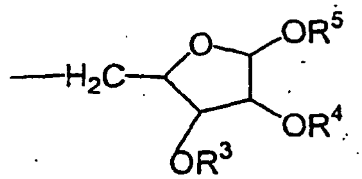 .

wherein R, $R^a$ and $R^b$ are each independently selected from the group consisting of H, -OH, halo, $-NH_2$, azido, $(C_1-C_6)$alkoxy$(C_1-C_6)$alkoxy or $-W-R^{30}$;

W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are each independently selected from the group consisting of H, $(C_1-C_6)$alkyl, acetyl, aryl and aryl$(C_1-C_6)$alkyl;

$R^3$, $R^4$, $R^5$, $R^7$, $R^{3a}$ and $R^{4a}$ are each independently selected from the group consisting of H, $(C_1-C_6)$alkyl, acetyl, aryl$(C_1-C_6)$alkyl, $-C(O)(C_1-C_6)$alkyl and-C(O)aryl;

$R^{30}$ is independently selected from the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3-C_7)$cycloalkyl and $R^{32}$-substituted-$(C_3-C_7)$cycloalkyl$(C_1-C_6)$alkyl;

$R^{31}$ is independently selected from the group consisting of H and $(C_1-C_4)$alkyl;

T is independently selected from the group consisting of phenyl, furyl, thienyl, pyrrolyl, oxazolyl, isoxazolyl, thiazolyl, isothiazolyl, benzothiazolyl, thiadiazolyl, pyrazolyl, imidazolyl and pyridyl;

$R^{32}$ is independently selected from 1-3 substituents which are each independently selected from the group consisting of H, halo, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl, $-C(O)-N((C_1-C_4)$alkyl$)_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$G^1$ is represented by the structure:

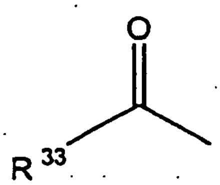

wherein $R^{33}$ is independently selected from the group consisting of unsubstituted alkyl, $R^{34}$-substituted alkyl, $(R^{35})(R^{36})$alkyl-,

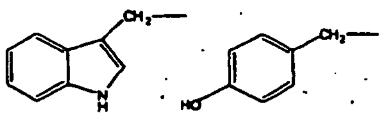 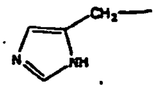 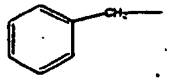

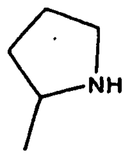 and 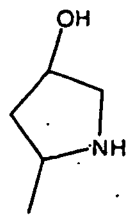 ;

$R^{34}$ is one to three substituents, each $R^{34}$ being independently selected from the group consisting of HOOC-, HS-, $(CH_3)S$-, $H_2N$-, $(NH_2)(NH)C(NH)$-, $(NH_2)C(O)$- and $HOOCCH(NH_3^+)CH_2SS$-;
$R^{35}$ is independently selected from the group consisting of H and $NH_2$-;
$R^{36}$ is independently selected from the group consisting of H, unsubstituted alkyl, $R^{34}$-substituted alkyl, unsubstituted cycloalkyl and $R^{34}$-substituted cycloalkyl;
$G^2$ is represented by the structure:

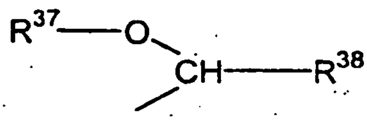

wherein $R^{37}$ and $R^{38}$ are each independently selected from the group consisting of $(C_1$-$C_6)$alkyl and aryl;
R26 is one to five substituents, each $R^{26}$ being independently selected from the group consisting of:

a) H;
b) -OH;
c) $-OCH_3$;
d) fluorine;
e) chlorine;
f) -O-G;
g) $-O-G^1$;
h) $-O-G^2$;
i) $-SO_3H$; and
j) $-PO_3H$;

provided that when $R^1$ is H, $R^{26}$ is not H, -OH, $-OCH_3$ or -O-G;
$Ar^1$ is aryl, $R^{10}$-substituted aryl, heteroaryl or $R^{10}$-substituted heteroaryl;
$Ar^2$ is aryl, $R^{11}$-substituted aryl, heteroaryl or $R^{11}$-substituted heteroaryl;
L is selected from the group consisting of:

a) a covalent bond;
b) $-(CH_2)_q$-, wherein q is 1-6;
c) $-(CH_2)_e-E-(CH_2)_r$-, wherein E is -O-, -C(O)-, phenylene, $-NR^{22}$- or $-S(O)_{0-2}$-, e is 0-5 and r is 0-5, provided that the sum of e and r is 1-6;
d) $-(C_2$-$C_6)$alkenylene-;
e) $-(CH_2)_f-V-(CH_2)_g$-, wherein V is $C_3$-$C_6$cycloalkylene, f is 1-5 and g is 0-5, provided that the sum of f and g is 1-6; and
f)

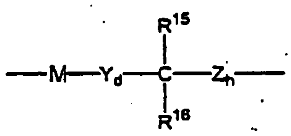, 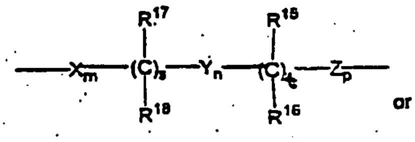 or 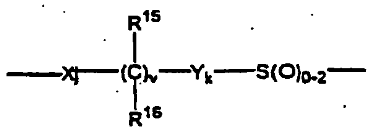 ;

wherein M is -O-, -S-, -S(O)- or $-S(O)_2-$;
X, Y and Z are each independently selected from the group consisting of $-CH_2-$, $-CH(C_1-C_6)alkyl-$ and $-C(di-(C_1-C_6)alkyl)-$;

$R^8$ is selected from the group consisting of H and alkyl;
$R^{10}$ and $R^{11}$ are each independently selected from the group consisting of 1-3 substituents which are each independently selected from the group consisting of $(C_1-C_6)$alkyl,- $OR^{19}$, $-O(CO)R^{19}$, $-O(CO)OR^{21}$, $-O(CH_2)_{1-5}OR^{19}$, $O(CO)NR^{19}R^{20}$,$-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$,$-SO_2NR^{19}R^{20}$ $S(O)_{0-2}R^{21}$, $-O(CH_2)_{1-10}-COOR^{19}$, $-O(CH_2)_{1-10}CONR^{19}R^{20}$, $-(C_1-C_6$ alkylene)-$COOR^{19}$, -CH=CH-$COOR^{19}$, $-CF_3$, =CN, $-NO_2$ and halo;
$R^{15}$ and $R^{17}$ are each independently selected from the group consisting of $-OR^{19}$, $-OC(O)R^{19}$, $-OC(O)OR^{21}$, $-OC(O)NR^{19}R^{20}$;
$R^{16}$ and $R^{13}$ are each independently selected from the group consisting of H, $(C_1-C_6)$alkyl and aryl;
or $R^{15}$ and $R^{16}$ together are =O, or $R^{17}$ and $R^{18}$ together are =O;
d is 1, 2 or 3;
h is 0, 1, 2, 3 for 4;
s is 0 or 1;
t is 0 or 1;
m, n and p are each independently selected from 0-4;
provided that at least one of s and t is 1, and the sum of m, n, p, s and t is 1-6; provided that when p is 0 and t is 1, the sum of m, n and p is 1-5; and provided that when p is 0 and s is 1. the sum of m, t and n is 1-5;
v is 0 or 1;
j and k are each independently 1-5, provided that the sum of j, k and v is 1-5;
Q is a bond, $-(CH_2)_q-$, wherein q is 1-6, or, with the 3-position ring carbon of the azetidinone, forms the spiro group

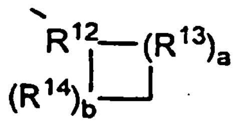 ;

wherein $R^{12}$ is

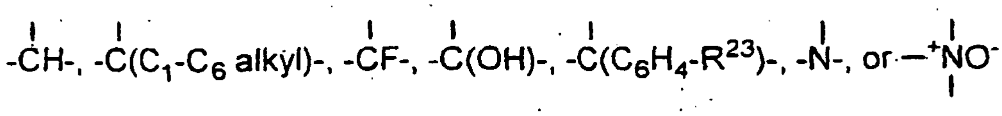 ;

$R^{13}$ and $R^{14}$ are each independently selected from the group consisting or $-CH_2-$, $-CH(C_1-C_6$ alkyl)-, $-C(di-(C_1-C_6)$ alkyl), -CH=CH- and $-C(C_1-C_6$ alkyl)=CH-; or $R^{12}$ together with an adjacent $R^{13}$, or $R^{12}$ together with an adjacent $R^{14}$, form a-CH=CH- or a $-CH=C(C_1-C_6$ alkyl)- group;
a and b are each independently 0, 1, 2 or 3, provided both are not zero; provided that when $R^{13}$ is -CH=CH-or-$C(C_1-C_6$ alkyl)=CH-, a is 1; provided that when $R^{14}$ is -CH=CH- or $-C(C_1-C_6$ alkyl)=CH=, b is 1; provided that when a is 2 or 3, the $R^{13}$'s can be the same or different; and provided that when b is 2 or 3, the $R^{14}$'s can be the same or different;
and when Q is a bond and L is

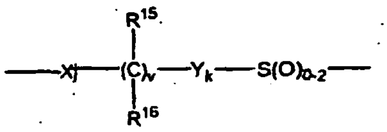

then $Ar^1$ can also be pyridyl, isoxazolyl, furanyl, pyrrolyl, thienyl, imidazolyl, pyrazolyl, thiazolyl, pyrazinyl, pyrimidinyl or pyridazinyl;
$R^{19}$ and $R^{20}$ are each independently selected from the group consisting of H, $(C_1-C_6)$alkyl, aryl and aryl-substituted $(C_1-C_6)$alkyl;
$R^{21}$ is $(C_1-C_6)$alkyl, aryl or $R^{24}$-substituted aryl;
$R^{22}$ is H, $(C_1-C_6)$alkyl, aryl $(C_1-C_6)$alkyl, $-C(O)R^{19}$ or-$COOR^{19}$;
$R^{23}$ and $R^{24}$ are each independently selected from the group consisting of 1-3 substituents which are each independently selected from the group consisting of H, $(C_1-C_6)$alkyl, $(C_1-C_6)$alkoxy, -COOH, $NO_2$, $-NR^{19}R^{20}$, -OH and halo; and
R25 is H, -OH or $(C_1-C_6)$alkoxy.

**11.** The use according to claim 1, wherein the c-reactive protein blood level is greater than about 1.0 mg/dL.

**12.** The use according to claim 1, wherein the c-reactive protein blood level is greater than about 3.4 mg/dL.

**13.** The use according to claim 1, further comprising administering at least one peroxisome proliferator-activated receptor activator.

**14.** The use according to claim 13, wherein the at least one .peroxisome proliferator-activated receptor activator is a fibric acid derivative.

**15.** The use according to claim 14 wherein the fibric acid derivative is selected from the group consisting of fenofibrate, clofibrate, gemfibrozil, ciprofibrate, bezafibrate, clinofibrate, binifibrate, lifibrol and mixtures thereof.

**16.** The use according to claim 1, wherein the at least one sterol absorption inhibitor is administered to a mammal in an amount ranging from about 0.1 to about 1000 milligrams of sterol absorption inhibitor per day.

**17.** The use according to claim 1, wherein the at least one cholesterol biosynthesis inhibitor comprises at least one HMG CoA reductase inhibitor.

**18.** The use according to claim 17, therein the at least one HMG CoA reductase inhibitor is selected from the group consisting of lovastatin, pravastatin, fluvastatin, simvastatin, atorvastatin, cerivastatin and mixtures thereof.

**19.** The use according to claim 1, further comprising administering at least one bile acid sequestrant.

**20.** The use according to claim 1, further comprising administering nicotinic acid or a derivative thereof.

**21.** The use according to claim 1, further comprising administering at least one AcylCoA: Cholesterol O-acyltransferase Inhibitor.

**22.** The use according to claim 1, further comprising administering probucol or a derivative thereof.

**23.** The use according to claim 1, further comprising administering at least one low-density lipoprotein receptor activator.

**24.** The use according to claim 1, further comprising administering at least one Omega 3 fatty acid.

**25.** The use according to claim 1, further comprising administering at least one natural water soluble fiber.

**26.** The use according to claim 1, further comprising administering at least one of plant sterols, plant stanols or fatty acid esters of plant stanols.

**27.** The use according to claim 1, further comprising administering at least one antioxidant or vitamin.

**28.** The use according to claim 1, comprising the step of administering a therapeutically effective amount of the sterol absorption inhibitor and a pharmaceutically acceptable carrier.

**29.** The use according to claim 1, wherein the subject is a human.

**30.** Use of a combination of (1) at least one sterol absorption inhibitor or at least one 5α-stanol absorption inhibitor and (2) at least one cholesterol, biosynthesis inhibitor for the manufacture of a medicament for reducing vascular c-reative protein levels in mammals, having a blood level of c-reactive protein of greater than 0.4 mg/dL.

**31.** The use according to claim 30, wherein the c-reactive protein blood level is reduced to about 3.4 mg/dL or lower.

**32.** The use according to claim 30, wherein the c-reactive protein blood level is reduced to about 1.0 mg/dL or lower.

**33.** The use according to claim 30, wherein the c-reactive protein blood level is reduced to about 0.4 mg/dL or lower.

**Patentansprüche**

**1.** Verwendung einer Kombination aus (1) mindestens einem Sterolabsorptionsinhibitor oder mindestens einem 5α-Stanolabsorptionsinhibitor und (2) mindestens einem Cholesterol-Biosyntheseinhibitor zur Herstellung eines Medikaments für die Behandlung oder Prävention einer Gefäßentzündung in einem Subjekt mit einem Blutspiegel von c-reaktivem Protein von mehr als 0,4 mg/dl.

**2.** Verwendung gemäß Anspruch 1, worin der mindestens eine Sterolabsorptionsinhibitor durch die folgende Formel (I) dargestellt wird:

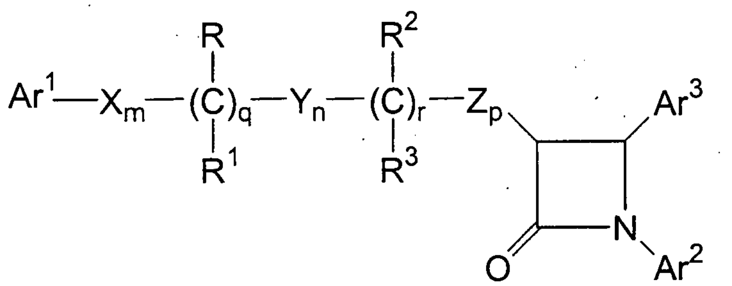

(I)

oder ein pharmazeutisch akzeptables Salz davon oder ein Solvat davon,
worin:

$Ar^1$ und $Ar^2$ unabhängig ausgewählt sind aus der Gruppe bestehend aus Aryl und $R^4$-substituiertem Aryl;
$Ar^3$ Aryl oder $R^5$-substituiertes Aryl ist;
X, Y und Z unabhängig ausgewählt sind aus der Gruppe bestehend aus $-CH_2-$, -CH(Niederalkyl)- und -C(di-Niederalkyl)-;
R und $R^2$ unabhängig ausgewählt sind aus der Gruppe bestehend aus $-OR^6$, $-O(CO)R^6$, $-O(CO)OR^9$ und $-O(CO)NR^6R^7$;
$R^1$ und $R^3$ unabhängig ausgewählt sind aus der Gruppe bestehend aus Wasserstoff, Niederalkyl und Aryl;
q 0 oder 1 ist;
r 0 oder 1 ist;
m, n und p unabhängig ausgewählt sind aus 0, 1, 2, 3 oder 4; mit der Maßgabe, daß mindestens eines von q und r 1 ist und die Summe von m, n, p, q und r 1, 2, 3, 4, 5 oder 6 ist; und mit der Maßgabe, daß, wenn p 0 und r 1 ist, die Summe von m, q und n 1, 2, 3, 4 oder 5 ist;
$R^4$ 1 bis 5 Substituenten unabhängig ausgewählt aus der Gruppe bestehend aus Niederalkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, -O

$(CH_2)_{1-10}CONR^6R^7$, -(Niederalkylen)$COOR^6$, $-CH=CH-COOR^6$, $-CF_3$, -CN, $-NO_2$ und Halogen darstellt;

$R^5$ 1 bis 5 Substituenten unabhängig ausgewählt aus der Gruppe bestehend aus $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, - (Niederalkylen) $COOR^6$ und $-CH=CH-COOR^6$ darstellt;

$R^6$, $R^7$ und $R^8$ unabhängig ausgewählt sind aus der Gruppe bestehend aus Wasserstoff, Niederalkyl, Aryl und Arylsubstituiertem Niederalkyl; und

$R^9$ Niederalkyl, Aryl oder Aryl-substituiertes Niederalkyl ist.

**3.** Verwendung gemäß Anspruch 2, worin der mindestens eine Sterolabsorptionsinhibitor durch die folgende Formel (II) dargestellt wird:

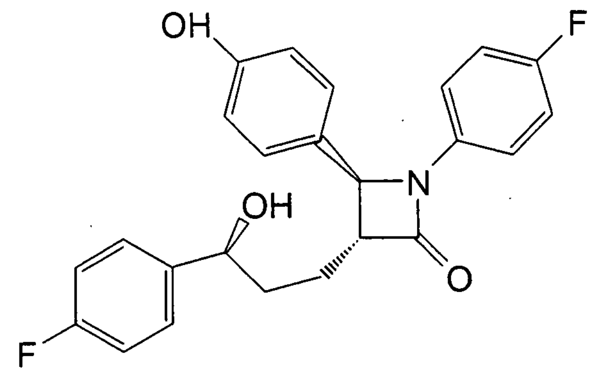

(II)

**4.** Verwendung gemäß Anspruch 1, worin der mindestens eine Sterolabsorptionsinhibitor durch die Formel (III) dargestellt wird:

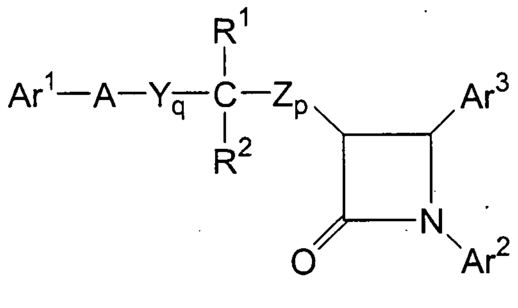

(III)

oder ein pharmazeutisch akzeptables Salz davon oder ein Solvat davon, worin in der obigen Formel (III):

$Ar^1$ $R^3$-substituiertes Aryl ist;

$Ar^2$ $R^4$-substituiertes Aryl ist;

$Ar^3$ $R^5$-substituiertes Aryl ist;

Y und Z unabhängig ausgewählt sind aus der Gruppe bestehend aus $-CH_2-$, -CH(Niederalkyl)- und -C(di-Niederalkyl)-;

A ausgewählt ist aus -O-, -S-, -S(O)- oder $-S(O)_2-$;

$R^1$ ausgewählt ist aus der Gruppe bestehend aus $-OR^6$, $-O(CO)R^6$, $-O(CO)OR^9$ und $-O(CO)NR^6R^7$; $R^2$ ausgewählt ist aus der Gruppe bestehend aus Wasserstoff, Niederalkyl und Aryl; oder $R^1$ und $R^2$ zusammen =O sind;

q 1, 2 oder 3 ist;

p 0, 1, 2, 3 oder 4 ist;

$R^5$ 1 bis 3 Substituenten unabhängig ausgewählt aus der Gruppe bestehend aus $-OR^6$, $-O(CO)R^6$ $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-Niederalkyl, $-NR^6SO_2$-Aryl, $-CONR^6R^7$, $-COR^6$, $-SO_2NR^6R^7$, $S(O)_{0-2}$-Alkyl, $S(O)_{0-2}$-Aryl, $-O(CH_2)_{1-10}-COOR^6$, -O $(CH_2)_{1-10}CONR^6R^7$, o-Halogen, m-Halogen, o-Niederalkyl, m-Niederalkyl, -(Niederalkylen)-$COOR^6$ und $-CH=CH-COOR^6$ ist;

$R^3$ und $R^4$ unabhängig 1 bis 3 Substituenten unabhängig ausgewählt aus der Gruppe bestehend aus $R^5$, Wasserstoff, p-Niederalkyl, Aryl, $-NO_2$, $-CF_3$ und p-Halogen sind;
$R^6$, $R^7$ und $R^8$ unabhängig ausgewählt sind aus der Gruppe bestehend aus Wasserstoff, Niederalkyl, Aryl und Arylsubstituiertem Niederalkyl; und
$R^9$ Niederalkyl, Aryl oder Aryl-substituiertes Niederalkyl ist.

**5.** Verwendung gemäß Anspruch 1, worin der mindestens eine Sterolabsorptionsinhibitor durch die Formel (IV) dargestellt wird:

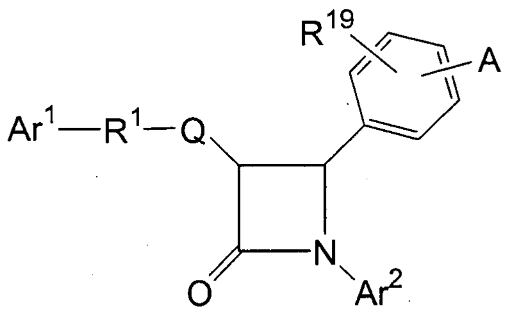

(IV)

oder ein pharmazeutisch akzeptables Salz davon oder ein Solvat davon ist, worin in der obigen Formel (IV):

A ausgewählt ist aus der Gruppe bestehend aus $R^2$-substitutiertem Heterocycloalkyl, $R^2$-substituiertem Heteroaryl, $R^2$-substituiertem benzokondensierten Heterocycloalkyl und $R^2$-substituiertem benzokondensierten Heteroaryl;
$Ar^1$ Aryl oder $R^3$-substituiertes Aryl ist;
$Ar^2$ Aryl oder $R^4$-substituiertes Aryl ist;
Q eine Bindung ist oder mit dem Ringkohlenstoff an Position 3 des Azetidinons die Spirogruppe

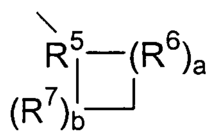

bildet; und
$R^1$ ausgewählt ist aus der Gruppe bestehend aus:

$-(CH_2)_q-$, worin q 2 bis 6 ist, mit der Maßgabe, daß, wenn Q einen Spiroring bildet, q auch 0 oder 1 sein kann;
$-(CH_2)_e-G-(CH_2)_r-$, worin G -O-, -C(O)-, Phenylen, $-NR^8-$ oder $-S(O)_{0-2}-$ ist, e 0 bis 5 und r 0 bis 5 ist, mit der Maßgabe, daß die Summe von e und r 1 bis 6 ist;
$-(C_2-C_6\text{-Alkenylen})-$; und
$-(CH_2)_f-V-(CH_2)g-$, worin V $C_3$-$C_6$-Cycloalkylen ist, f 1 bis 5 und g 0 bis 5 ist, mit der Maßgabe, daß die Summe von f und g 1 bis 6 ist;

$R^5$ ausgewählt ist aus:

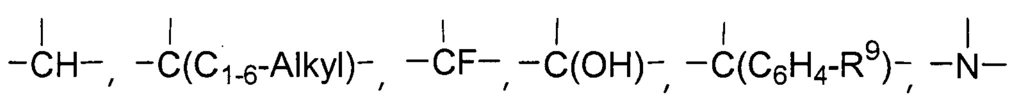

oder

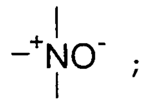

$R^6$ und $R^7$ unabhängig ausgewählt sind aus der Gruppe bestehend aus $-CH_2-$, $-CH(C_1-C_6\text{-Alkyl})-$, $-C(di-(C_{1-6}-)$ Alkyl), -CH=CH und $-C(C_1-C_6\text{-Alkyl})=CH-$; oder $R^5$ zusammen mit einem benachbarten $R^6$ oder $R^5$ zusammen mit einem benachbarten $R^7$ bilden eine -CH=CH- oder eine $-CH=C(C_1-C_6\text{-Alkyl})$-Gruppe;
a und b unabhängig 0, 1, 2 oder 3 sind, mit der Maßgabe, daß beide nicht 0 sind; mit der Maßgabe, daß, wenn $R^6$. -CH=CH- oder $-C(C_1-C_6\text{-Alkyl})=CH-$ ist, a 1 ist; mit der Maßgabe, daß, wenn $R^7$ -CH=CH- oder $-C(C_1-C_6\text{-Alkyl})=CH-$ ist, b 1 ist; mit der Maßgabe, daß, wenn a 2 oder 3 ist, die $R^6$ gleich oder verschieden sein können; und mit der Maßgabe, daß, wenn b 2 oder 3 ist, die $R^7$ gleich oder verschieden sein können;
und wenn Q eine Bindung ist, $R^1$ auch ausgewählt sein kann aus:

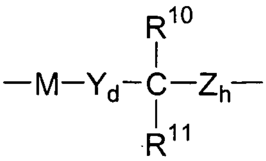 , 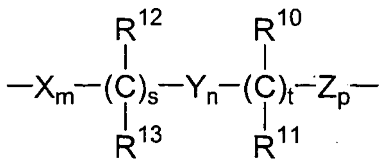 oder 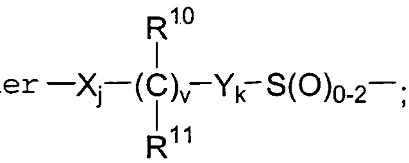 ;

worin M -O-, -S-, -S(O)- oder $S(O)_2$- ist;
X, Y und Z unabhängig ausgewählt sind aus der Gruppe bestehend aus $-CH_2-$, $-CH(C_1-C_6\text{-Alkyl})-$ und C $(di-(C_1-C_6\text{-Alkyl})$;
$R^{10}$ und $R^{12}$ unabhängig ausgewählt sind aus der Gruppe bestehend aus $-OR^{14}$, $-O(CO)R^{14}$, $-O(CO)OR^{16}$ und $-O(CO)NR^{14}R^{15}$;
$R^{11}$ und $R^{13}$ unabhängig ausgewählt sind aus der Gruppe bestehend aus Wasserstoff, $(C_1-C_6)$Alkyl und Aryl; oder $R^{10}$ und $R^{11}$ zusammen =O sind oder $R^{12}$ und $R^{13}$ zusammen =O sind;
d 1, 2 oder 3 ist;
h 0, 1, 2, 3 oder 4 ist;
s 0 oder 1 ist; t 0 oder 1 ist; m, n und p unabhängig 0 bis 4 sind; mit der Maßgabe, daß mindestens eines von s und t 1 ist und die Summe von m, n, p, s und t 1 bis 6 ist; mit der Maßgabe, daß, wenn p 0 und t 1 ist, die Summe von m, s und n 1 bis 5 ist; und mit der Maßgabe, daß, wenn p 0 und s 1 ist, die Summe von m, t und n 1 bis 5 ist;
v 0 oder 1 ist;
j und k unabhängig 1 bis 5 sind, mit der Maßgabe, daß die Summe von j, k und v 1 bis 5 ist;
$R^2$ 1 bis 3 Substituenten an Ringkohlenstoffatomen ist, ausgewählt aus der Gruppe bestehend aus Wasserstoff, $(C_1-C_{10})$Alkyl, $(C_2-C_{10})$Alkenyl, $(C_2-C_{10})$Alkinyl, $(C_3-C_6)$Cycloalkyl, $(C_3-C_6)$Cycloalkenyl, $R^{17}$-substituiertem Aryl, $R^{17}$-substituiertem Benzyl, $R^{17}$-substituiertem Benzyloxy, $R^{17}$-substituiertem Aryloxy, Halogen, $-NR^{14}R^{15}$, $NR^{14}R^{15}((C_1-C_6\text{-Alkylen})-$, $NR^{14}R^{15}C(O)(C_1-C_6\text{-Alkylen})-$, $-NHC(O)R^{16}$, OH, $C_1-C_6$-Alkoxy, $-OC(O)R^{16}$, $-COR^{14}$, Hydroxy$(C_1-C_6)$alkyl, $(C_1-C_6)$Alkoxy$(C_1-C_6)$alkyl, $NO_2$, $-S(O)_{0-2}R^{16}$, $-SO_2NR^{14}R^{15}$ und $-(C_1-C_6\text{-Alkylen})COOR^{14}$; wenn $R^2$ ein Substituent an einem Heterocycloalkylring ist, ist $R^2$ wie definiert oder =O oder

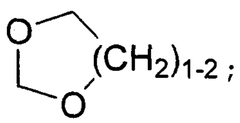

und wenn $R^2$ ein Substituent an einem substituierbaren Ringstickstoff ist, es Wasserstoff, $(C_1-C_6)$Alkyl, Aryl, $(C_1-C_6)$Alkoxy, Aryloxy, $(C_1-C_6)$Alkylcarbonyl, Arylcarbonyl, Hydroxy, $-(CH_2)_{1-6}CONR^{18}R^{18}$,

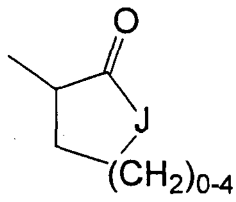
oder
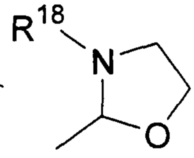

ist; worin J -O-, -NH-, -$NR^{18}$- oder -$CH_2$- ist;

$R^3$ und $R^4$ unabhängig ausgewählt sind aus der Gruppe bestehend aus 1 bis 3 Substituenten unabhängig ausgewählt aus der Gruppe bestehend aus $(C_1\text{-}C_6)$Alkyl, -$OR^{14}$, -$O(CO)R^{14}$, -$O(CO)OR^{16}$, -$O(CH_2)_{1\text{-}5}OR^{14}$, -$O(CO)NR^{14}R^{15}$, -$NR^{14}R^{15}$, -$NR^{14}(CO)R^{15}$, -$NR^{14}(CO)OR^{16}$, -$NR^{14}(CO)NR^{15}R^{19}$, -$NR^{14}SO_2R^{16}$, -$COOR^{14}$, -$CONR^{14}R^{15}$, -$COR^{14}$, -$SO_2NR^{14}R^{15}$, $S(O)_{0\text{-}2}R^{16}$, -$O(CH_2)_{1\text{-}10}$-$COOR^{14}$, -$O(CH_2)_{1\text{-}10}CONR^{14}R^{15}$, -$(C_1\text{-}C_6$-Alkylen)-$COOR^{14}$, -CH=CH-$COOR^{14}$, -$CF_3$, -CN, -$NO_2$ und Halogen;

$R^8$-Wasserstoff, $(C_1\text{-}C_6)$Alkyl, Aryl$(C_1\text{-}C_6)$alkyl, -$C(O)R^{14}$ oder -$COOR^{14}$ ist;

$R^9$ und $R^{17}$ unabhängig 1 bis 3 Gruppen sind, unabhängig ausgewählt aus der Gruppe bestehend aus Wasserstoff, $(C_1\text{-}C_6)$Alkyl, $(C_1\text{-}C_6)$Alkoxy, -COOH, $NO_2$, -$NR^{14}R^{15}$, OH und Halogen;

$R^{14}$ und $R^{15}$ unabhängig ausgewählt sind aus der Gruppe bestehend aus Wasserstoff, $(C_1\text{-}C_6)$Alkyl, Aryl und Arylsubstituiertem $(C_1\text{-}C_6)$Alkyl;

$R^{16}$ $(C_1\text{-}C_6)$Alkyl, Aryl oder $R^{17}$-substituiertes Aryl ist;

$R^{18}$ Wasserstoff oder $(C_1\text{-}C_6)$Alkyl ist; und

$R^{19}$ Wasserstoff, Hydroxy oder $(C_1\text{-}C_6)$Alkoxy ist.

**6.** Verwendung gemäß Anspruch 1, worin der mindestens eine Sterolabsorptionsinhibitor durch die Formel (V) dargestellt wird:

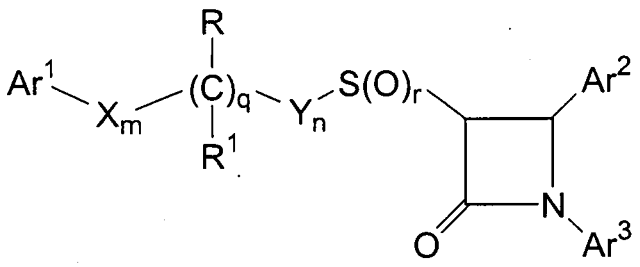
(V)

oder ein pharmazeutisch akzeptables Salz davon oder ein Solvat davon, worin in der obigen Formel (V):

$Ar^1$ Aryl, $R^{10}$-substituiertes Aryl oder Heteroaryl ist;

$Ar^2$ Aryl oder $R^4$-substituiertes Aryl ist;

$Ar^3$ Aryl oder $R^5$-substituiertes Aryl ist;

X und Y unabhängig ausgewählt sind aus der Gruppe bestehend aus -$CH_2$-, -CH(Niederalkyl)- und -C(di-Niederalkyl)-;

R -$OR^6$, -$O(CO)R^6$, -$O(CO)OR^9$ oder -O $(CO)NR^6R^7$ ist; $R^1$ Wasserstoff, Niederalkyl oder Aryl ist; oder R und $R^1$ zusammen =O sind;

q 0 oder 1 ist;

r 0, 1 oder 2 ist;

m und n unabhängig 0, 1, 2, 3, 4 oder 5 sind; mit der Maßgabe, daß die Summe von m, n und q 1, 2, 3, 4 oder 5 ist;

$R^4$ 1 bis 5 Substituenten ist, unabhängig ausgewählt aus der Gruppe bestehend aus Niederalkyl, -$OR^6$, -$O(CO)R^6$, -$O(CO)OR^9$, -$O(CH_2)_{1\text{-}5}OR^6$, -$O(CO)NR^6R^7$, -$NR^6R^7$, -$NR^6(CO)R^7$, -$NR^6(CO)OR^9$, -$NR^6(CO)NR^7R^8$, -$NR^6SO_2R^9$, -$COOR^6$, -$CONR^6R^7$, -$COR^6$, -$SO_2NR^6R^7$, $S(O)_{0\text{-}2}R^9$, -$O(CH_2)_{1\text{-}10}$-$COOR^6$, -$(CH_2)_{1\text{-}10}CONR^6R^7$, -(Niederalkylen)$COOR^6$ und -CH=CH-$COOR^6$;

$R^5$ 1 bis 5 Substituenten ist, unabhängig ausgewählt aus der Gruppe bestehend aus -$OR^6$, -$O(CO)R^6$, -$O(CO)OR^9$, -$O(CH_2)_{1\text{-}5}OR^6$, -$O(CO)NR^6R^7$, -$NR^6R^7$, -$NR^6(CO)R^7$, -$NR^6(CO)OR^9$, -$NR^6(CO)NR^7R^8$, -$NR^6SO_2R^9$, -$COOR^6$, -$CONR^6R^7$, -$COR^6$, -$SO_2NR^6R^7$, $S(O)_{0\text{-}2}R^9$, -$O(CH_2)_{1\text{-}10}$-$COOR^6$, -$O(CH_2)_{1\text{-}10}CONR^6R^7$, -$CF_3$, -CN, -$NO_2$, Halogen, -(Niederalkylen)$COOR^6$ und -CH=CH-$COOR^6$;

$R^6$, $R^7$ und $R^8$ unabhängig ausgewählt sind aus der Gruppe bestehend aus Wasserstoff, Niederalkyl, Aryl und

Arylsubstituiertem Niederalkyl;
$R^9$ Niederalkyl, Aryl oder Aryl-substituiertes Niederalkyl ist; und
$R^{10}$ 1 bis 5 Substituenten ist, unabhängig ausgewählt aus der Gruppe bestehend aus Niederalkyl, $-OR^6$, -O(CO)$R^6$,- O(CO)$OR^9$, $-O(CH_2)_{1-5}OR^6$, -O(CO)$NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, $-CF_3$, -CN, $-NO_2$ und Halogen.

**7.** Verwendung gemäß Anspruch 1, worin der mindestens eine Sterolabsorptionsinhibitor durch die Formel (VI) dargestellt wird:

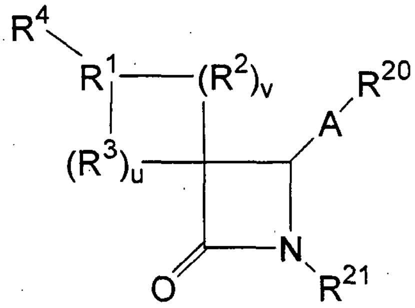

(VI)

oder ein pharmazeutisch akzeptables Salz davon oder ein Solvat davon, worin:

$R^1$

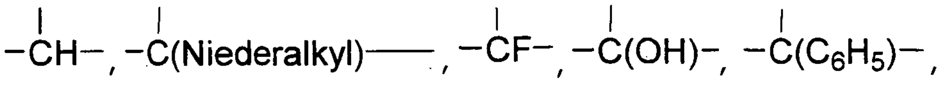

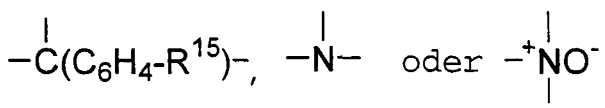

oder

ist.
$R^2$ und $R^3$ unabhängig ausgewählt sind aus der Gruppe bestehend aus $-CH_2-$, -CH(Niederalkyl)-, -C(di-Niederalkyl)-, -CH=CH- und -C(Niederalkyl)=CH-; oder $R^1$ zusammen mit einem benachbarten $R^2$ oder $R^1$ zusammen mit einem benachbarten $R^3$ eine -CH=CH- oder eine -CH=C(Niederalkyl)-Gruppe bilden;
u und v unabhängig 0, 1, 2 oder 3 sind, mit der Maßgabe, daß beide nicht 0 sind; mit der Maßgabe, daß, wenn $R^2$ -CH=CH- oder -C(Niederalkyl)=CH- ist, v 1 ist; mit der Maßgabe, daß, wenn $R^3$ -CH=CH- oder -C(Niederalkyl)=CH- ist, u 1 ist; mit der Maßgabe, daß, wenn v 2 oder 3 ist, die $R^2$ gleich oder verschieden sein können; und mit der Maßgabe, daß, wenn u 2 oder 3 ist, die $R^3$ gleich oder verschieden sein können;
$R^4$ ausgewählt ist aus $B-(CH_2)_mC(O)-$, worin m 0, 1, 2, 3, 4 oder 5 ist;
$B-(CH_2)_q-$, worin q 0, 1, 2, 3, 4, 5 oder 6 ist;
$B-(CH_2)_e-Z-(CH_2)_r-$, worin Z -O-, -C(O)-, Phenylen, $-N(R^8)-$ oder $-S(O)_{0-2}$ ist, e 0, 1, 2, 3, 4 oder 5 und r 0, 1, 2, 3, 4 oder 5 ist; mit der Maßgabe, daß die Summe von e und r 0, 1, 2, 3, 4, 5 oder 6 ist;
$B-(C_2-C_6\text{-Alkenylen})-$;
$B-(C_4-C_6\text{-Alkadienylen})-$;
$B-(CH_2)_t-Z-(C_2-C_6\text{-Alkenylen})-$, worin Z wie oben definiert ist und worin t 0, 1, 2 oder 3 ist; mit der Maßgabe, daß die Summe von t und die Zahl der Kohlenstoffatome in der Alkenylenkette 2,3,4,5 oder 6 ist;
$B-(CH_2)_f-V-(CH_2)_g-$, worin V $C_3-C_6$-Cycloalkylen ist, f 1, 2, 3, 4 oder 5 und g 0, 1, 2, 3, 4 oder 5 ist; mit der Maßgabe, daß die Summe von f und g 1, 2, 3, 4, 5 oder 6 ist;
$B-(CH_2)_t-V-(C_2-C_6\text{-Alkenylen})-$ oder
$B-(C_2-C_6\text{-Alkenylen})-V-(CH_2)_t-$, worin V und t wie oben definiert sind, mit der Maßgabe, daß die Summe von t

und die Zahl der Kohlenstoffatome in der Alkenylenkette 2, 3, 4, 5 oder 6 ist;
$B-(CH_2)_a-Z-(CH_2)_b-V-(CH_2)_d-$, worin Z und V wie oben definiert sind und a, b und d unabhängig 0, 1, 2, 3, 4, 5 oder 6 sind; mit der Maßgabe, daß die Summe von a, b und d 0, 1, 2, 3, 4, 5 oder 6 ist; oder
$T-(CH_2)_s-$, worin T ein Cycloalkyl aus 3 bis 6 Kohlenstoffatomen ist und s 0, 1, 2, 3, 4, 5 oder 6 ist; oder
$R^1$ und $R^4$ zusammen die Gruppe

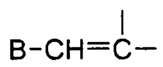

bilden;
B ausgewählt ist aus Indanyl, Indenyl, Naphthyl, Tetrahydronaphthyl, Heteroaryl oder W-substituiertem Heteroaryl, worin Heteroaryl ausgewählt ist aus der Gruppe bestehend aus Pyrrolyl, Pyridinyl, Pyrimidinyl, Pyrazinyl, Triazinyl, Imidazolyl, Thiazolyl, Pyrazolyl, Thienyl, Oxazolyl und Furanyl, und für Stickstoff enthaltende Heteroaryle, die N-Oxide davon, oder

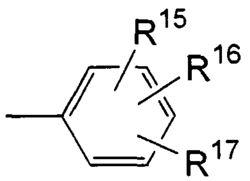

W 1 bis 3 Substituenten ist, unabhängig ausgewählt aus der Gruppe bestehend aus Niederalkyl, Hydroxy-Niederalkyl, Niederalkoxy, Alkoxyalkyl, Alkoxyalkoxy, Alkoxycarbonylalkoxy, (Niederalkoxyimino)-Niederalkyl, Niederalkandioyl, Niederalkyl-Niederalkandioyl, Allyloxy, $-CF_3$, $-OCF_3$, Benzyl, $R^7$-Benzyl, Benzyloxy, $R^7$-Benzyloxy, Phenoxy, $R^7$-Phenoxy, Dioxolanyl, $NO_2$,
$-N(R^8)(R^9)$, $N(R^8)(R^9)$-Niederalkylen-, $N(R^8)(R^9)$-Niederalkenyloxy-, OH, Halogen, -CN, $-N_3$, $-NHC(O)OR^{10}$, $-NHC(O)R^{10}$, $R^{11}O_2SNH-$, $(R^{11}O_2S)_2N-$, $-S(O)_2NH_2$, $-S(O)_{0-2}R^8$, tert-Butyldimethylsilyloxymethyl, $-C(O)R^{12}$, $-COOR^{19}$, $-CON(R^8)(R^9)$, $-CH=CHC(O)R^{12}$, -Niederalkylen-$C(O)R^{12}$, $R^{10}C(O)$(Niederalkylenyloxy)-, $N(R^8)(R^9)C(O)$(Niederalkylenyloxy)- und

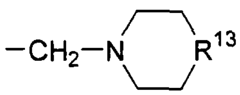

für die Substitution an Ringkohlenstoffatomen, und die Substituenten an den substituierten Heteroarylring-Stickstoffatomen, wenn vorhanden, ausgewählt sind aus der Gruppe bestehend aus Niederalkyl, Niederalkoxy, $-C(O)OR^{10}$, $-C(O)R^{10}$, OH, $N(-R^8)(R^9)$- Niederalkylen-, $N(R^8)(R^9)$- Niederalkylenyloxy-, $S(O)_2NH_2$ und 2-(Trimethylsilyl)ethoxymethyl;
$R^7$ 1 bis 3 Gruppen ist, unabhängig ausgewählt aus der Gruppe bestehend aus Niederalkyl, Niederalkoxy, -COOH, $NO_2$, $-N(R^8)(R^9)$, OH und Halogen;
$R^8$ und $R^9$ unabhängig ausgewählt sind aus H oder Niederalkyl;
$R^{10}$ ausgewählt ist aus Niederalkyl, Phenyl, $R^7$-Phenyl, Benzyl oder $R^7$-Benzyl;
$R^{11}$ ausgewählt ist aus OH, Niederalkyl, Phenyl, Benzyl, $R^7$-Phenyl oder $R^7$-Benzyl;
$R^{12}$ ausgewählt ist aus H, OH, Alkoxy, Phenoxy, Benzyloxy,

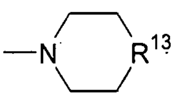
,

$-N(R^8)(R^9)$, Niederalkyl, Phenyl oder $R^7$-Phenyl;
$R^{13}$ ausgewählt ist aus -O-, $-CH_2-$, -NH-, -N(Niederalkyl)- oder $-NC(O)R^{19}$;

$R^{15}$, $R^{16}$ und $R^{17}$ unabhängig ausgewählt sind aus der Gruppe bestehend aus H und den Gruppen definiert für W; oder $R^{15}$ Wasserstoff ist und $R^{16}$ und $R^{17}$ zusammen mit den benachbarten Kohlenstoffatomen, an die sie gebunden sind, einen Dioxolanylring bilden;

$R^{19}$ H, Niederalkyl, Phenyl oder Phenyl-Niederalkyl ist; und

$R^{20}$ und $R^{21}$ unabhängig ausgewählt sind aus der Gruppe bestehend aus Phenyl, W-substituiertem Phenyl, Naphthyl, W-substituiertem Naphthyl, Indanyl, Indenyl, Tetrahydronaphthyl, Benzodioxolyl, Heteroaryl, W-substituiertem Heteroaryl, benzokondensiertem Heteroaryl, W-substituiertem benzokondensiertem Heteroaryl und Cyclopropyl, worin Heteroaryl wie oben definiert ist.

**8.** Verwendung gemäß Anspruch 1, worin der mindestens eine Sterolabsorptionsinhibitor durch die Formel (VIIA) oder (VIIB) dargestellt wird:

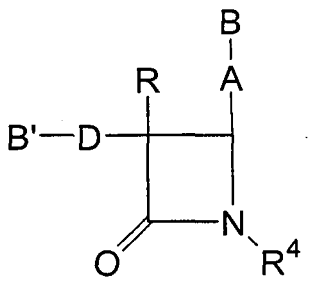

(VIIA) oder

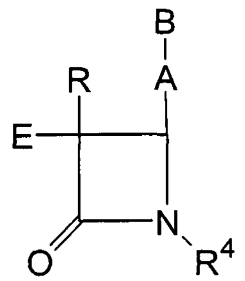

(VIIB)

oder ein pharmazeutisch akzeptables Salz oder ein Solvat davon, worin:

A -CH=CH-, -C=C- oder $-(CH_2)_p-$ ist, worin p 0, 1 oder 2 ist;

B

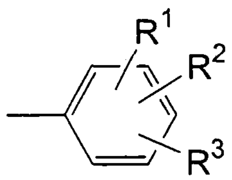

ist;

B'

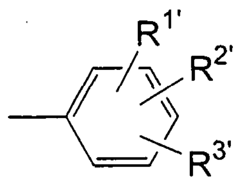

ist;

D $-(CH_2)_mC(O)-$ oder $-(CH_2)_q-$ ist, worin m 1, 2, 3 oder 4 und q 2, 3 oder 4 ist;

E $C_{10}$-$C_{20}$-Alkyl oder $-C(O)-(C_9$-$C_{19})$-Alkyl ist, worin das Alkyl unverzweigt oder verzweigt ist, gesättigt ist oder eine oder mehrere Doppelbindungen enthält;

R Wasserstoff, $C_1$-$C_{15}$-Alkyl, das unverzweigt oder verzweigt ist, gesättigt ist oder eine oder mehrere Doppelbindungen enthält, oder $B-(CH_2)_r-$, worin r 0, 1,-2 oder 3 ist, ist;

$R^1$, $R^2$, $R^3$, $R^{1'}$, $R^{2'}$ und $R^{3'}$ unabhängig ausgewählt sind aus der Gruppe bestehend aus Wasserstoff, Niederalkyl, Niederalkoxy, Carboxy, $NO_2$, $NH_2$, OH, Halogen, Niederalkylamino, di-Niederalkylamino, $-NHC(O)OR^5$,

$R^6O_2SNH$- und $-S(O)_2NH_2$;
$R^4$

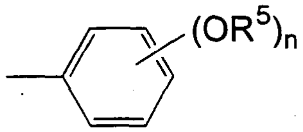

ist,
worin n 0, 1, 2 oder 3 ist;
$R^5$ Niederalkyl ist; und
$R^6$ OH, Niederalkyl, Phenyl, Benzyl oder substituiertes Phenyl ist, worin die Substituenten 1 bis 3 Gruppen unabhängig ausgewählt aus der Gruppe bestehend aus Niederalkyl, Niederalkoxy, Carboxy, $NO_2$, $NH_2$, OH, Halogen, Niederalkylamino und di-Niederalkylamino sind.

**9.** Verwendung gemäß Anspruch 1, worin der mindestens eine Sterolabsorptionsinhibitor durch die Formel (VIII) dargestellt wird:

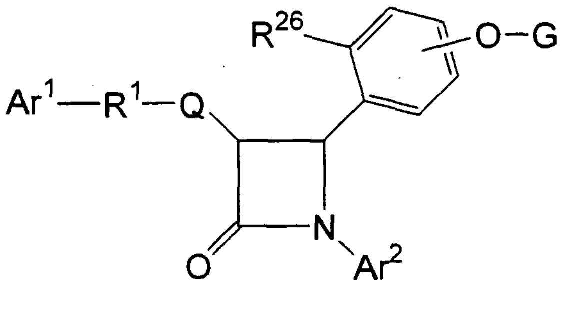

(VIII)

oder ein pharmazeutisch akzeptables Salz davon oder ein Solvat davon, worin in der obigen Formel (VIII):

$R^{26}$ H oder $OG^1$ ist;
G und $G^1$ unabhängig ausgewählt sind aus der Gruppe bestehend aus

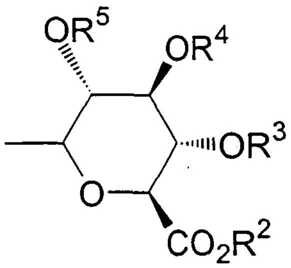 , 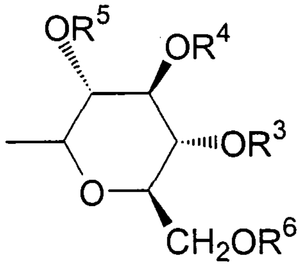 , 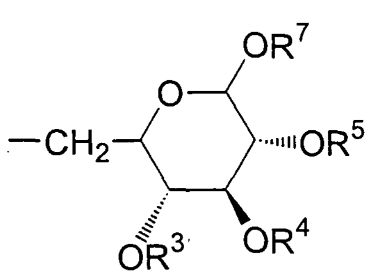

und

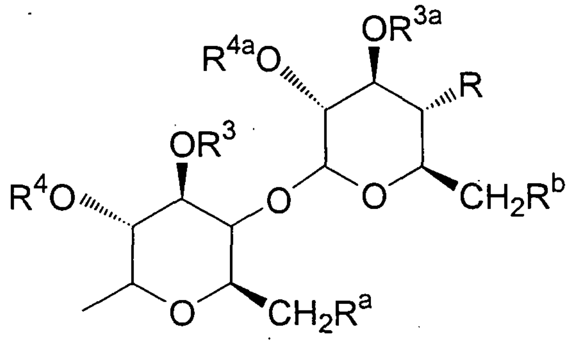 ;

mit der Maßgabe, daß, wenn $R^{26}$ H oder OH ist, G nicht H ist;

R, $R^a$ und $R^b$ unabhängig ausgewählt sind aus der Gruppe bestehend aus H, -OH, Halogen, $-NH_2$, Azido, $(C_1-C_6)$Alkoxy$(C_1-C_6)$alkoxy oder $-W-R^{30}$;

W unabhängig ausgewählt ist aus der Gruppe bestehend aus -NH-C(O)-, -O-C(O)-, $-O-C(O)-N(R^{31})-$, $-NH-C(O)-N(R^{31})-$ und $-O-C(S)-N(R^{31})-$;

$R^2$ und $R^6$ unabhängig ausgewählt sind aus der Gruppe bestehend aus H, $(C_1-C_6)$Alkyl, Aryl und Aryl$(C_1-C_6)$alkyl;

$R^3$, $R^4$, $R^5$, $R^7$, $R^{3a}$ und $R^{4a}$ unabhängig ausgewählt sind aus der Gruppe bestehend aus H, $(C_1-C_6)$Alkyl, Aryl$(C_1-C_6)$alkyl, $-C(O)(C_1-C_6)$Alkyl und -C(O)Aryl;

$R^{30}$ ausgewählt ist aus der Gruppe bestehend aus $R^{32}$-substituiertem T, $R^{32}$-substituiertem T-$(C_1-C_6)$Alkyl, $R^{32}$-substituiertem $(C_2-C_4)$Alkenyl, $R^{32}$-substituiertem $(C_1-C_6)$Alkyl, $R^{32}$-substituiertem $(C_3-C_7)$Cycloalkyl und $R^{32}$-substituiertem $(C_3-C_7)$Cycloalkyl$(C_1-C_6)$alkyl;

$R^{31}$ ausgewählt ist aus der Gruppe bestehend aus H und $(C_1-C_4)$Alkyl;

T ausgewählt ist aus der Gruppe bestehend aus Phenyl, Furyl, Thienyl, Pyrrolyl, Oxazolyl, Isoxazolyl, Thiazolyl, Isothiazolyl, Benzothiazolyl, Thiadiazolyl; Pyrazolyl, Imidazolyl und Pyridyl;

$R^{32}$ unabhängig ausgewählt ist aus 1 bis 3 Substituenten unabhängig ausgewählt aus der Gruppe bestehend aus Halogen, $(C_1-C_4)$Alkyl, -OH, Phenoxy, $-CF_3$, $-NO_2$, $(C_1-C_4)$Alkoxy, Methylendioxy, Oxo, $(C_1-C_4)$Alkylsulfanyl, $(C_1-C_4)$Alkylsulfinyl, $(C_1-C_4)$Alkylsulfonyl, $-N(CH_3)_2$, $-C(O)-NH(C_1-C_4)$Alkyl, $-C(O)-N((C_1-C_4)Alkyl)_2$, $-C(O)-(C_1-C_4)$Alkyl, $-C(O)-(C_1-C_4)$Alkoxy und Pyrrolidinylcarbonyl; oder $R^{32}$ eine kovalente Bindung ist und $R^{31}$, der Stickstoff, an das es gebunden ist, und $R^{32}$ eine Pyrrolidinyl-, eine Piperidinyl-, eine N-Methylpiperazinyl-, eine Indolinyl- oder eine Morpholinylgruppe oder eine $(C_1-C_4)$Alkoxycarbonyl-substituierte Pyrrolidinyl-, Piperidinyl-, N-Methylpiperazinyl-, Indolinyl- oder Morpholinylgruppe bilden;

$Ar^1$ Aryl oder $R^{10}$-substituiertes Aryl ist;

$Ar^2$ Aryl oder $R^{11}$-substituiertes Aryl ist;

Q eine Bindung ist oder mit dem Ringkohlenstoff an Position 3 des Azetidinons die Spirogruppe

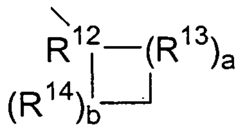

bildet; und

$R^1$ ausgewählt ist aus der Gruppe bestehend aus

$-(CH_2)_q-$, worin q 2 bis 6 ist, mit der Maßgabe, daß, wenn Q einen Spiroring bildet, q auch 0 oder 1 sein kann;

$-(CH_2)_e-E-(CH_2)_r-$; worin E -O-, =C(O)-, Phenylen, $-NR^{22}-$ oder $-S(O)_{0-2}-$ ist, e 0 bis 5 und r 0 bis 5 ist, mit der Maßgabe, daß die Summe von e und r 1 bis 6 ist; $-(C_2-C_6)$Alkenylen-; und

$-(CH2)_f-V-(CH2)_g-$, worin V $C_3-C_6$-Cycloalkylen ist, f 1 bis 5 und g 0 bis 5 ist, mit der Maßgabe, daß die Summe von f und g 1 bis 6 ist;

$R^{12}$

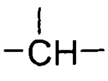, 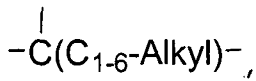, 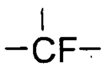, 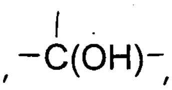, 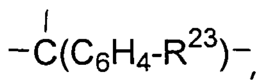, 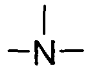

oder

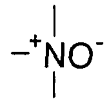

ist;
$R^{13}$ und $R^{14}$ unabhängig ausgewählt sind aus der Gruppe bestehend aus $-CH_2-$, $-CH(C_1-C_6)$Alkyl-, -C(di-$(C_1-C_6)$ Alkyl), -CH=CH- und $-C(C_1-C_6)$Alkyl=CH-; oder $R^{12}$ zusammen mit dem benachbarten $R^{13}$ oder $R^{12}$ zusammen mit einem benachbarten $R^{14}$ eine -CH=CH- oder eine $-CH=C(C_1-C_6$-Alkyl)-Gruppe bilden;
a und b unabhängig 0, 1, 2 oder 3 sind, mit der Maßgabe, daß beide nicht 0 sind;
mit der Maßgabe, daß, wenn $R^{13}$ -CH=CH- oder $-C(C_1-C_6$-Alkyl)=CH- ist, a 1 ist;
mit der Maßgabe, daß, wenn $R^{14}$ -CH=CH- oder $-C(C_1-C_6$-Alkyl)=CH- ist, b 1 ist;
mit der Maßgabe, daß, wenn a 2 oder 3 ist, die $R^{13}$ gleich oder verschieden sein können; und
mit der Maßgabe, daß, wenn b 2 oder 3 ist, die $R^{14}$ gleich oder verschieden sein können;
und wenn Q eine Bindung ist, kann $R^1$ auch sein:

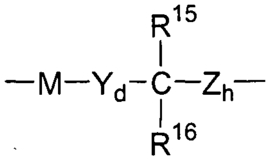, 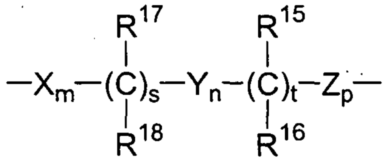 oder 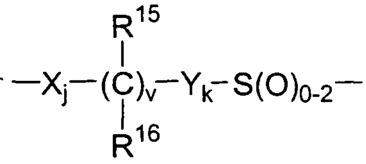;

M -O-, -S-, -S(O)- oder $-S(O)_2-$ ist;
X, Y und Z unabhängig ausgewählt sind aus der Gruppe bestehend aus $-CH_2-$, $-CH(C_1-C_6)$Alkyl- und -C (di-$(C_1-C_6)$Alkyl);
$R^{10}$ und $R^{11}$ unabhängig ausgewählt sind aus der Gruppe bestehend aus 1 bis 3 Substituenten unabhängig ausgewählt aus der Gruppe bestehend aus $(C_1-C_6)$Alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$-Alkylen)-$COOR^{19}$, $-CH=CH-COOR^{19}$, $-CF_3$, -CN, $-NO_2$ und Halogen;
$R^{15}$ und $R^{17}$ unabhängig ausgewählt sind aus der Gruppe bestehend aus $-OR^{19}$, $-O(CO)R^{19}$, $-O(CO)OR^{21}$ und $-O(CO)NR^{19}R^{20}$;
$R^{16}$ und $R^{18}$ und abhängig ausgewählt sind aus der Gruppe bestehend aus H, $(C_1-C_6)$Alkyl und Aryl; oder $R^{15}$ und $R^{16}$ zusammen =O sind oder $R^{17}$ und $R^{18}$ zusammen =O sind;
d 1, 2 oder 3 ist;
h 0, 1, 2, 3 oder 4 ist;
s 0 oder 1 ist; t 0 oder 1 ist; m, n und p unabhängig 0 bis 4 sind;
mit der Maßgabe, daß mindestens eines von s und t 1 ist und die Summe von m, n, p, s und t 1 bis 6 ist;
mit der Maßgabe, daß, wenn p 0 und t 1 ist, die Summe von m, s und n 1 bis 5 ist; und mit der Maßgabe, daß, wenn p 0 und s 1 ist, die Summe von m, t und n 1 bis 5 ist;
v 0 oder 1 ist;
j und k und abhängig 1 bis 5 sind, mit der Maßgabe, daß die Summe von j, k und v 1 bis 5 ist;
und wenn Q eine Bindung und $R^1$

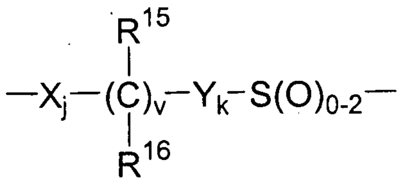

ist, kann $Ar^1$ auch Pyridyl, Isoxazolyl, Furanyl, Pyrrolyl, Thienyl, Imidazolyl, Pyrazolyl, Thiazolyl, Pyrazinyl, Pyrimidinyl oder Pyridazinyl sein;

$R^{19}$ und $R^{20}$ unabhängig ausgewählt sind aus der Gruppe bestehend aus H, $(C_1-C_6)$Alkyl, Aryl und Arylsubstituiertem $(C_1-C_6)$Alkyl;

$R^{21}$ $(C_1-C_6)$Alkyl, Aryl oder $R^{24}$-substituiertes Aryl ist;

$R^{22}$ H, $(C_1-C_6)$Alkyl, Aryl$(C_1-C_6)$alkyl, -C(O)$R^{19}$ oder -COOR$^{19}$ ist;

$R^{23}$ und $R^{24}$ unabhängig 1 bis 3 Gruppen unabhängig ausgewählt aus der Gruppe bestehend aus H, $(C_1-C_6)$ Alkyl, $(C_1-C_6)$Alkoxy, -COOH, $NO_2$, -NR$^{19}$R$^{20}$, -OH und Halogen sind; und

$R^{25}$ H, -OH oder $(C_1-C_6)$Alkoxy ist.

**10.** Verwendung gemäß Anspruch 1, worin der mindestens eine Sterolabsorptionsinhibitor durch die Formel (IX) dargestellt wird:

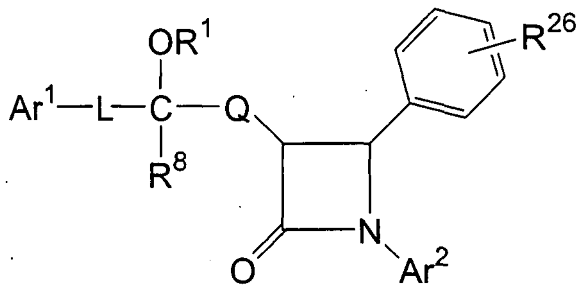

(IX)

oder ein pharmazeutisch akzeptables Salz oder Solvat davon, worin in der Formel (IX):.

$R^1$ ausgewählt ist aus der Gruppe bestehend aus H, G, $G^1$, $G^2$, $-SO_3H$ und $-PO_3H$;

G ausgewählt ist aus der Gruppe bestehend aus: H,

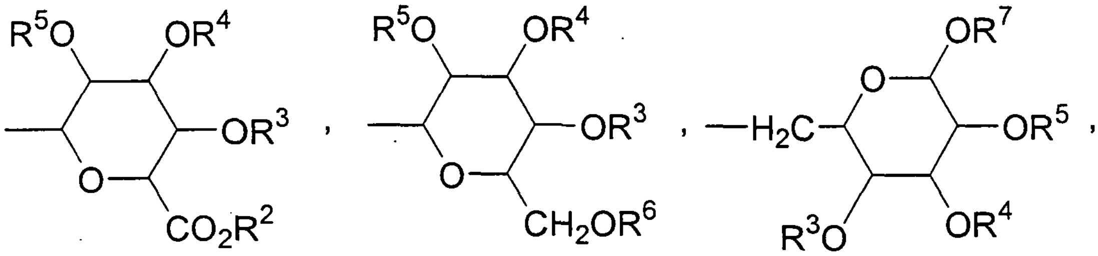

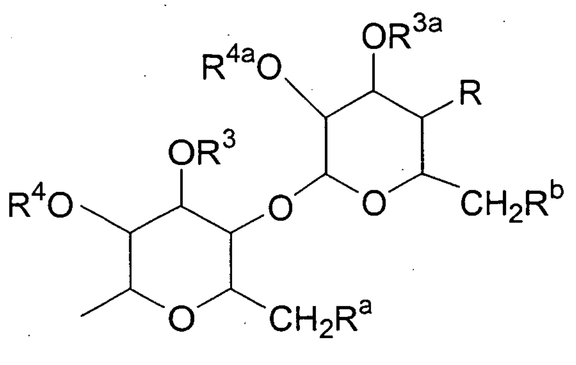 und 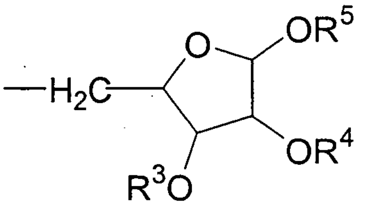 ,

worin R, $R^a$ und $R^b$ jeweils unabhängig ausgewählt sind aus der Gruppe bestehend aus H, -OH, Halogen, $-NH_2$, Azido, $(C_1\text{-}C_6)$Alkoxy$(C_1\text{-}C_6)$alkoxy oder $-W\text{-}R^{30}$;
W unabhängig ausgewählt ist aus der Gruppe bestehend aus -NH-C(O)-, -O-C(O)-, -O-C(O)-N($R^{31}$)-, -NH-C (O)-N($R^{31}$)- und -O-C(S)-N($R^{31}$)-;
$R^2$ und $R^6$ jeweils unabhängig ausgewählt sind aus der Gruppe bestehend aus H, $(C_1\text{-}C_6)$Alkyl, Acetyl, Aryl und Aryl$(C_1\text{-}C_6)$alkyl;
$R^3$, $R^4$, $R^5$, $R^7$, $R^{3a}$ und $R^{4a}$ jeweils unabhängig ausgewählt sind aus der Gruppe bestehend aus H, $(C_1\text{-}C_6)$ Alkyl, Acetyl, Aryl$(C_1\text{-}C_6)$alkyl, -C(O) $(C_1\text{-}C_6)$Alkyl und -C(O)Aryl;
$R^{30}$ unabhängig ausgewählt ist aus der Gruppe bestehend aus $R^{32}$-substituiertem T, $R^{32}$-substituiertem T-$(C_1\text{-}C_6)$Alkyl, $R^{32}$-substituiertem $(C_2\text{-}C_4)$Alkenyl, $R^{32}$-substituiertem $(C_1\text{-}C_6)$Alkyl, $R^{32}$-substituiertem $(C_3\text{-}C_7)$ Cycloalkyl und $R^{32}$-substituiertem $(C_3\text{-}C_7)$Cycloalkyl$(C_1\text{-}C_6)$alkyl;
$R^{31}$ unabhängig ausgewählt ist aus der Gruppe bestehend aus H und $(C_1\text{-}C_4)$Alkyl;
T unabhängig ausgewählt ist aus der Gruppe bestehend aus Phenyl, Furyl, Thienyl, Pyrrolyl, Oxazolyl, Isoxazolyl, Thiazolyl, Isothiazolyl, Benzothiazolyl, Thiadiazolyl, Pyrazolyl, Imidazolyl und Pyridyl;
$R^{32}$ unabhängig ausgewählt ist aus 1 bis 3 Substituenten, die jeweils unabhängig ausgewählt sind aus der Gruppe bestehend aus H, Halogen, $(C_1\text{-}C_4)$Alkyl, -OH, Phenoxy, $-CF_3$, $-NO_2$, $(C_1\text{-}C_4)$Alkoxy, Methylendioxy, Oxo, $(C_1\text{-}C_4)$Alkylsulfanyl, $(C_1\text{-}C_4)$Alkylsulfinyl, $(C_1\text{-}C_4)$Alkylsulfonyl, $-N(CH_3)_2$, -C(O)-NH$(C_1\text{-}C_4)$Alkyl, -C (O)-N($(C_1\text{-}C_4)$Alkyl$)_2$, -C(O)-$(C_1\text{-}C_4)$Alkyl, -C(O)-$(C_1\text{-}C_4)$Alkoxy und Pyrrolidinylcarbonyl; oder $R^{32}$ eine kovalente Bindung ist und $R^{31}$, der Stickstoff, an welchen es gebunden ist, und $R^{32}$ eine Pyrrolidinyl-, eine Piperidinyl-, eine N-Methylpiperazinyl-, eine Indolinyl- oder eine Morpholinylgruppe oder eine $(C_1\text{-}C_4)$Alkoxycarbonyl-substituierte Pyrrolidinyl-, Piperidinyl-, N-Methylpiperazinyl-, Indolinyl- oder Morpholinylgruppe bilden;
$G^1$ durch die Struktur

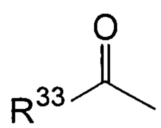

dargestellt wird, worin $R^{33}$ unabhängig ausgewählt ist aus der Gruppe bestehend aus unsubstituiertem Alkyl, $R^{34}$-substituiertem Alkyl, $(R^{35})(R^{36})$Alkyl-,

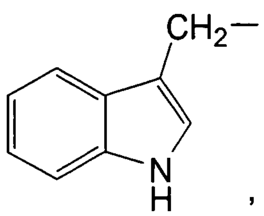 , 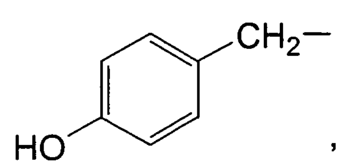 , 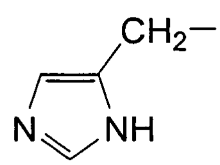 ,

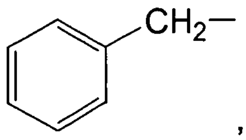, 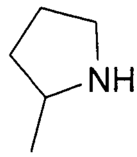 und 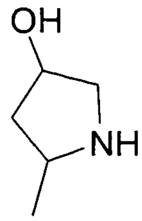 ;

$R^{34}$ 1 bis 3 Substituenten ist, wobei jedes $R^{34}$ unabhängig ausgewählt ist aus der Gruppe bestehend aus HOOC-, HS-, $(CH_3)S$-, $H_2N$-, $(NH_2)(NH)C(NH)$-, $(NH_2)C(O)$- und $HOOCCH(NH_3^+)CH_2SS$-;
$R^{35}$ unabhängig ausgewählt ist aus der Gruppe bestehend aus H und $NH_2$-;
$R^{36}$ unabhängig ausgewählt ist aus der Gruppe bestehend aus H, unsubstituiertem Alkyl, $R^{34}$-substituiertem Alkyl, unsubstituiertem Cycloalkyl und $R^{34}$-substituiertem Cycloalkyl;
$G^2$ durch die Struktur

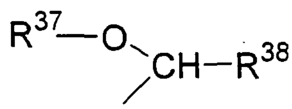

dargestellt wird, worin $R^{37}$ und $R^{38}$ jeweils unabhängig ausgewählt sind aus der Gruppe bestehend aus $(C_1$-$C_6)$ Alkyl und Aryl;
$R^{26}$ 1 bis 5 Substituenten ist, wobei jedes $R^{26}$ unabhängig ausgewählt ist aus der Gruppe bestehend aus:

a) H;
b) -OH;
c) $-OCH_3$;
d) Fluor;
e) Chlor;
f) -O-G;
g) $-O-G^1$;
h) $-O-G^2$;
i) $-SO_3H$ und
j) $-PO_3H$;

mit der Maßgabe, daß, wenn $R^1$ H ist, $R^{26}$ nicht H, -OH, $-OCH_3$ oder -O-G ist;
$Ar^1$ Aryl, $R^{10}$-substituiertes Aryl, Heteroaryl oder $R^{10}$-substituiertes Heteroaryl ist;
$Ar^2$ Aryl, $R^{11}$-substituiertes Aryl, Heteroaryl oder $R^{11}$-substituiertes Heteroaryl ist;
L ausgewählt ist aus der Gruppe bestehend aus:

a) einer kovalenten Bindung;
b) $-(CH_2)_q-$, worin q 1 bis 6 ist;
c) $-(CH_2)_e-E-(CH_2)_r-$, worin E -O-, -C(O)-, Phenylen, $-NR^{22}-$ oder $-S(O)_{0-2}-$ ist, e 0 bis 5 und r 0 bis 5 ist, mit der Maßgabe, daß die Summe von e und r 1 bis 6 ist;
d) $-(C_2$-$C_6)$Alkenylen-;
e) $-(CH_2)_f-V-(CH_2)_g-$ ist, worin V $C_3$-$C_6$-Cycloalkylen ist, f 1 bis 5 und g 0 bis 5 ist, mit der Maßgabe, daß die Summe von f und g 1 bis 6 ist; und
f)

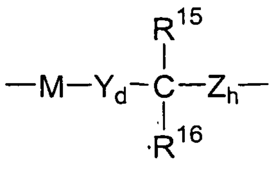 , 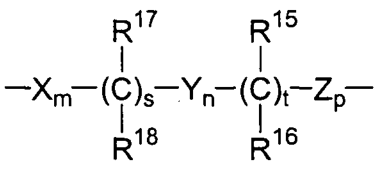 oder 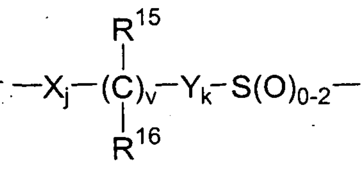

worin M -O-, -S-, -S(O)- oder $-S(O)_2-$ ist;

X, Y und Z jeweils unabhängig ausgewählt sind aus der Gruppe bestehend aus $-CH_2-$, $-CH(C_1-C_6)$Alkyl- und $-C(di-(C_1-C_6)$Alkyl)-;
$R^8$ ausgewählt ist aus der Gruppe bestehend aus H und Alkyl;
$R^{10}$ und $R^{11}$ jeweils unabhängig ausgewählt sind aus der Gruppe bestehend aus 1 bis 3 Substituenten, die jeweils unabhängig ausgewählt sind aus der Gruppe bestehend aus $(C_1-C_6)$Alkyl, $-OR^{19}$, $-O(CO)R^{19}$, $-O(CO)OR^{21}$,- $O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$-Alkylen)-$COOR^{19}$, $-CH=CH-COOR^{19}$, $-CF_3$, -CN, $-NO_2$ und Halogen;
$R^{15}$ und $R^{17}$ jeweils unabhängig ausgewählt sind aus der Gruppe bestehend aus $-OR^{19}$, $-OC(O)R^{19}$, $-OC(O)OR^{21}$, $-OC(O)NR^{19}R^{20}$;
$R^{16}$ und $R^{18}$ jeweils unabhängig ausgewählt sind aus der Gruppe bestehend aus H, $(C_1-C_6)$Alkyl und Aryl;
oder $R^{15}$ und $R^{16}$ zusammen =O sind oder $R^{17}$ und $R^{18}$ zusammen =O sind;
d 1, 2 oder 3 ist;
h 0, 1, 2, 3 oder 4 ist;
s 0 oder 1 ist;
t 0 oder 1 ist;
m, n und p jeweils unabhängig ausgewählt sind aus 0 bis 4;
mit der Mäßgabe, daß mindestens eines von s und t 1 ist und die Summe von m, n, p, s und t 1 bis 6 ist; mit der Maßgabe, daß, wenn p 0 und t 1 ist, die Summe von m, n und p 1 bis 5 ist; und mit der Maßgabe, daß, wenn p 0 und s 1 ist, die Summe von m, t und n 1 bis 5 ist;
v 0 oder 1 ist;
j und k jeweils unabhängig 1 bis 5 sind, mit der Maßgabe, daß die Summe von j, k und v 1 bis 5 ist;
Q eine Bindung oder $-(CH_2)_q-$ ist, worin q 1 bis 6 ist oder mit dem Ringkohlenstoff an Position 3 des Azetidinons die Spirogruppe

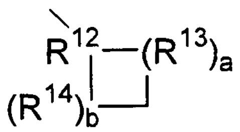

bildet;
worin $R^{12}$

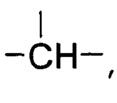, 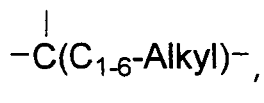, 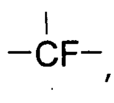, 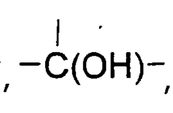, 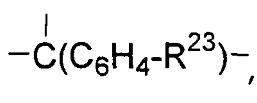,

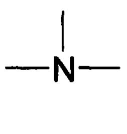 oder 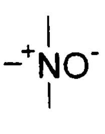

ist;
$R^{13}$ und $R^{14}$ jeweils unabhängig ausgewählt sind aus der Gruppe bestehend aus $-CH_2-$, $-CH(C_1-C_6$-Alkyl)-, -C

(di-($C_1$-$C_6$)Alkyl), -CH=CH- und -C($C_1$-$C_6$-Alkyl)=CH-; oder $R^{12}$ zusammen mit einem benachbarten $R^{13}$ oder $R^{12}$ zusammen mit einem benachbarten $R^{14}$ eine -CH=CH- oder eine -CH=C($C_1$-$C_6$-Alkyl)-Gruppe bilden;
a und b jeweils unabhängig 0, 1, 2 oder 3 sind, mit der Maßgabe, daß beide nicht 0 sind;
mit der Maßgabe, daß, wenn $R^{13}$ -CH=CH- oder -C($C_1$-$C_6$-Alkyl)=CH- ist, a 1 ist; mit der Maßgabe, daß, wenn $R^{14}$ -CH=CH- oder -C($C_1$-$C_6$-Alkyl)=CH- ist, b 1 ist;
mit der Maßgabe, daß, wenn a 2 oder 3 ist, die $R^{13}$ gleich oder verschieden sein können; und mit der Maßgabe, daß, wenn b 2 oder 3 ist, die $R^{14}$ gleich oder verschieden sein können;
und wenn Q eine Bindung ist und L

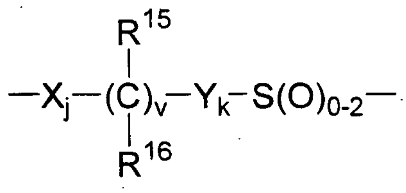

ist, dann kann $Ar^1$ auch Pyridyl, Isoxazolyl, Furanyl, Pyrrolyl, Thienyl, Imidazolyl, Pyrazolyl, Thiazolyl, Pyrazinyl, Pyrimidinyl oder Pyridazinyl sein;
$R^{19}$ und $R^{20}$ jeweils unabhängig ausgewählt sind aus der Gruppe bestehend aus H, ($C_1$-$C_6$)Alkyl, Aryl und Arylsubstituiertem ($C_1$-$C_6$)Alkyl;
$R^{21}$ ($C_1$-$C_6$)Alkyl, Aryl oder $R^{24}$-substituiertes Aryl ist;
$R^{22}$ H, ($C_1$-$C_6$)Alkyl, Aryl($C_1$-$C_6$)alkyl, -C(O)$R^{19}$ oder -COO$R^{19}$ ist;
$R^{23}$ und $R^{24}$ jeweils unabhängig ausgewählt sind aus der Gruppe bestehend aus 1 bis 3 Substituenten, die jeweils unabhängig ausgewählt sind aus der Gruppe bestehend aus H, ($C_1$-$C_6$)Alkyl, ($C_1$-$C_6$)Alkoxy, -COOH, $NO_2$, -N$R^{19}R^{20}$, -OH und Halogen; und
$R^{25}$ H, -OH oder ($C_1$-$C_6$)Alkoxy ist.

**11.** Verwendung gemäß Anspruch 1, worin der Blutspiegel von c-reaktivem Protein größer ist als ungefähr 1,0 mg/dl.

**12.** Verwendung gemäß Anspruch 1, worin der Blutspiegel von c-reaktivem Protein größer ist als ungefähr 3,4 mg/dl.

**13.** Verwendung gemäß Anspruch 1, ferner umfassend die Verabreichung von mindestens einem Peroxisomproliferator-aktivierten Rezeptoraktivator.

**14.** Verwendung gemäß Anspruch 13, worin der mindestens eine Peroxisomproliferator-aktivierte Rezeptoraktivator ein Fibrinsäurederivat ist.

**15.** Verwendung gemäß Anspruch 14, worin das Fibrinsäurederivat ausgewählt ist aus der Gruppe bestehend aus Fenofibrat, Clofibrat, Gemfibrozil, Ciprofibrat, Bezafibrat, Clinofibrat, Binifibrat, Lifibrol und Mischungen davon.

**16.** Verwendung gemäß Anspruch 1, worin der mindestens eine Sterolabsorptionsinhibitor in einer Menge im Bereich von ungefähr 0,1 bis ungefähr 1.000 mg des Sterolabsorptionsinhibitors pro Tag an einen Säuger verabreicht wird.

**17.** Verwendung gemäß Anspruch 1, worin der mindestens eine Cholesterol-Biosyntheseinhibitor mindestens einen HMG CoA-Reduktaseinhibitor umfaßt.

**18.** Verwendung gemäß Anspruch 17, worin der mindestens eine HMG CoA-Reduktaseinhibitor ausgewählt ist aus der Gruppe bestehend aus Lovastatin, Pravastatin, Fluvastatin, Simvastatin, Atorvastatin, Cerivastatin und Mischungen davon.

**19.** Verwendung gemäß Anspruch 1, ferner umfassend die Verabreichung von mindestens einem Gallensäure-Komplexbildner.

**20.** Verwendung gemäß Anspruch 1, ferner umfassend die Verabreichung von Nikotinsäure oder eines Derivats davon.

**21.** Verwendung gemäß Anspruch 1, ferner umfassend die Verabreichung von mindestens einem AcylCoA:Cholesterol

O-Acyltransferaseinhibitor.

**22.** Verwendung gemäß Anspruch 1, ferner umfassend die Verabreichung von Probucol oder einem Derivat davon.

**23.** Verwendung gemäß Anspruch 1, ferner umfassend die Verabreichung von mindestens einem Low-density Lipoprotein-Rezeptoraktivator.

**24.** Verwendung gemäß Anspruch 1, ferner umfassend die Verabreichung von mindestens einer Omega-3-Fettsäure.

**25.** Verwendung gemäß Anspruch 1, ferner umfassend die Verabreichung von mindestens einem natürlichen wasserlöslichen Ballaststoff.

**26.** Verwendung gemäß Anspruch 1, ferner umfassend die Verabreichung von mindestens einem der pflanzlichen Sterole, pflanzlichen Stanole oder Fettsäureestern pflanzlicher Stanole.

**27.** Verwendung gemäß Anspruch 1, ferner umfassend die Verabreichung von mindestens einem Antioxidans oder einem Vitamin.

**28.** Verwendung gemäß Anspruch 1, umfassend den Schritt der Verabreichung einer therapeutisch wirksamen Menge des Sterolabsorptionsinhibitors und eines pharmazeutisch akzeptablen Trägers.

**29.** Verwendung gemäß Anspruch 1, worin das Subjekt ein Mensch ist.

**30.** Verwendung einer Kombination von (1) mindestens einem Sterolabsorptionsinhibitor oder mindestens einem 5α-Stanolabsorptionsinhibitor und (2) mindestens einem Cholesterol-Biosyntheseinhibitor zur Herstellung eines Medikaments zur Reduktion der vaskulären c-reaktiven Protein-Spiegel in Säugern mit einem Blutspiegel von c-reaktivem Protein von mehr als 0,4 mg/dl.

**31.** Verwendung gemäß Anspruch 30, worin der Blutspiegel von c-reaktivem Protein auf ungefähr 3,4 mg/dl oder niedriger reduziert wird.

**32.** Verwendung gemäß Anspruch 30, worin der Blutspiegel von c-reaktivem Protein auf ungefähr 1,0 mg/dl oder niedriger reduziert wird.

**33.** Verwendung gemäß Anspruch 30, worin der Blutspiegel von c-reaktivem Protein auf ungefähr 0,4 mg/dl oder niedriger reduziert wird.

## Revendications

**1.** Emploi d'une combinaison constituée :

1) d'au moins un inhibiteur d'absorption des stérols ou d'au moins un inhibiteur d'absorption des 5α-stanols,
2) et d'au moins un inhibiteur de biosynthèse du cholestérol,

en vue de la fabrication d'un médicament destiné à traiter ou prévenir l'inflammation vasculaire chez un sujet présentant un taux sanguin de protéine C-réactive supérieur à 0,4 mg/dL.

**2.** Emploi conforme à la revendication 1, l'inhibiteur d'absorption des stérols au nombre d'au moins un étant représenté par la formule (I)

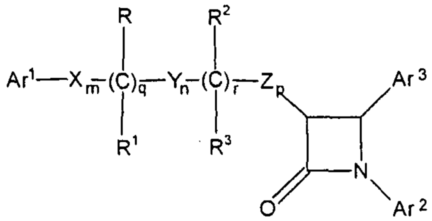

(I)

ou étant un sel ou solvat pharmacologiquement admissible d'un tel composé,
dans laquelle formule (I) les symboles ont les significations qui suivent :

- $Ar^1$ et $Ar^2$ représentent chacun indépendamment un groupe aryle ou un groupe aryle à substituant(s) $R^4$ ;
- $Ar^3$ représente un groupe aryle ou un groupe aryle à substituant(s) $R^5$ ;
- X, Y et Z représentent chacun indépendamment un groupe méthanediyle $-CH_2-$, -CH(alkyle inférieur)- ou -C (alkyle inférieur)$_2$- ;
- R et $R^2$ représentent chacun indépendamment un groupe symbolisé par $-OR^6$, $-O(CO)R^6$, $-O(CO)OR^9$ ou -O (CO)$NR^6R^7$ ;
- $R^1$ et $R^3$ représentent chacun indépendamment un atome d'hydrogène ou un groupe alkyle inférieur ou aryle ;
- l'indice q vaut 0 ou 1
- l'indice r vaut 0 ou 1 ;
- les indices m, n et p valent chacun indépendamment 0, 1 , 2, 3 ou 4 ; sous réserve que l'un au moins des indices q et r vaille 1 et que la somme des indices m, n, p, q et r soit comprise entre 1 et 6 ; et sous réserve que si p vaut 0 et r vaut 1, la somme des indices m, q et n soit comprise entre 1 et 5 ;
- $R^4$ représente 1 à 5 substituants choisis chacun indépendamment parmi les atomes d'halogène, les groupes alkyle inférieur, trifluorométhyle $-CF_3$, cyano -CN et nitro $-NO_2$ et les groupes symbolisés par $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6S(O)_2R^9$, $-COOR^6$, $-CONR^6R^7$, $-COR^6$, $-S(O)_2NR^6R^7$, $-S(O)_{0-2}R^9$, $-O(CH_2)_{1-10}-COOR^6$, $-O(CH_2)_{1-10}-CONR^6R^7$, -(alcanediyle inférieur)$COOR^6$ et $-CH=CH-COOR^6$ ;
- $R^5$ représente 1 à 5 substituants choisis chacun indépendamment parmi les groupes symbolisés par $-OR^6$, $-O(CO)R^6$, $-O(CO)OR^9$, $O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6S(O)_2R^9$, $-COOR^6$, $-CONR^6R^7$, $-COR^6$, $-S(O)_2NR^6R^7$, $-S(O)_{0-2}R^9$, $-O(CH_2)_{1-10}-COOR^6$, $-O(CH_2)_{1-10}-CONR^6R^7$, -(alcanediyle inférieur)$COOR^6$ et $-CH=CH-COOR^6$ ;
- $R^6$, $R^7$ et $R^8$ représentent chacun indépendamment un atome d'hydrogène ou un groupe alkyle inférieur, aryle ou alkyle inférieur à substituant aryle ;
- et $R^9$ représente un groupe alkyle inférieur, aryle ou alkyle inférieur à substituant aryle.

**3.** Emploi conforme à la revendication 2, l'inhibiteur d'absorption de stérol au nombre d'au moins un étant représenté par la formule (II) :

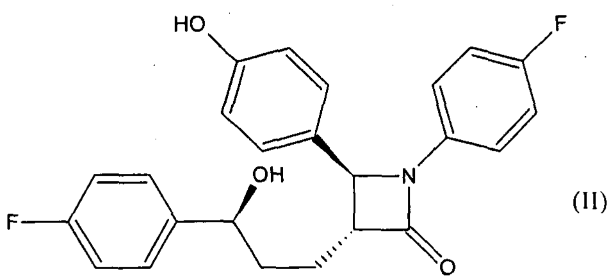

(II)

**4.** Emploi conforme à la revendication 1, l'inhibiteur d'absorption de stérol au nombre d'au moins un étant représenté par la formule (III)

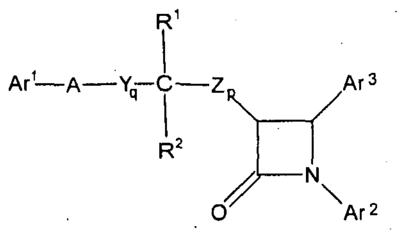

(III)

ou étant un sel ou solvat pharmacologiquement admissible d'un tel composé, dans laquelle formule (III) les symboles ont les significations qui suivent :

- $Ar^1$ représente un groupe aryle à substituant(s) $R^3$ ;
- $Ar^2$ représente un groupe aryle à substituant(s) $R^4$ ;
- $Ar^3$ représente un groupe aryle à substituant(s) $R^5$ ;
- Y et Z représentent chacun indépendamment un groupe méthanediyle $-CH_2-$, -CH-(alkyle inférieur)- ou -C (alkyle inférieur)$_2$- ;
- A représente une entité choisie parmi -O-, -S-, -S(O)- et $-S(O)_2-$ ;
- $R^1$ représente un groupe symbolisé par $-OR^6$, $-O(CO)R^6$, $-O(CO)OR^9$ ou $-O(CO)NR^6R^7$; $R^2$ représente un atome d'hydrogène ou un groupe alkyle inférieur ou aryle ; ou bien les entités représentées par $R^1$ et $R^2$ forment conjointement un substituant oxo =O ;
- l'indice q vaut 1, 2 ou 3 ;
- l'indice p vaut 0, 1, 2, 3 ou 4 ;
- $R^5$ représente 1 à 3 substituants choisis chacun indépendamment parmi les substituants o-halogéno et *m*-halogéno, les groupes *o*-(alkyle inférieur) et *m*-(alkyle inférieur) et les groupes symbolisés par $-OR^6$, $-O(CO)R^6$, $-O(CO)OR^9$, $O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6S(O)_2$-(alkyle inférieur), $-NR^6S(O)_2$-aryle, $-CONR^6R^7$, $-COR^6$, $-S(O)_2NR^6R^7$, $-S(O)_{0-2}$-alkyle, $-S(O)_{0-2}$-aryle, $-O(CH_2)_{1-10}-COOR^6$, $-O(CH_2)_{1-10}-CONR^6R^7$, -(alcanediyle inférieur)$COOR^6$ et $-CH=CH-COOR^6$ ;
- $R^3$ et $R^4$ représentent chacun indépendamment 1 à 3 substituants choisis chacun indépendamment parmi l'atome d'hydrogène, les substituants $R^5$, les substituants *p*-halogéno et les groupes p-(alkyle inférieur), aryle, nitro $-NO_2$ et trifluorométhyle $-CF_3$ ;
- $R^6$, $R^7$ et $R^8$ représentent chacun indépendamment un atome d'hydrogène ou un groupe alkyle inférieur, aryle ou alkyle inférieur à substituant aryle ;
- et $R^9$ représente un groupe alkyle inférieur, aryle ou alkyle inférieur à substituant aryle.

**5.** Emploi conforme à la revendication 1, l'inhibiteur d'absorption de stérol au nombre d'au moins un étant représenté par la formule (IV)

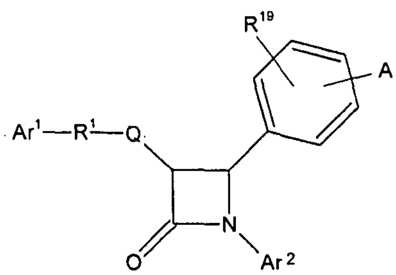

(IV)

ou étant un sel ou solvat pharmacologiquement admissible d'un tel composé, dans laquelle formule (IV) les symboles ont les significations qui suivent :

- A représente une entité choisie parmi les groupes hétérocycloalkyle à substituant(s) $R^2$, hétéroaryle à substituant(s) $R^2$, hétérocycloalkyle benzocondensé à substituant(s) $R^2$ et hétéroaryle benzocondensé à substituant (s) $R^2$ ;
- $Ar^1$ représente un groupe aryle ou aryle à substituant(s) $R^3$ ;
- $Ar^2$ représente un groupe aryle ou aryle à substituant(s) $R^4$ ;
- Q représente une liaison ou une entité qui forme, conjointement avec l'atome de carbone en position 3 du

cycle azétidinone, le groupe spiro de formule

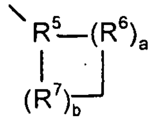

;

- et $R^1$ représente un groupe choisi parmi les suivants :

  - $-(CH_2)_q-$, où l'indice q vaut de 2 à 6, sous réserve que lorsque Q représente un cycle spiro, l'indice q puisse aussi valoir 0 ou 1 ;
  - $-(CH_2)_e-G-(CH_2)_r-$, où G représente -O-, -C(O)-, $-S(O)_{0-2}-$, un groupe phénylène ou symbolisé par $-NR^8-$, les indices e et r valent chacun de 0 à 5, sous réserve que la somme des deux soit comprise entre 1 et 6 ;
  - alcènediyle en $C_{2-6}$ ;
  - et $-(CH_2)_f-V-(CH_2)_g-$, où V représente un groupe cycloalcanediyle en $C_{3-6}$, les indices f et g valent, respectivement, de 1 à 5 et de 0 à 5, sous réserve que la somme des deux soit comprise entre 1 et 6 ;

- $R^5$ représente un groupe choisi parmi les suivants :

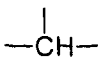 , 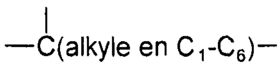 , 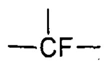 , 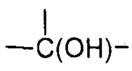 , 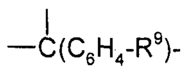 , 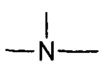 et 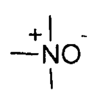

- $R^6$ et $R^7$ représentent chacun indépendamment un groupe méthanediyle $-CH_2-$, -CH(alkyle en $C_{1-6}$)-, -C(alkyle en $C_{1-6})_2-$, -CH=CH- ou -C(alkyle en $C_{1-6}$)=CH- ; ou bien soit l'entité représentée par $R^5$ et une entité adjacente représentée par $R^6$, soit l'entité représentée par $R^5$ et une entité adjacente représentée par $R^7$ forment ensemble un groupe -CH=CH- ou -CH=C(alkyle en $C_{1-6}$)- ;
- les indices a et b valent chacun indépendamment 0, 1, 2 ou 3 ; sous réserve que l'un et l'autre ne soient pas nuls ; sous réserve que lorsque $R^6$ représente -CH=CH- ou -C(alkyle en $C_{1-6}$)=CH-, a vaille 1 ; sous réserve que lorsque $R^7$ représente -CH=CH- ou -C(alkyle en $C_{1-6}$)=CH-, b vaille 1 ; sous réserve que lorsque a vaut 2 ou 3, les entités représentées par $R^6$ puissent être identiques ou différentes ; et sous réserve que lorsque b vaut 2 ou 3, les entités représentées par $R^7$ puissent être identiques ou différentes ;
- et lorsque Q représente une liaison, $R^1$ peut également représenter un groupe choisi parmi les groupes de formules suivantes :

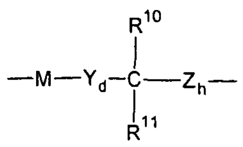 , 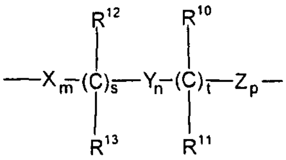 et 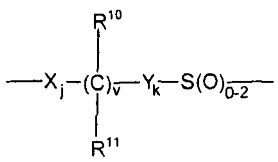 ;

dans lesquelles

  - M représente une entité choisie parmi -O-, -S-, -S(O)- et $-S(O)_2-$ ;
  - X, Y et Z représentent chacun indépendamment un groupe méthanediyle $-CH_2-$, -CH(alkyle en $C_{1-6}$)- ou -C(alkyle en $C_{1-6})_2-$ ;
  - $R^{10}$ et $R^{12}$ représentent chacun indépendamment un groupe symbolisé par $-OR^{14}$, $-O(CO)R^{14}$, $-O(CO)OR^{16}$ ou $-O(CO)NR^{14}R^{15}$ ;
  - $R^{11}$ et $R^{13}$ représentent chacun indépendamment un atome d'hydrogène ou un groupe alkyle en $C_{1-6}$ ou aryle ; ou bien soit les entités représentées par $R^{10}$ et $R^{11}$, soit les entités représentées par $R^{12}$ et $R^{13}$ forment conjointement un substituant oxo =O ;
  - l'indice d vaut 1, 2 ou 3 ;
  - l'indice h vaut 0, 1, 2, 3 ou 4 ;

- l'indice s vaut 0 ou 1 ; l'indice t vaut 0 ou 1 ; les indices m, n et p valent chacun indépendamment 0, 1, 2, 3 ou 4 ; sous réserve que l'un au moins des indices s et t vaille 1 et que la somme des indices m, n, p, s et t soit comprise entre 1 et 6 ; sous réserve que si p vaut 0 et t vaut 1, la somme des indices m, s et n soit comprise entre 1 et 5 ; et sous réserve que si p vaut 0 et s vaut 1, la somme des indices m, t et n soit comprise entre 1 et 5 ;
- l'indice v vaut 0 ou 1 ;
- les indices j et k valent chacun indépendamment 1, 2, 3, 4 ou 5, sous réserve que la somme des indices j, k et v soit comprise entre 1 et 5 ;

- $R^2$ représente 1 à 3 substituants portés par les atomes de carbone cycliques choisis parmi les atomes d'hydrogène et d'halogène, les groupes hydroxyle -OH, nitro $-NO_2$, alkyle en $C_{1-10}$, alcényle en $C_{2-10}$, alcynyle en $C_{2-10}$, cycloalkyle en $C_{3-6}$, cycloalcényle en $C_{3-6}$, alcoxy en $C_{1-6}$, aryle à substituant(s) $R^{17}$, benzyle à substituant (s) $R^{17}$, benzyloxy à substituant(s) $R^{17}$ et aryloxy à substituant(s) $R^{17}$ et les groupes symbolisés par $-NR^{14}R^{15}$, $-NR^{14}R^{15}$(alcanediyle en $C_{1-6}$)-, $-NR^{14}R^{15}C(O)$(alcanediyle en $C_{1-6}$)-, $-NHC(O)R^{16}$, $-OC(O)R^{16}$, $-C(O)R^{14}$, hydroxy(alkyle en $C_{1-6}$), (alcoxy en $C_{1-6}$)-alkyle en $C_{1-6}$, $-S(O)_{0-2}R^{16}$, $-SO_2NR^{14}R^{15}$ et -(alcanediyle en $C_{1-6}$) $COOR^{14}$ ; lorsque $R^2$ représente un substituant porté par un cycle hétérocycloalkyle, $R^2$ a a la signification indiquée ici ou représente un substituant oxo =O ou un groupe de formule

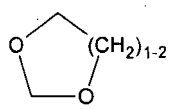 ;

et lorsque $R^2$ représente un substituant porté par un atome d'azote cyclique pouvant être le siège d'une substitution, il s'agit d'un atome d'hydrogène, d'un groupe hydroxyle -OH, alkyle en $C_{1-6}$, aryle, alcoxy en $C_{1-6}$, aryloxy, (alkyle en $C_{1-6}$)-carbonyle, arylcarbonyle ou d'un groupe de formule $-(CH_2)_{1-6}CONR^{18}R^{18}$,

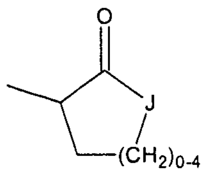 ou 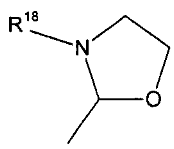 ,

dans laquelle J représente -O-, -NH-, $-NR^{18}$- ou $-CH_2$- ;
- $R^3$ et $R^4$ représentent chacun indépendamment 1 à 3 substituants choisis chacun indépendamment parmi les atomes d'halogène, les groupes trifluorométhyle $-CF_3$, cyano -CN, nitro $-NO_2$ et alkyle en $C_{1-6}$ et les groupes symbolisés par $-OR^{14}$, $-O(CO)R^{14}$, $-O(CO)OR^{16}$, $-O(CH_2)_{1-5}OR^{14}$, $-O(CO)NR^{14}R^{15}$-, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $-S(O)_{0-2}R^{16}$, $O(CH_2)_{1-10}-COOR^{14}$, $-O(CH_2)_{1-10}CONR^{14}R^{15}$, -(alcanediyle en $C_{1-6}$)-$COOR^{14}$ et $-CH=CH-COOR^{14}$;
- $R^8$ représente un atome d'hydrogène ou un groupe alkyle en $C_{1-6}$, aryl-(alkyle en $C_{1-6}$), $-C(O)R^{14}$ ou $-COOR^{14}$ ;
- $R^9$ et $R^{17}$ représentent chacun indépendamment 1 à 3 substituants choisis chacun indépendamment parmi les atomes d'hydrogène et d'halogène, les groupes alkyle en $C_{1-6}$, alcoxy en $C_{1-6}$, carboxyle -COOH, nitro $-NO_2$ et hydroxyle -OH et les groupes symbolisés par $-NR^{14}R^{15}$;
- $R^{14}$ et $R^{15}$ représentent chacun indépendamment un atome d'hydrogène ou un groupe alkyle en $C_{1-6}$, aryle ou alkyle en $C_{1-6}$ à substituant aryle ;
- $R^{16}$ représente un groupe alkyle en $C_{1-6}$, aryle ou aryle à substituant(s) $R^{17}$ ;
- $R^{18}$ représente un atome d'hydrogène ou un groupe alkyle en $C_{1-6}$ ;
- et $R^{19}$ représente un atome d'hydrogène ou un groupe hydroxyle -OH ou alcoxy en $C_{1-6}$.

**6.** Emploi conforme à la revendication 1, l'inhibiteur d'absorption des stérols au nombre d'au moins un étant représenté par la formule (V)

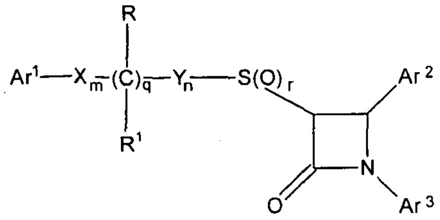

(V)

ou étant un sel ou solvat pharmacologiquement admissible d'un tel composé,
dans laquelle formule (V) les symboles ont les significations qui suivent :

- $Ar^1$ représente un groupe aryle, aryle à substituant(s) $R^{10}$ ou hétéroaryle ;
- $Ar^2$ représente un groupe aryle ou aryle à substituant(s) $R^4$ ;
- $Ar^3$ représente un groupe aryle ou aryle à substituant(s) $R^5$ ;
- X et Y représentent chacun indépendamment un groupe méthanediyle $-CH_2-$, -CH(alkyle inférieur)- ou -C (alkyle inférieur)$_2$- ;
- R représente un groupe symbolisé par $-OR^6$, $-O(CO)R^6$, $-O(CO)OR^9$ ou $-O(CO)NR^6R^7$ ;
- $R^1$ représente un atome d'hydrogène ou un groupe alkyle inférieur ou aryle ; ou bien les entités représentées par R et $R^1$ forment conjointement un substituant oxo =O ;
- l'indice q vaut 0 ou 1 ;
- l'indice r vaut 0, 1 ou 2 ;
- les indices m et n valent chacun indépendamment 0, 1, 2, 3, 4 ou 5 ; sous réserve que la somme des indices m, n, et q soit comprise entre 1 et 5 ;
- $R^4$ représente 1 à 5 substituants choisis chacun indépendamment parmi les groupes alkyle inférieur et les groupes symbolisés par $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6S(O)_2R^9$, $-COOR^6$, $-CONR^6R^7$, $-COR^6$, $-S(O)_2NR^6R^7$, $-S(O)_{0-2}R^9$, $-O(CH_2)_{1-10}-COOR^6$, $-O(CH_2)_{1-10}-CONR^6R^7$, -(alcanediyle inférieur)$COOR^6$ et $-CH=CH-COOR^6$ ;
- $R^5$ représente 1 à 5 substituants choisis chacun indépendamment parmi les atomes d'halogène, les groupes trifluorométhyle $-CF_3$, cyano -CN et nitro $-NO_2$ et les groupes symbolisés par $-OR^6$, $-O(CO)R^6$, $-O(CO)OR^9$, $O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6S(O)_2R^9$, $-COOR^6$, $-CONR^6R^7$, $-COR^6$, $-S(O)_2NR^6R^7$,- $S(O)_{0-2}R^9$, $-O(CH_2)_{1-10}-COOR^6$, $-O(CH_2)_{1-10}-CONR^6R^7$, (alcanediyle inférieur)$COOR^6$ et $-CH=CH-COOR^6$ ;
- $R^6$, $R^7$ et $R^8$ représentent chacun indépendamment un atome d'hydrogène ou un groupe alkyle inférieur, aryle ou alkyle inférieur à substituant aryle ;
- $R^9$ représente un groupe alkyle inférieur, aryle ou alkyle inférieur à substituant aryle ;
- et $R^{10}$ représente 1 à 5 substituants choisis chacun indépendamment parmi les atomes d'halogène, les groupes trifluorométhyle $-CF_3$, cyano -CN et nitro $-NO_2$, les groupes alkyle inférieur et les groupes symbolisés par $-OR^6$, $-O(CO)R^6$, $-O(CO)OR^9$, $O(CH_2)_{1-5}OR^6$, $-O(CO)NR^6R^7$, $-NR^6R^7$, $NR^6(CO)R^7$, $-NR^6(CO)OR^9$, $-NR^6(CO)NR^7R^8$, $-NR^6S(O)_2R^9$, $-COOR^6$, $-CONR^6R^7$, $-COR^6$, $-S(O)_2NR^6R^7$, $-S(O)_{0-2}R^9$, $-O(CH_2)_{1-10}-COOR^6$ et $-O(CH_2)_{1-10}-CONR^6R^7$.

**7.** Emploi conforme à la revendication 1, l'inhibiteur d'absorption des stérols au nombre d'au moins un étant représenté par la formule (VI)

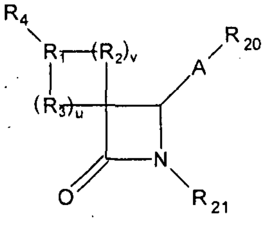

(VI)

ou étant un sel ou solvat pharmacologiquement admissible d'un tel composé,
dans laquelle formule (VI) les symboles ont les significations qui suivent :

- $R_1$ représente un groupe choisi parmi les suivants :

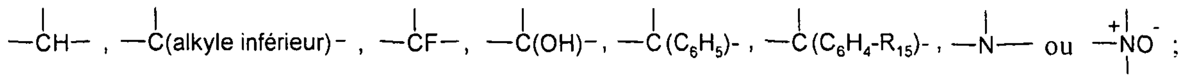

- $R_2$ et $R_3$ représentent chacun indépendamment un groupe méthanediyle $-CH_2-$, -CH(alkyle inférieur)- ou -C (alkyle inférieur)$_2$-, -CH=CH- ou -C(alkyle inférieur)=CH- ; ou bien soit l'entité représentée par $R_1$ et une entité adjacente représentée par $R_2$, soit l'entité représentée par $R_1$ et une entité adjacente représentée par $R_3$ forment ensemble un groupe -CH=CH- ou -CH=C(alkyle inférieur)- ;
- les indices u et v valent chacun indépendamment 0, 1, 2 ou 3 ; sous réserve que l'un et l'autre ne soient pas nuls ; sous réserve que lorsque $R_2$ représente- CH=CH- ou -C(alkyle inférieur)=CH-, v vaille 1 ; sous réserve que lorsque $R_3$ représente -CH=CH- ou -C(alkyle inférieur)=CH-, u vaille 1 ; sous réserve que lorsque v vaut 2 ou 3, les entités représentées par $R_2$ puissent être identiques ou différentes ; et sous réserve que lorsque u vaut 2 ou 3, les entités représentées par $R_3$ puissent être identiques ou différentes ;
- $R_4$ représente un groupe choisi parmi les suivants :

  - B-$(CH_2)_m$C(O)-, où l'indice m vaut de 0 à 5 ;
  - B-$(CH_2)_q$-, où l'indice q vaut de 0 à 6 ;
  - B-$(CH_2)_e$-Z-$(CH_2)_r$-, où Z représente -O-, -C(O)-, -S(O)$_{0-2}$-, un groupe phénylène ou symbolisé par -$NR_8$-, les indices e et r valent chacun de 0 à 5, sous réserve que la somme des deux soit comprise entre 0 et 6 ;
  - B-(alcènediyle en $C_{2-6}$)- ;
  - B-(alcadiènediyle en $C_{4-6}$)-;
  - B-$(CH_2)_t$-Z-(alcènediyle en $C_{2-6}$)-, où Z a la signification indiquée ci-dessus, et l'indice t vaut de 0 à 3, sous réserve que la somme de t et du nombre d'atomes de carbone dans la chaîne alcènediyle soit comprise entre 2 et 6 ;
  - B-$(CH_2)_f$-V-$(CH_2)_g$-, où V représente un groupe cycloalcanediyle en $C_{3-6}$. l'indice f vaut de 1 à 5 et l'indice g vaut de 0 à 5, sous réserve que la somme des deux soit comprise entre 1 et 6 ;
  - B-$(CH_2)_t$-V-(alcènediyle en $C_{2-6}$)- ou B-(alcènediyle en $C_{2-6}$)-V-$(CH_2)_1$-, où V et t ont les significations indiquées ci-dessus, sous réserve que la somme de t et du nombre d'atomes de carbone dans la chaîne alcènediyle soit comprise entre 2 et 6 ;
  - B-$(CH_2)_a$-Z-$(CH_2)_b$-V-$(CH_2)_d$-, où Z et V ont les significations indiquées ci-dessus et les indices a, b et d valent chacun indépendamment de 0 à 6, sous réserve que la somme de a, b et d soit comprise entre 0 et 6 ;
  - ou T-$(CH_2)_s$-, où T représente un groupe cycloalkyle en $C_{3-6}$ et s vaut de 0 à 6 ;

- ou bien les entités représentées par $R_1$ et $R_4$ forment conjointement un groupe symbolisé par B-CH=C< ;
- B représente un groupe choisi dans l'ensemble que constituent les groupes indanyle, indényle, naphtyle, tétrahydronaphtyle, hétéroaryle ou hétéroaryle à substituant(s) W, ledit groupe hétéroaryle étant choisi parmi les groupes pyrrolyle, pyridinyle, pyrimidinyle, pyrazinyle, triazinyle, imidazolyle, thiazolyle, pyrazolyle, thiényle, oxazolyle et furanyle et, dans le cas de groupes hétéroaryle azotés, parmi les N-oxydes de ces derniers, et les groupes de formule

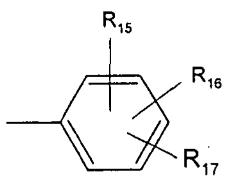

- W représente 1 à 3 substituants choisis chacun indépendamment dans l'ensemble formé par les atomes d'halogène, les groupes hydroxyle -OH, cyano -CN, nitro $-NO_2$, azido $-N_3$, trifluorométhyle $-CF_3$, $-OCF_3$, les groupes alkyle inférieur, hydroxyalkyle inférieur, alcoxy inférieur, alcoxyalkyle, alcoxy-alcoxy, alcoxycarbonylalcoxy, (alcoxy inférieur)-imino-(alkyle inférieur), alcanedioyle inférieur, -(alcanedioyle inférieur)-(alkyle inférieur), allyloxy, benzyle, benzyloxy, phénoxy et dioxolanyle et les groupes symbolisés par $R_7$-benzyle, $R_7$-benzyloxy, $R_7$-phénoxy, -N($R_8$)($R_9$), N($R_8$)($R_9$)-(alcanediyle inférieur)-, N($R_8$)($R_9$)-(alcanediyle inférieur)-oxy-, -NHC (O)O$R_{10}$, -NHC(O)$R_{10}$, $R_{11}O_2SNH$-, $(R_{11}O_2S)_2N$-, $-S(O)_2NH_2$, -S(O)$_{0-2}R_8$, tertiobutyldiméthyl-silyloxyméthyle,

$-C(O)R_{12}$, $-COOR_{19}$, $-CON(R_8)(R_9)$, - $CH{=}CHC(O)R_{12}$, -(alcanediyle inférieur)-$C(O)R_{12}$, $R_{10}C(O)$-(alcanediyle inférieur)-oxy-, $N(R_8)(R_9)C(O)$(alcanediyle inférieur)-oxy- et

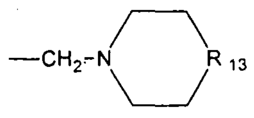

, dans le cas des substituants portés par les atomes de carbone du cycle hétéroaryle, tandis que les substituants portés par les éventuels atomes d'azote du cycle hétéroaryle sont eux choisis dans l'ensemble formé par les groupes hydroxyle -OH, alkyle inférieur, alcoxy inférieur et les groupes symbolisés par $-C(O)OR_{10}$, $-C(O)R_{10}$, $N(R_8)(R_9)$-(alcanediyle inférieur)-, $N(R_8)(R_9)$-(alcanediyle inférieur)-oxy-, $-S(O)_2NH_2$ et 2-(triméthylsilyl)-éthoxyméthyle ;

- $R_7$ représente 1 à 3 substituants choisis chacun indépendamment parmi les atomes d'halogène, les groupes hydroxyle -OH, carboxyle -COOH, nitro $-NO_2$, alkyle inférieur et alcoxy inférieur et les groupes symbolisés par $-N(R_8)(R_9)$ ;
- $R_8$ et $R_9$ représentent indépendamment un atome d'hydrogène ou un groupe alkyle inférieur ;
- $R_{10}$ représente un groupe choisi parmi les groupes alkyle inférieur, phényle et benzyle et les groupes symbolisés par $R_7$-phényle et $R_7$-benzyle ;
- $R_{11}$ représente un groupe choisi parmi les groupes hydroxyle OH, alkyle inférieur, phényle et benzyle et les groupes symbolisés par $R_7$-phényle et $R_7$-benzyle ;
- $R_{12}$ représente une entité choisie parmi l'atome d'hydrogène, les groupes hydroxyle -OH, alkyle inférieur, phényle, alcoxy, phénoxy et benzyloxy et les groupes symbolisés par

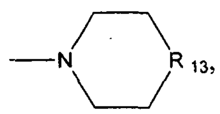

- $N(R_8)(R_9)$ et $R_7$-phényle ;
- $R_{13}$ représente une entité choisie parmi -O-, $-CH_2-$, -NH-, -N(alkyle inférieur)- ou un groupe symbolisé par $-NC(O)R_{19}$ ;
- $R_{15}$, $R_{16}$ et $R_{17}$ représentent chacun indépendamment une entité choisie indépendamment parmi l'atome d'hydrogène et les groupes que W peut représenter ; ou bien $R_{15}$ représente un atome d'hydrogène tandis que les entités représentées par $R_{16}$ et $R_{17}$ forment conjointement avec les atomes de carbone adjacents auxquels elles sont attachées, un cycle dioxolanyle ;
- $R_{19}$ représente une entité choisie parmi l'atome d'hydrogène, les groupes alkyle inférieur, phényle ou phényle-(alkyle inférieur) ;
- et $R_{20}$ et $R_{21}$ représentent chacun indépendamment un groupe choisi parmi les groupes phényle, phényle à substituant(s) W, naphtyle, naphtyle à substituant(s) W, indanyle, indényle, tétrahydronaphtyle, benzodioxolyle, hétéroaryle, hétéroaryle à substituant(s) W, hétéroaryle benzocondensé, hétéroaryle benzocondensé à substituant(s) W et cyclopropyle, le groupe hétéroaryle étant l'un de ceux énumérés ci-dessus.

**8.** Emploi conforme à la revendication 1, l'inhibiteur d'absorption des stérols au nombre d'au moins un étant représenté par la formule (VIIA) ou (VIIB)

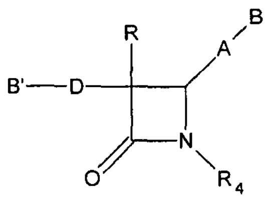

(VIIA)

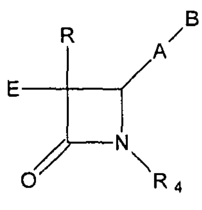

(VIIB)

ou étant un sel ou solvat pharmacologiquement admissible d'un tel composé,

dans lesquelles formules (VIIA) et (VIIB) les symboles ont les significations qui suivent :

- A représente un groupe -CH=CH-, -C≡C- ou $-(CH_2)_p-$, l'indice p valant 0, 1 ou 2 ;
- B représente un groupe de formule

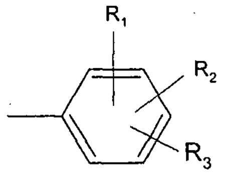

;

- B' représente un groupe de formule

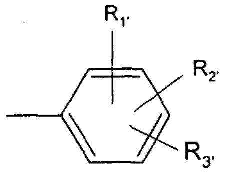

;

- D représente un groupe de formule $-(CH_2)_mC(O)-$ ou $-(CH_2)_q-$, l'indice m valant 1, 2, 3 ou 4 et l'indice q valant 2, 3 ou 4 ;
- E représente un groupe alkyle en $C_{10-20}$ ou -C(O)-(alkyle en $C_{9-19}$) dont la chaîne est linéaire ou ramifiée, saturée ou porteuse d'une ou de plusieurs doubles liaisons ;
- R représente un atome d'hydrogène, un groupe alkyle en $C_{1-15}$ dont la chaîne est linéaire ou ramifiée, saturée ou porteuse d'une ou de plusieurs doubles liaisons, ou un groupe de formule $B-(CH_2)_r-$, l'indice r valant 0, 1, 2 ou 3 ;
- $R_1$, $R_2$, $R_3$, $R_{1'}$, $R_{2'}$ et $R_{3'}$ représentent chacun indépendamment une entité choisie indépendamment parmi les atomes d'hydrogène et d'halogène, les groupes nitro $-NO_2$, amino $-NH_2$, hydroxyle -OH, $-S(O)_2NH_2$, carboxyle -COOH, alkyle inférieur, alcoxy inférieur, (alkyle inférieur)amino et (alkyle inférieur)$_2$-amino et les groupes symbolisés par $-NHC(O)OR_5$ et $R_6O_2SNH-$ ;
- $R_4$ représente un groupe de formule

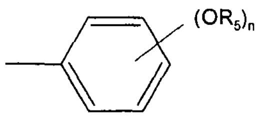

,

l'indice n valant 0, 1, 2 ou 3 ;
- $R_5$ représente un groupe alkyle inférieur ;
- $R_6$ représente un groupe hydroxyle -OH, alkyle inférieur, phényle, benzyle ou phényle porteur de substituant (s), lesdits substituants étant 1 à 3 entités choisies chacune indépendamment parmi les atomes d'halogène, les groupes nitro $-NO_2$, amino $-NH_2$, -OH, carboxyle -COOH, alkyle inférieur, alcoxy inférieur, (alkyle inférieur) amino et (alkyle inférieur)$_2$-amino.

**9.** Emploi conforme à la revendication 1, l'inhibiteur d'absorption des stérols au nombre d'au moins un étant représenté par la formule (VIII):

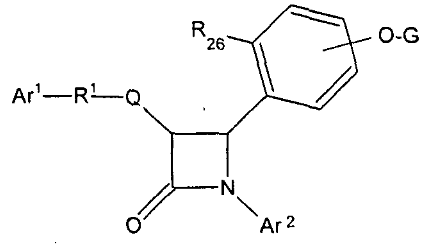

(VIII)

ou étant un sel ou solvat pharmacologiquement admissible d'un tel composé, dans laquelle formule (VIII) les symboles ont les significations qui suivent :

- $R^{26}$ représente un atome d'hydrogène ou un groupe de formule $-OG^1$ ;
- G et $G^1$ représentent chacun indépendamment une entité choisie parmi l'atome d'hydrogène et les groupes de formules

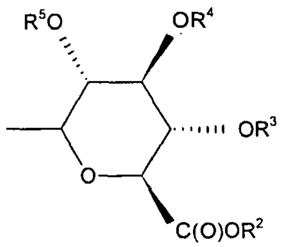
,
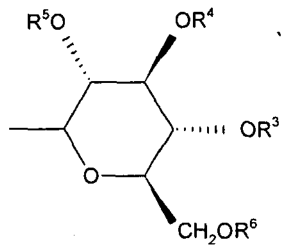
,
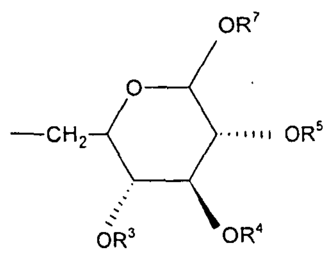

et

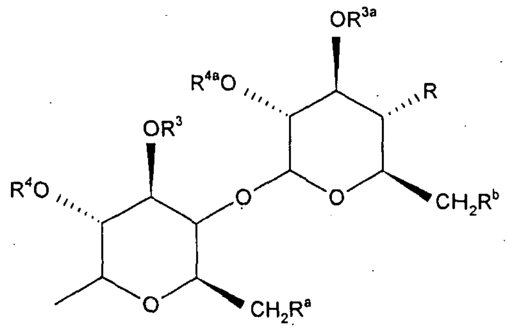
,

sous réserve que lorsque $R^{26}$ représente un atome d'hydrogène ou un groupe hydroxyle -OH, G ne représente pas un atome d'hydrogène ;
- R, $R^a$ et $R^b$ représentent chacun indépendamment une entité choisie parmi les atomes d'hydrogène et d'halogène, les groupes hydroxyle -OH, amino $-NH_2$, azido $-N_3$ et (alcoxy en $C_{1-6}$)-(alcoxy en $C_{1-6}$) et les groupes de formule $-W-R^{30}$ ;
- chaque symbole W est choisi indépendamment parmi les groupes symbolisés par -NH-C(O)-, -O-C(O)-, -O-C(O)-N($R^{31}$)-, -NH-C(O)-N($R^{31}$)- et -O-C(S)-N($R^{31}$)- ;
- $R^2$ et $R^6$ représentent chacun indépendamment un atome d'hydrogène ou un groupe alkyle en $C_{1-6}$, aryle ou aryle-(alkyle en $C_{1-6}$) ;
- $R^3$, $R^4$, $R^5$, $R^7$ $R^{3a}$ et $R^{4a}$ représentent chacun indépendamment un atome d'hydrogène ou un groupe alkyle en $C_{1-6}$, aryle-(alkyle en $C_{1-6}$), -C(O)-(alkyle en $C_{1-6}$) ou -C(O)-aryle ;
- $R^{30}$ représente un groupe choisi parmi les groupes T à substituant(s) $R^{32}$, (T à substituant(s) $R^{32}$)-(alkyle en $C_{1-6}$), alcényle en $C_{2-4}$ à substituant(s) $R^{32}$, alkyle en $C_{1-6}$ à substituant(s) $R^{32}$, cycloalkyle en $C_{3-7}$ à substituant (s) $R^{32}$ et (cycloalkyle en $C_{3-7}$ à substituant(s) $R^{32}$)-(alkyle en $C_{1-6}$) ;
- $R^{31}$ représente un atome d'hydrogène ou un groupe alkyle en $C_{1-4}$ ;

- T représente un groupe choisi dans l'ensemble que constituent les groupes phényle, furyle, thiényle, pyrrolyle, oxazolyle, isoxazolyle, thiazolyle, isothiazolyle, benzothiazolyle, thiadiazolyle, pyrazolyle, imidazolyle et pyridyle ;
- chaque symbole $R^{32}$ représente indépendamment 1 à 3 substituants choisis chacun indépendamment parmi les atomes d'halogène, le substituant oxo, les groupes hydroxyle -OH, trifluorométhyle $-CF_3$, nitro $-NO_2$, alkyle en $C_{1-4}$, alcoxy en $C_{1-4}$, phénoxy, méthylènedioxy, alkylsulfanyle en $C_{1-4}$, alkylsulfinyle en $C_{1-4}$, alkylsulfonyle en $C_{1-4}$, $-N(CH_3)_2$, -C(O)-NH(alkyle en $C_{1-4}$), -C(O)-N(alkyle en $C_{1-4})_2$, -C(O)-(alkyle en $C_{1-4}$), -C(O)-(alcoxy en $C_{1-4}$) et pyrrolidinylcarbonyle ; ou bien $R^{32}$ représente une liaison covalente et l'entité représentée par $R^{31}$, l'atome d'azote auquel elle est liée, ainsi que l'entité représentée par $R^{32}$ forment ensemble un groupe pyrrolidinyle, pipéridinyle, N-méthyl-pipérazinyle, indolinyle ou morpholinyle ou encore un groupe pyrrolidinyle à substituant (alcoxy en $C_{1-4}$)-carbonyle, pipéridinyle à substituant (alcoxy en $C_{1-4}$)-carbonyle, N-méthyl-pipérazinyle à substituant (alcoxy en $C_{1-4}$)-carbonyle, indolinyle à substituant (alcoxy en $C_{1-4}$)-carbonyle ou morpholinyle à substituant (alcoxy en $C_{1-4}$)-carbonyle ;
- $Ar^1$ représente un groupe aryle à substituant(s) $R^{10}$;
- $Ar^2$ représente un groupe aryle à substituant(s) $R^{11}$;
- Q représente une liaison ou une entité qui forme, conjointement avec l'atome de carbone en position 3 du cycle azétidinone, le groupe spiro de formule

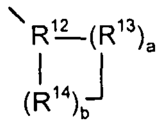

;

- $R^1$ représente un groupe choisi parmi les suivants :

  - $-(CH_2)_q-$, où l'indice q vaut de 2 à 6, sous réserve que lorsque Q forme un cycle spiro, l'indice q puisse aussi valoir 0 ou 1 ;
  - $-(CH_2)_e-E-(CH_2)_r-$, où E représente -O-, -C(O)-, $-S(O)_{0-2}-$, un groupe phénylène ou symbolisé par $-NR^{22}-$ et les indices e et r valent chacun de 0 à 5, sous réserve que la somme des deux soit comprise entre 1 et 6 ;
  - alcènediyle en $C_{2-6}$ ;
  - et $-(CH_2)_f-V-(CH_2)_g-$, où V représente un groupe cycloalcanediyle en $C_{3-6}$ et les indices f et g valent, respectivement, de 1 à 5 et de 0 à 5, sous réserve que la somme des deux soit comprise entre 1 et 6 ;

- $R^{12}$ représente un groupe choisi parmi les suivants :

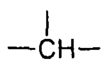 , 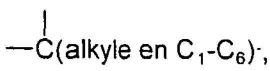 , 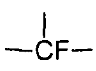 , 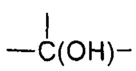 , 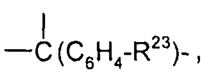 , 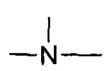 et 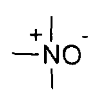

- $R^{13}$ et $R^{14}$ représentent chacun indépendamment un groupe méthanediyle $-CH_2-$, -CH(alkyle en $C_{1-6}$)-, -C(alkyle en $C_{1-6})_2$-, -CH=CH- ou -C(alkyle en $C_{1-6}$)=CH- ; ou bien soit l'entité représentée par $R^{12}$ et une entité adjacente représentée par $R^{13}$, soit l'entité représentée par $R^{12}$ et une entité adjacente représentée par $R^{14}$ forment ensemble un groupe -CH=CH- ou -CH=C(alkyle en $C_{1-6}$)- ;
- les indices a et b valent chacun indépendamment 0, 1, 2 ou 3 ; sous réserve que l'un et l'autre ne soient pas nuls ; sous réserve que lorsque $R^{13}$ représente -CH=CH- ou -C(alkyle en $C_{1-6}$)=CH-, a vaille 1 ; sous réserve que lorsque $R^{14}$ représente -CH=CH- ou -C(alkyle en $C_{1-6}$)=CH-, b vaille 1 ; sous réserve que lorsque a vaut 2 ou 3, les entités représentées par $R^{13}$ puissent être identiques ou différentes ; et sous réserve que lorsque b vaut 2 ou 3, les entités représentées par $R^{14}$ puissent être identiques ou différentes ;
- et lorsque Q représente une liaison, $R^1$ peut également représenter un groupe choisi parmi les suivants :

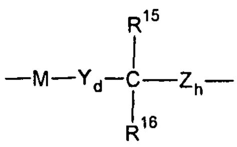 , 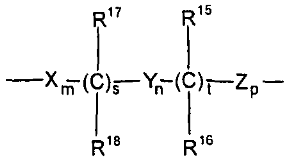 et 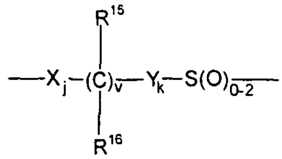 ;

- M représente une entité choisie parmi -O-, -S-, -S(O)- et $-S(O)_2-$ ;
- X, Y et Z représentent chacun indépendamment un groupe méthanediyle $-CH_2-$, -CH(alkyle en $C_{1-6}$)- ou -C (alkyle en $C_{1-6}$)$_2$- ;
- $R^{10}$ et $R^{11}$ représentent chacun indépendamment 1 à 3 substituants choisis chacun indépendamment parmi les atomes d'halogène, les groupes trifluorométhyle $-CF_3$, cyano -CN, nitro $NO_2$ et alkyle en $C_{1-6}$ et les groupes symbolisés par $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}S(O)_2R^{21}$, $-COOR^{19}$, $-CONR^{19}R^{20}$, $-COR^{19}$, $-S(O)_2NR^{19}R^{20}$, $-S(O)_{0-2}R^{21}$, $-O(CH_2)_{1-10}-COOR^{19}$, $-O(CH_2)_{1-10}-CONR^{19}R^{20}$, -(alcanediyle en $C_{1-6}$)$COOR^{19}$ et $-CH=CH-COOR^{19}$ ;
- $R^{15}$ et $R^{17}$ représentent chacun indépendamment un groupe choisi parmi les groupes symbolisés par $-OR^{19}$, $-O(CO)R^{19}$, $-O(CO)OR^{21}$ et $-O(CO)NR^{19}R^{20}$ ;
- $R^{16}$ et $R^{18}$ représentent chacun indépendamment un atome d'hydrogène ou un groupe alkyle en $C_{1-6}$ ou aryle ; ou bien soit les entités représentées par $R^{15}$ et $R^{16}$, soit les entités représentées par $R^{17}$ et $R^{18}$ forment conjointement un substituant oxo =O ;
- l'indice d vaut 1, 2 ou 3 ;
- l'indice h vaut 0, 1, 2, 3 ou 4 ;
- l'indice s vaut 0 ou 1 ; l'indice t vaut 0 ou 1 ; les indices m, n et p valent chacun indépendamment 0, 1, 2, 3 ou 4 ; sous réserve que l'un au moins des indices s et t vaille 1 et que la somme des indices m, n, p, s et t soit comprise entre 1 et 6 ; sous réserve que si p vaut 0 et t vaut 1 , la somme des indices m, s et n soit comprise entre 1 et 5 ; et sous réserve que si p vaut 0 et s vaut 1, la somme des indices m, t et n soit comprise entre 1 et 5 ;
- l'indice v vaut 0 ou 1 ;
- les indices j et k valent chacun indépendamment 1, 2, 3, 4 ou 5, sous réserve que la somme des indices j, k et v soit comprise entre 1 et 5 ;
- et lorsque Q représente une liaison et $R^1$ représente le groupe de formule

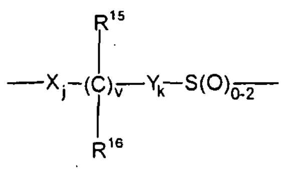 ,

$Ar^1$ peut également représenter un groupe pyridyle, isoxazolyle, furanyle, pyrrolyle, thiényle, imidazolyle, pyrazolyle, thiazolyle, pyrazinyle, pyrimidinyle ou pyridazinyle ;
- $R^{19}$ et $R^{20}$ représentent chacun indépendamment un atome d'hydrogène ou un groupe alkyle en $C_{1-6}$, aryle ou alkyle en $C_{1-6}$ à substituant aryle ;
- $R^{21}$ représente un groupe alkyle en $C_{1-6}$, aryle ou aryle à substituant(s) $R^{24}$ ;
- $R^{22}$ représente un atome d'hydrogène, un groupe alkyle en $C_{1-6}$, aryle-(alkyle en $C_{1-6}$) ou un groupe symbolisé par $-C(O)R^{19}$ ou $-COOR^{19}$ ;
- $R^{23}$ et $R^{24}$ représentent chacun indépendamment 1 à 3 substituants choisis chacun indépendamment parmi les atomes d'hydrogène et d'halogène, les groupes hydroxyle -OH, carboxyle -COOH, nitro $-NO_2$, alkyle en $C_{1-6}$ et alcoxy en $C_{1-6}$ et les groupes symbolisés par $-NR^{19}R^{20}$ ;
- et $R^{25}$ représente un atome d'hydrogène ou un groupe hydroxyle -OH ou alcoxy en $C_{1-6}$.

**10.** Emploi conforme à la revendication 1, l'inhibiteur d'absorption des stérols au nombre d'au moins un étant représenté par la formule (IX) :

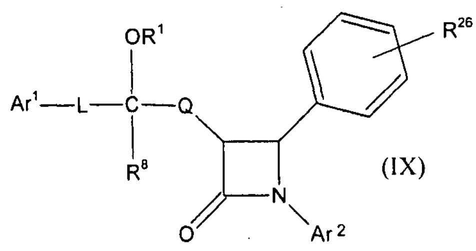

ou étant un sel ou solvat pharmacologiquement admissible d'un tel composé,
dans laquelle formule (IX) les symboles ont les significations qui suivent :

- $R^1$ représente un atome d'hydrogène, un groupe $-SO_3H$ ou $-PO_3H$ ou une entité représentée par G, $G^1$ ou $G^2$ ;
- G représente une entité choisie parmi l'atome d'hydrogène et les groupes de formules suivantes

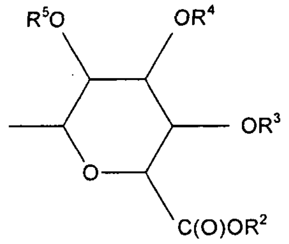 , 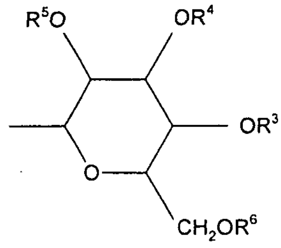 , 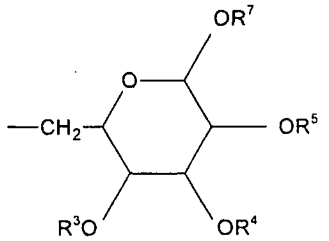 ,

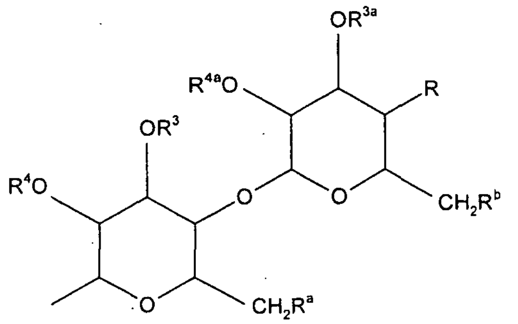 et 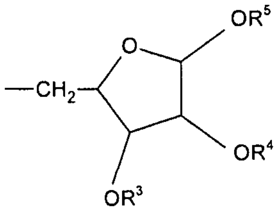 ;

dans lesquelles R, $R^a$ et $R^b$ représentent chacun indépendamment une entité choisie parmi les atomes d'hydrogène et d'halogène, les groupes hydroxyle -OH, amino $-NH_2$, azido $-N_3$ et (alcoxy en $C_{1-6}$)-(alcoxy en $C_{1-6}$) et les groupes symbolisés par $-W-R^{30}$ ;
- chaque symbole W est choisi indépendamment parmi les groupes symbolisés par -NH-C(O)-, -O-C(O)-, -O-C(O)-N($R^{31}$)-, -NH-C(O)-N($R^{31}$)- ou -O-C(S)-N($R^{31}$)- ;
- $R^2$ et $R^6$ représentent chacun indépendamment un atome d'hydrogène ou un groupe alkyle en $C_{1-6}$, acétyle, aryle ou aryle-(alkyle en $C_{1-6}$) ;
- $R^3$, $R^4$, $R^5$, $R^7$, $R^{3a}$ et $R^{4a}$ représentent chacun indépendamment un atome d'hydrogène ou un groupe alkyle en $C_{1-6}$, acétyle, aryle-(alkyle en $C_{1-6}$), -C(O)-(alkyle en $C_{1-6}$) ou -C(O)-aryle ;
- chaque symbole $R^{30}$ représente un groupe choisi indépendamment parmi les groupes T à substituant(s) $R^{32}$, (T à substituant(s) $R^{32}$)-(alkyle en $C_{1-6}$), alcényle en $C_{2-4}$ à substituant(s) $R^{32}$, alkyle en $C_{1-6}$ à substituant(s) $R^{32}$, cycloalkyle en $C_{3-7}$ à substituant(s) $R^{32}$ et (cycloalkyle en $C_{3-7}$ à substituant(s) $R^{32}$)-(alkyle en $C_{1-6}$) ;
- chaque symbole $R^{31}$ représente indépendamment un atome d'hydrogène ou un groupe alkyle en $C_{1-4}$ ;
- chaque symbole T représente un groupe choisi indépendamment dans l'ensemble que constituent les groupes phényle, furyle, thiényle, pyrrolyle, oxazolyle, isoxazolyle, thiazolyle, isothiazolyle, benzothiazolyle, thiadiazolyle, pyrazolyle, imidazolyle et pyridyle ;
- chaque symbole $R^{32}$ représente indépendamment 1 à 3 substituants choisis chacun indépendamment parmi

les atomes d'hydrogène et d'halogène, le substituant oxo, les groupes hydroxyle -OH, trifluorométhyle $-CF_3$, nitro $-NO_2$, alkyle en $C_{1-4}$, alcoxy en $C_{1-4}$, phénoxy, méthylènedioxy, alkylsulfanyle en $C_{1-4}$, alkylsulfinyle en $C_{1-4}$, alkylsulfonyle en $C_{1-4}$, $-N(CH_3)_2$, -C(O)-NH(alkyle en $C_{1-4}$), -C(O)-N(alkyle en $C_{1-4}$)$_2$, -C(O)-(alkyle en $C_{1-4}$), -C(O)-(alcoxy en $C_{1-4}$) et pyrrolidinylcarbonyle ; ou bien $R^{32}$ représente une liaison covalente et l'entité représentée par $R^{31}$, l'atome d'azote auquel elle est liée, ainsi que l'entité représentée par $R^{32}$ forment ensemble un groupe pyrrolidinyle, pipéridinyle, N-méthyl-pipérazinyle, indolinyle ou morpholinyle ou encore un groupe pyrrolidinyle à substituant (alcoxy en $C_{1-4}$)-carbonyle, pipéridinyle à substituant (alcoxy en $C_{1-4}$)-carbonyle, N-méthyl-pipérazinyle à substituant (alcoxy en $C_{1-4}$)-carbonyle, indolinyle à substituant (alcoxy en $C_{1-4}$)-carbonyle ou morpholinyle à substituant (alcoxy en $C_{1-4}$)-carbonyle ;

- $G^1$ représente un groupe de formule

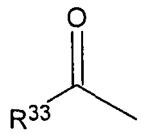

dans laquelle $R^{33}$ représente un groupe alkyle sans substituant ou à substituant(s) $R^{34}$ ou un groupe de formule ($R^{35}$)($R^{36}$)alkyle-,

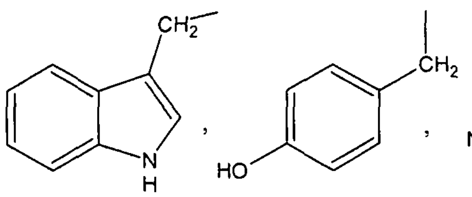 , 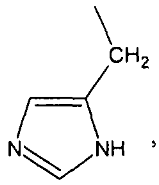 , 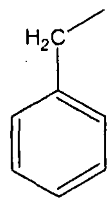 , 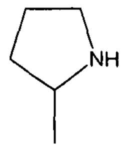 ou 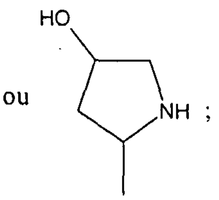 ;

- $R^{34}$ représente 1 à 3 substituants choisis chacun indépendamment parmi les groupes HOOC-, HS-, $(CH_3)S-$, $H_2N-$, $(NH_2)(NH)C(NH)-$, $(NH_2)C(O)-$ et $HOOCCH(NH_3^+)CH_2SS-$ ;
- $R^{35}$ représente un atome d'hydrogène ou un groupe amino $-NH_2$ ;
- $R^{36}$ représente un atome d'hydrogène, un groupe alkyle sans substituant ou à substituant(s) $R^{34}$ ou un groupe cycloalkyle sans substituant ou à substituant(s) $R^{34}$ ;
- $G^2$ représente un groupe de formule

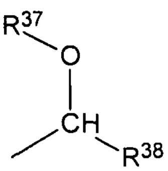

dans laquelle $R^{37}$ et $R^{38}$ représentent chacun indépendamment un groupe alkyle en $C_{1-6}$ ou aryle ;

- $R^{26}$ représente 1 à 5 substituants choisis chacun indépendamment dans l'ensemble que constituent :

les atomes

a) d'hydrogène ;
b) de fluor ;
c) et de chlore ;

et les groupes symbolisés par

d) -OH ;

e) $-OCH_3$ ;
f) -O-G ;
g) $-O-G^1$ ;
h) $-O-G^2$ ;
i) $-SO_3H$;
j) et $-PO_3H$ ;

sous réserve que lorsque $R^1$ représente un atome d'hydrogène, $R^{26}$ ne représente ni un atome d'hydrogène, ni un groupe hydroxyle -OH, ni un groupe méthoxy $-OCH_3$ ni un groupe symbolisé par -O-G ;

- $Ar^1$ représente un groupe aryle, aryle à substituant(s) $R^{10}$, hétéroaryle ou hétéroaryle à substituant(s) $R^{10}$ ;
- $Ar^2$ représente un groupe aryle, aryle à substituant(s) $R^{11}$, hétéroaryle ou hétéroaryle à substituant(s) $R^{11}$;
- L représente une entité choisie parmi les suivantes :

a) une liaison covalente ;
b) un groupe $-(CH_2)_q-$, où l'indice q vaut de 1 à 6 ;
c) un groupe $-(CH_2)_e-E-(CH_2)_r-$, où E représente -O-, -C(O)-, $-S(O)_{0-2}-$, un groupe phénylène ou symbolisé par $-NR^{22}-$ et les indices e et r valent chacun de 0 à 5, sous réserve que la somme des deux soit comprise entre 1 et 6 ;
d) un groupe alcènediyle en $C_{2-6}$ ;
e) un groupe $-(CH_2)_f-V-(CH_2)_g-$, où V représente un groupe cycloalcanediyle en $C_{3-6}$ et les indices f et g valent, respectivement, de 1 à 5 et de 0 à 5, sous réserve que la somme des deux soit comprise entre 1 et 6 ;
f) les groupes de formules

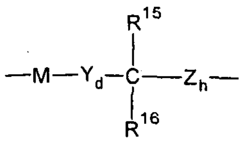 , 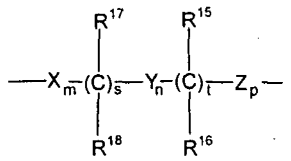 et 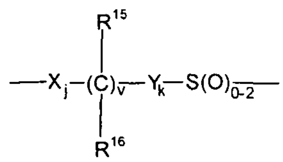 ;

dans lesquelles

- M représente une entité choisie parmi -O-, -S-, -S(O)- et $-S(O)_2-$ ;
- et X, Y et Z représentent chacun indépendamment un groupe méthanediyle $-CH_2-$, -CH(alkyle en $C_{1-6}$)- ou -C(alkyle en $C_{1-6})_2-$ ;

- $R^8$ représente un atome d'hydrogène ou un groupe alkyle ;
- $R^{10}$ et $R^{11}$ représentent chacun indépendamment 1 à 3 substituants choisis chacun indépendamment parmi les atomes d'halogène, les groupes trifluorométhyle $-CF_3$, cyano -CN, nitro $-NO_2$, et alkyle en $C_{1-6}$ et les groupes symbolisés par $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}S(O)_2R^{21}$, $-COOR^{19}$, $-CONR^{19}R^{20}$, $-COR^{19}$, $-S(O)_2NR^{19}R^{20}$, $-S(O)_{0-2}R^{21}$, $-O(CH_2)_{1-10}-COOR^{19}$, $-O(CH_2)_{1-10}-CONR^{19}R^{20}$, -(alcanediyle en $C_{1-6})COOR^{19}$ et $-CH=CH-COOR^{19}$ ;
- $R^{15}$ et $R^{17}$ représentent chacun indépendamment un groupe choisi parmi les groupes symbolisés par $-OR^{19}$, $-O(CO)R^{19}$, $-O(CO)OR^{21}$ et $-O(CO)NR^{19}R^{20}$;
- $R^{16}$ et $R^{18}$ représentent chacun indépendamment un atome d'hydrogène ou un groupe alkyle en $C_{1-6}$ ou aryle ; ou bien soit les entités représentées par $R^{15}$ et $R^{16}$, soit les entités représentées par $R^{17}$ et $R^{18}$ forment conjointement un substituant oxo =O ;
- l'indice d vaut 1, 2 ou 3 ;
- l'indice h vaut 0, 1, 2, 3 ou 4 ;
- l'indice s vaut 0 ou 1 ; l'indice t vaut 0 ou 1 ; les indices m, n et p valent chacun indépendamment 0, 1, 2, 3 ou 4 ; sous réserve que l'un au moins des indices s et t vaille 1 et que la somme des indices m, n, p, s et t soit comprise entre 1 et 6 ; sous réserve que si p vaut 0 et t vaut 1, la somme des indices m, n et p soit comprise entre 1 et 5 ; et sous réserve que si p vaut 0 et s vaut 1, la somme des indices m, t et n soit comprise entre 1 et 5 ;
- l'indice v vaut 0 ou 1 ;
- les indices j et k valent chacun indépendamment 1, 2, 3, 4 ou 5, sous réserve que la somme des indices j, k et v soit comprise entre 1 et 5 ;

- Q représente une liaison, un groupe $-(CH_2)_q-$, où l'indice q vaut de 1 à 6, ou bien une entité qui forme, conjointement avec l'atome de carbone en position 3 du cycle azétidinone, le groupe spiro de formule

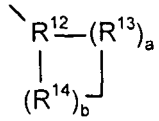 ;

dans laquelle $R^{12}$ représente un groupe choisi parmi les suivants :

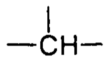 , 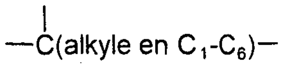 , 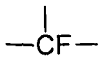 , 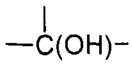 , 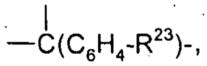 , 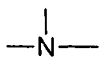 et 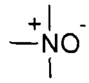

- $R^{13}$ et $R^{14}$ représentent chacun indépendamment un groupe méthanediyle $-CH_2-$, -CH(alkyle en $C_{1-6}$)-, -C (alkyle en $C_{1-6}$)$_2$-, -CH=CH- ou -C(alkyle en $C_{1-6}$)=CH- ; ou bien soit l'entité représentée par $R^{12}$ et une entité adjacente représentée par $R^{13}$, soit l'entité représentée par $R^{12}$ et une entité adjacente représentée par $R^{14}$ forment ensemble un groupe -CH=CH- ou -CH=C(alkyle en $C_{1-6}$)-;
- les indices a et b valent chacun indépendamment 0, 1, 2 ou 3 ; sous réserve que l'un et l'autre ne soient pas nuls ; sous réserve que lorsque $R^{13}$ représente -CH=CH- ou -C(alkyle en $C_{1-6}$)=CH-, a vaille 1 ; sous réserve que lorsque $R^{14}$ représente -CH=CH- ou -C(alkyle en $C_{1-6}$)=CH-, b vaille 1 ; sous réserve que lorsque a vaut 2 ou 3, les entités représentées par $R^{13}$ puissent être identiques ou différentes ; et sous réserve que lorsque b vaut 2 ou 3, les entités représentées par $R^{14}$ puissent être identiques ou différentes ;
- et lorsque Q représente une liaison et L représente le groupe de formule

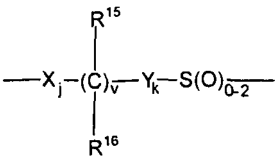 ;

$Ar^1$ peut également représenter un groupe pyridyle, isoxazolyle, furanyle, pyrrolyle, thiényle, imidazolyle, pyrazolyle, thiazolyle, pyrazinyle, pyrimidinyle ou pyridazinyle ;
- $R^{19}$ et $R^{20}$ représentent chacun indépendamment un atome d'hydrogène ou un groupe alkyle en $C_{1-6}$, aryle ou alkyle en $C_{1-6}$ à substituant aryle ;
- $R^{21}$ représente un groupe alkyle en $C_{1-6}$, aryle ou aryle à substituant(s) $R^{24}$ ;
- $R^{22}$ représente un atome d'hydrogène, un groupe alkyle en $C_{1-6}$, aryle-(alkyle en $C_{1-6}$) ou un groupe symbolisé par $-C(O)R^{19}$ ou $-COOR^{19}$ ;
- $R^{23}$ et $R^{24}$ représentent chacun indépendamment 1 à 3 substituants choisis chacun indépendamment parmi les atomes d'hydrogène et d'halogène, les groupes hydroxyle -OH, carboxyle -COOH, nitro $-NO_2$, alkyle en $C_{1-6}$ et alcoxy en $C_{1-6}$ et les groupes symbolisés par $-NR^{19}R^{20}$ ;
- et $R^{25}$ représente un atome d'hydrogène ou un groupe hydroxyle -OH ou alcoxy en $C_{1-6}$.

**11.** Emploi conforme à la revendication 1, le taux sanguin de protéine C-réactive étant supérieur à environ 1,0 mg/dL.

**12.** Emploi conforme à la revendication 1, le taux sanguin de protéine C-réactive étant supérieur à environ 3,4 mg/dL.

**13.** Emploi conforme à la revendication 1, comprenant en outre le fait d'administrer au moins un activateur des récepteurs activés par les proliférateurs de peroxysomes.

**14.** Emploi conforme à la revendication 13, l'activateur des récepteurs activés par les proliférateurs de peroxysomes au nombre d'au moins un étant un dérivé de l'acide fibrique.

**15.** Emploi conforme à la revendication 14, le dérivé de l'acide fibrique étant choisi dans l'ensemble que constituent le

fénofibrate, le clofibrate, le gemfibrozil, le ciprofibrate, le bézafibrate, le clinofibrate, le binifibrate, le lifibrol et les mélanges de ces composés.

**16.** Emploi conforme à la revendication 1, l'inhibiteur d'absorption des stérols au nombre d'au moins un étant administré à un mammifère en une quantité journalière approximativement comprise entre 0,1 et 1000 mg d'inhibiteur d'absorption des stérols.

**17.** Emploi conforme à la revendication 1, l'inhibiteur de biosynthèse du cholestérol au nombre d'au moins un comprenant au moins un inhibiteur de la HMG CoA-réductase.

**18.** Emploi conforme à la revendication 17, l'inhibiteur de la HMG CoA-réductase au nombre d'au moins un étant choisi dans l'ensemble que constituent la lovastatine, la pravastatine, la fluvastatine, la simvastatine, l'atorvastatine, la cérivastatine et les mélanges de ces composés.

**19.** Emploi conforme à la revendication 1, comprenant en outre le fait d'administrer au moins un agent chélateur des acides biliaires.

**20.** Emploi conforme à la revendication 1, comprenant en outre le fait d'administrer de l'acide nicotinique ou l'un de ses dérivés.

**21.** Emploi conforme à la revendication 1, comprenant en outre le fait d'administrer au moins un inhibiteur de l'acylCoA: cholestérol O-acyltransférase.

**22.** Emploi conforme à la revendication 1, comprenant en outre le fait d'administrer du probucol ou un dérivé de celui-ci.

**23.** Emploi conforme à la revendication 1, comprenant en outre le fait d'administrer au moins un activateur des récepteurs des lipoprotéines de basse densité.

**24.** Emploi conforme à la revendication 1, comprenant en outre le fait d'administrer au moins un acide gras de type oméga-3.

**25.** Emploi conforme à la revendication 1, comprenant en outre le fait d'administrer au moins une fibre hydrosoluble naturelle.

**26.** Emploi conforme à la revendication 1, comprenant en outre le fait d'administrer l'un au moins des composés que sont les stérols végétaux, les stanols végétaux ou les esters d'acides gras des stanols végétaux.

**27.** Emploi conforme à la revendication 1, comprenant en outre le fait d'administrer au moins un agent antioxydant ou une vitamine.

**28.** Emploi conforme à la revendication 1, comprenant l'étape consistant à administrer une quantité à effet thérapeutique de l'inhibiteur d'absorption des stérols et d'un véhicule pharmacologiquement admissible.

**29.** Emploi conforme à la revendication 1, le sujet étant un être humain.

**30.** Emploi d'une combinaison constituée :

1) d'au moins un inhibiteur d'absorption des stérols ou d'au moins un inhibiteur d'absorption des 5$\alpha$-stanols,
2) et d'au moins un inhibiteur de biosynthèse du cholestérol,

en vue de la fabrication d'un médicament destiné à diminuer les taux vasculaires de protéine C-réactive chez un mammifère présentant un taux sanguin de protéine C-réactive supérieur à 0,4 mg/dL.

**31.** Emploi conforme à la revendication 30, le taux sanguin de protéine C-réactive étant abaissé à environ 3,4 mg/dL ou moins.

**32.** Emploi conforme à la revendication 30, le taux sanguin de protéine C-réactive étant abaissé à environ 1,0 mg/dL ou moins.

**33.** Emploi conforme à la revendication 30, le taux sanguin de protéine C-réactive étant abaissé à environ 0,4 mg/dL ou moins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 32393701 P
- US 16694202 A
- US 5358852 A
- US 6040147 A
- US 6277584 B
- US 5631365 A
- US 5767115 A
- US 5846966 A
- US 6207822 B
- US 10571002 A
- WO 9302048 A
- US 5688990 A
- US 5656624 A
- US 5624920 A
- US 5698548 A
- WO 9417038 A
- WO 9508532 A
- US 9503196 W
- US 08463619 B
- WO 9728149 A
- US 3948973 A
- US 3781328 A
- US 3716583 A
- BE 884722
- US 6028109 A
- WO 0075103 A
- WO 9843081 A
- WO 9805331 A
- WO 0076488 A
- US 5994554 A
- US 5859051 A
- WO 9920275 A
- WO 9938845 A
- WO 0063161 A
- WO 0100579 A
- WO 0112612 A
- WO 0112187 A
- WO 9731907 A
- WO 0100603 A
- US 5093365 A
- WO 9904815 A
- US 6248781 B
- WO 0023416 A
- WO 0023415 A
- WO 0023425 A
- WO 0023445 A
- WO 0023451 A
- WO 0063153 A
- WO 9725042 A
- WO 0063190 A
- WO 0121181 A
- WO 0116120 A
- WO 0063196 A
- WO 0063209 A
- US 6008237 A
- WO 0078312 A
- WO 0078313G A
- US 6166049 A
- WO 0117994 A
- WO 0125225 A
- WO 0125226 A
- WO 0114349 A
- WO 0114350 A
- WO 0104351 A
- WO 0050392 A
- WO 0053563 A
- WO 9946232 A
- WO 9912534 A
- WO 9915520 A
- WO 0121578 A
- WO 0140192 A
- WO 9711345 A
- WO 9857652 A
- US 3692895 A
- US 5703188 A
- WO 0038727 A
- WO 0038721 A
- US 6147090 A
- US 6121319 A
- US 6147250 A
- WO 9938498 A

### Non-patent literature cited in the description

- **MENDALL MA ; PATEL P ; BALLAM L et al.** C-reactive protein and its relation to cardiovascular risk factor: A population based cross sectional study. *BMJ.,* 1996, vol. 312, 1061-1065
- **RIKER P ; HAUGHIE P.** Prospective studies of C-reactive protein as a risk factor for cardiovascular disease. *J Investig Med.,* 1998, vol. 46, 391-395

- **P. CHANG et al.** Current, New and Future Treatments in Dyslipidaemia and Atherosclerosis. *Drugs,* July 2000, vol. 60 (1), 55-93
- **M. HUETTINGER et al.** Hypolipidemic activity of HOE-402 is Mediated by Stimulation of the LDL Receptor Pathway. *Arterioscler. Thromb.,* 1993, vol. 13, 1005-12
- **H. GYLLING et al.** Serum Sterols During Stanol Ester Feeding in a Mildly Hypercholesterolemic Population. *J. Lipid Res.,* 1999, vol. 40, 593-600